# EUROPEAN PATENT SPECIFICATION

(11) **EP 2 141 248 B1**
(45) Date of publication and mention of the grant of the patent: **12.12.2012**
(21) Application number: 08290656.1
(22) Date of filing: 03.07.2008
(51) Int. Cl.: C12Q 1/68

(54) **Methods to identify leishmania virulence factors**
Verfahren zur Identifizierung von Leishmania-Virulenzfaktoren
Procédés pour identifier des facteurs de virulence de Leishmania

(43) Date of publication of application: 06.01.2010
(73) Proprietor: Institut Pasteur, 75015 Paris (FR); INSTITUT PASTEUR DE TUNIS, 1002 Tunis (TN)
(72) Inventor: Guizani, Ikram, 2080 Ariana (TN); Kbaier-Hachemi, Houaida, 2034 Ez-Zahra-Tunis (TN); Chakroun, Ahmed Sahbi, 2070 Tunis (TN); Kaabi, Belhassen, 2053 Tunis (TN)
(74) Representative: Thomas, Dean

(56) References cited:
- EP-B- 0 716 697
- WO-A-02/103002
- ROCHETTE ANNIE ET AL: "Genome-wide gene expression profiling analysis of Leishmania major and Leishmania infantum developmental stages reveals substantial differences between the two species." 2008, BMC GENOMICS 2008, VOL. 9, PAGE(S) 255 , XP002496773 ISSN: 1471-2164 * abstract * * page 22 - page 23; figure 2 *
- AKOPYANTS NATALIA S ET AL: "Expression profiling using random genomic DNA microarrays identifies differentially expressed genes associated with three major developmental stages of the protozoan parasite Leishmania major." July 2004 (2004-07), MOLECULAR AND BIOCHEMICAL PARASITOLOGY JUL 2004, VOL. 136, NR. 1, PAGE(S) 71 - 86 , XP002496774 ISSN: 0166-6851 * page 72 - page 74 * * page 78; table 3 *
- SAXENA ALKA ET AL: "Evaluation of differential gene expression in Leishmania major Friedlin procyclics and metacyclics using DNA microarray analysis." June 2003 (2003-06), MOLECULAR AND BIOCHEMICAL PARASITOLOGY, VOL. 129, NR. 1, PAGE(S) 103-114 , XP002496775 ISSN: 0166-6851 * abstract; table 3 * * page 104 - page 105 *
- JOSHI MANJU ET AL: "Cloning and characterization of differentially expressed genes from in vitro-grown "amastigotes" of Leishmania donovani" 1993, MOLECULAR AND BIOCHEMICAL PARASITOLOGY, VOL. 58, NR. 2, PAGE(S) 345-354 , XP002496776 ISSN: 0166-6851 * abstract *
- RAMOS EDURNE ET AL: "A DNA aptamer population specifically detects Leishmania infantum H2A antigen." May 2007 (2007-05), LABORATORY INVESTIGATION; A JOURNAL OF TECHNICAL METHODS AND PATHOLOGY MAY 2007, VOL. 87, NR. 5, PAGE(S) 409 - 416 , XP002496777 ISSN: 0023-6837 * the whole document *
- TURCO S J ET AL: "Is lipophosphoglycan a virulence factor? A surprising diversity between Leishmania species." TRENDS IN PARASITOLOGY MAY 2001, vol. 17, no. 5, May 2001 (2001-05), pages 223-226, XP002496772 ISSN: 1471-4922

## Description

The present invention relates to new methods to identify genes which affect the virulence of *Leishmania*. In addition the present disclosure also relates to methods to identify factors which can affect these virulence genes as well as to the use of such identified genes for use as therapeutics, diagnostic tools and vaccines.

*Leishmania* parasites are members of the Trypanosomatidae family, responsible for a large spectrum of clinical manifestations called leishmaniasis that range from spontaneously healing skin ulcers to disfiguring muco-cutaneous lesions to fatal visceral disease. In the current Patent Application the two main classes of leishmaniases will be called visceral (VL) and cutaneous (CL). Visceral is used for any leishmaniasis pertaining to the internal organs and other internal tissues and cutaneous for any leishmaniasis pertaining to the skin.

These insect vector-transmitted diseases have a worldwide distribution, are endemic in more than 88 countries and threaten the health of over 350 million people. Global prevalence is estimated to be more than 12 million while the annual incidence is 2 million cases (Murray et al., 2005). At least 20 *Leishmania* species are pathogenic to humans.

*Leishmania* parasites have a digenetic life cycle involving a flagellated extracellular promastigote stage, encountered in the midgut of the sand fly vector and in axenic cultures and an aflagellated intracellular amastigote stage, which reside in macrophage phagolysosomes of the mammalian host.

As they migrate within the sand fly digestive tract, the promastigotes undergo a number of morphological transformations leading to infective metacyclics, which are egested with the sand fly saliva during a blood meal (Kamhawi, 2006). This process is also accompanied with molecular changes reflected at different levels including the surface coat and metabolism (Louassini et al., 1998; 1999a; 1999b).

Differences are also observed between promastigote and amastigote forms (Holzer et al., 2006; Saxena et al., 2007). In Rochette et al., 2008, genome wide expression analysis has been performed on *L. major* and *L. infantum* this work demonstrated substantial differences in gene expression patterns between these species at various developmental stages.

Different approaches have been considered to identify life-stage specific products particularly at the infective metacyclic or amastigote stages. Such products constitute potential parasite virulence factors that could be associated with invasion, evasion, pathogenesis or parasite protection (Chang et al., 1999; Chang and McGuire, 2002; Chang et al., 2003).

In many endemic countries, *L. infantum* parasites are responsible for visceral (VL) and cutaneous (CL) leishmaniasis (Belhadj et al., 2003; Pratlong et al., 2004; Ben Said et al., 2006; Campino et al., 2006). In addition, a number of infections remain asymptomatic (Kallel et al., 2007).

This clinical variability is likely the result of a complex interplay of several inherently complex biological processes including host genetic factors, the host immune response, vectorial determinants and intrinsic parasite factors (Sulahian et al., 1997; Honoré et al., 1998). In this milieu, intrinsic differential levels of expression of genes/proteins between cutaneous and visceral *L. infantum* parasites are hypothesized to be among the mechanisms potentially involved in this important interplay.

Such differentially expressed genes could constitute candidate virulence genes involved in *Leishmania* pathogenicity. Previous reports highlighted a correlation between gene dosage and virulence of *L. major* (Antoniazi et al., 2000) or clinical origin of *L. infantum* parasites (Guerbouj et al., 2001). Furthermore, two metabolic pathways (glycolysis and pentose phosphate) have been followed during *L. infantum* growth and differentiation, and significant variations in metabolism between a VL and a CL *L. infantum isolate* have been observed (Louassini et al., 1999a; 1999b).

Furthermore, negative selection with peanut agglutinin (PNA) proved efficient in separating infective (PNA -) from non-infective (PNA +) *L.infantum* promastigotes (Louassini et al., 1998), which has opened the way to screen for and study differences in gene expression within infective and non-infective organisms from this species.

In spite of the demonstrated validity of genomic or cDNA micro arrays in identifying genes differentially expressed in the different life stages of a *Leishmania* isolate of *L. major, L. amazonensis* or *L. donovani* (Saxena et al., 2003, Holzer et al., 2006, Leifso et al., 2007, Saxena et al., 2007), where in particular differential gene expression studies have been performed using microarrays comprising the complete genome of these organisms, such technologies are still restricted to a small number of researchers because of the cost and complexity of the equipment required to perform such studies. In addition these cost and complexity considerations, make it impractical to conduct a large number of small scale studies on particular isolates of interest.

Akopyants et al. (Expression profiling using random genomic DNA microarrays identifies differentially expressed genes associated with three major developmental stages of the protozoan parasite Leishmania major, Molecular and Biochemical Parasitology, 2004, Vol. 136, Nr. 1, p. 71 - 86) discloses a method to screen for differentially expressed genes in different developmental stages of Leishmania using a microarray containing 11484 PCR products that included a number of known genes and 10464 random 1 kb genomic DNA fragments.

Therefore an objective of the present invention is to identify intrinsic differences in gene expression among *Leishmania* isolates which are correlated with their clinical origin, VL or CL, of these parasites or based upon other criteria, so allowing a differential selection of putative virulence factors to be made.

To overcome the problems known in the prior art, the inventors have developed a new method which reduces the scope of the screen to a single chromosome or a library enriched for a specific single chromosome. This new methodology has a number of advantages, in particular this new method eliminates or minimises as far as possible the known problems with global screening methods, in particular the problem of the same genes products being identified in such global studies, because such gene products are intrinsically differentially expressed and/or are already known. In addition the inventor's new methods allow workers with less complex equipment to identify virulence factors from Leishmania and for quicker, smaller scale experiments to be conducted.

Therefore according to a first aspect of the present invention there is provided a method to screen for at least one genetic element which is differentially expressed between a first *Leishmania* sample (L₁) and a second *Leishmania* sample (L₂), comprising the steps:
a) generating a first cDNA pool and a second cDNA pool from said L₁ and said L₂ respectively;
b) contacting at least a portion of a nucleic acid library comprising genomic DNA enriched for the DNA of at least one specific chromosome of a reference *Leishmania* isolate, with said first cDNA pool and said second cDNA pool;
c) identifying at least one clone from said nucleic acid library with which said first cDNA pool and/or said second cDNA pool hybridise, so as to generate a first clone pool and a second clone pool respectively;
d) isolating at least one clone from said first clone pool which is not present in said second clone pool;
e) identifying said at least one genetic element which is differentially expressed between said L₁ and said L₂ from step d).

In the present disclosure, a genetic element is defined as any genomic DNA molecule coding for any peptide or RNA molecule or other element which has a biological effect or role in the organism being studied. In particular a genetic element may be a gene coding for a protein.

In the present Patent, a *Leishmania* sample (L₁ or L₂) is defined as a source of RNA, such as a live or stored Leishmania strain in laboratory culture or an isolate from a patient, in both cases the sample can be at any stage in the *Leishmania* life cycle and the *Leishmania* sample can also be an extract of such a sample comprising RNA as well as potentially contaminants such as DNA, lipids, carbohydrates and/or peptides.

In particular the first *Leishmania* sample (L₁) and second *Leishmania* sample (L₂) can be from the same isolate at different stages in the *Leishmania* life cycle or from different isolates.

In the present Patent the reference *Leishmania* isolate can be the same as L₁ or L₂ or different. In any case this reference *Leishmania* isolate does not vary as it comprises genomic DNA.

In the present Patent the first cDNA pool and second cDNA pool can be a collection of free nucleic acid molecules in solution or purified or a library of cloned cDNA molecules which can be maintained in a suitable bacterial or other host organism for iterative use.

This new method therefore allows the screening of a *Leishmania* genomic DNA library comprising a portion of the total *Leishmania* genome, for genetic elements such as gene products which are differentially expressed between two selected *Leishmania* samples. This method is simpler to perform than prior art methods and as it is more focussed the number of clones identified is smaller but the relevance of each of these clones is higher than in prior art methods. However to place this in context, the number of potential clones per chromosome identified is still high, in the experiments described herein fifty nine genes were identified of which fifteen have been investigated fully. This shows that the current methodology can identify a large number of genes which are differentially expressed between samples and which are chromosomally localised.

Preferably said L₁ is a visceral isolate and said L₂ is a cutaneous isolate.

As explained previously as well as the myriad of other factors affecting *Leishmania* infection, it is thought that intrinsic genetic and/or biochemical differences affect the result of *Leishmania* infection and therefore by comparing the expression patterns between visceral and cutaneous isolates the identification of visceral specific and/or cutaneous specific virulence factors may be identified.

Alternatively said L₁ is a promastigote life stage and said L₂ is an amastigote life stage.

Specific patterns of gene expression, probably transient in nature, which result in the differentiation of the parasite provide potentially large number of therapeutic and vaccine targets. For instance the specific targeting of an essential differentiation gene or combination of genes, would target differentiating organisms specifically so lessening potential side effects and also decreasing the overall selective pressure upon the organism per se to develop resistance.

Alternatively said L₁ is PNA+ and said L₂ is PNA-.

Most preferably, said L₁ and said L₂ are selected from the group comprising: *L. infantum;* L. *major; L. braziliensis.*

Said nucleic acid library comprises genomic DNA enriched for the DNA of at least one specific chromosome of a *Leishmania* isolate.

In the present invention a nucleic acid library comprising genomic DNA enriched for the DNA of at least one specific chromosome is one in which the selection of a particular chromosome for instance by centrifugation or gel electrophoresis has been made and this DNA sample is used to create the DNA library.

The inventors have used in the current work a fraction of a chromosome 5- enriched library generated by random mechanical shearing of a gel purified chromosome in the work described herein.

By focussing on one chromosome, this increases the chances of identifying novel candidate virulence genes and of avoiding picking up the classical repeated gene families (e.g. PSA2, gp63, amastins, etc..). Chromosome 5 shows size variability within *L. infantum* natural populations (Pages et al., 1989), includes relevant genes and markers like a protein associated with visceralization of *L. tropica* (Dillon et al., 1995), the trypanothione reductase (Ivens et al., 2005), *L. infantum* specific interspersed repeats (Ravel et al., 1995b) and polymorphic markers (Guizani et al., unpublished data). In addition, at the time of the study, sequencing of this chromosome through the *Leishmania* genome project was under way with available relevant data on a physical map (Ravel et al., 1995a, Tamar et al., 2000). The screening revealed a high proportion of inserts assigned to this chromosome. The others result either from contamination of the chromosome 5 cut from the gel with the closely migrating chromosome 4 or from presence of free DNA or destroyed promastigotes with the parasites embedded in the agarose blocks loaded on the PFGE gels.

Most preferably said at least one specific chromosome is selected from the group comprising: chromosome 4, chromosome 5, chromosome 35.

According to a further aspect of the present invention the method comprises an alternative step e1));
el) the quantitative amplification of said at least one clone from said L₁ and from said L₂ to determine the levels of expression of said at least one clone in said L₁ and said L₂ and comparing the ratio of these expression levels; and
a further step f);
f) wherein when the ratio of said expression levels differs from 1, identifying genetic elements differentially expressed between said L₁ and said L₂.

This elaboration of the method, allows the identification of virulence factors which are present in the two samples but which are expressed at different levels. In this method, amplification primers specific for the selected clone are designed based upon the sequence of the clone which can be determined using a first set of sequencing primers specific to the plasmid or vector in which the clone is present.

As a further elaboration to this method, it is possible that two or more different life stages of two or more clinical isolates, or any other suitable combination, could be made for instance looking at PNA- and PNA+ phenotypes of visceral and cutaneous isolates, to compare gene expression levels of the selected clone (s) at different life stages of the same isolate and also to compare these expression levels at different life stages between isolates.

Further elaborations of these methods involving extended time courses and/or phenotypic or biochemical selection steps are also encompassed within the present disclosure.

Preferably said levels of expression of said at least one clone in said L₁ and said L₂ are also compared to a constitutively expressed endogenous gene in said L₁ and said L₂ respectively.

In the present work the inventors have used alpha-tubulin as the constitutively expressed endogenous gene, but other constitutively active genes are also encompassed within the current technology, such as the various forms of actin, collagen and elastin.

Most preferably said at least one genetic element which is differentially expressed, is characterised in that it affects the invasive, patho-antigenic and/or evasive characteristics; differentiation; virulence or pathogenicity of the *Leishmania* isolate from which it was identified.

Using this new method the inventors have identified a number of Leishmania virulence factors including the following genes:
- LinJ04_V3.0080 (SEQ ID NO: 25) which encodes a conserved protein assigned to the S-adenosyl- L-methionine dependent methyltransferase super family (LT7) (SEQ ID NO: 26).
- LinJ05_V3.0830 (SEQ ID NO: 29) which encodes the methyl adenosine phosphorylase (MAP) (SEQ ID NO: 30).
- LinJ05_V3.0170 (SEQ ID NO: 27) which encodes a conserved hypothetical protein (SEQ ID NO: 28).
- LinJ05_V3.0180 (SEQ ID NO: 31) which encodes a Dihydrolipoamide branched chain transacyclase (LAT) (SEQ ID NO: 32).
- LinJ05_V3.0660 (SEQ ID NO: 33) which encodes a Nitroreductase-like enzyme (NR) (SEQ ID NO: 34).

The names of these genes correspond to the international nomenclature adopted by genedb; they refer, when taking LinJ04_V3.0080 as an example, to a gene from *L. infantum* clone JPCM5 (Denise et al. 2006) and specifically **Lin**J refers to *L. infantum* clone **J**PCM 5 (MCAN/98/LLM-877). 04 designates the chromosome number, in this case chromosome 4. V3 refers to the version of the genome sequence of *L. infantum* clone JPCM5 (MCAN/98/LLM-877) and 0080 represents the number assigned to this gene in this version of the genome sequence.

The characterisation of further aspects of these genes are described in the present disclosure.

According to a second aspect of the present disclosure there is provided a method to determine the effect of at least one candidate *Leishmania* virulence factor (L_{f0}) identified by a method according to the current invention, comprising the steps:
a) a first measurement of at least one virulence characteristic of a *Leishmania* culture comprising at least one endogenous copy of said L_{f0} or an extract thereof,
b) the elimination of the function of said L_{f0} from said *Leishmania* culture or an extract thereof,
c) a second measurement of said at least one virulence characteristic of said *Leishmania* culture of step b),
d) the comparison of said first measurement and said second measurement and wherein these differ identifying a *Leishmania* virulence factor.

In the present Patent Application virulence is defined as the relative capacity of a pathogen to cause damage in a host.

In the present Patent Application a pathogen is defined as a microbe capable of causing host damage; the definition can encompass classical pathogens and opportunistic pathogens; host damage can result from either direct microbial action or the host immune response.

In the present Patent Application a virulence factor is defined as a component of a pathogen that damages the host; can include components essential for viability, invasion or evasion from the host immune system, components that interfere with or modulate the host immune response or that interfere with the progression of the disease or the cure.

In the present Patent Application a virulence characteristic is defined as a measurable effect of the pathogen or an extract thereof that damages the host or has one of the other listed effects; and which is measured in an *in vitro* or *in vivo* assay, for instance an ELISA or other antibody titre assay or a viability assay or another type of suitable assay.

A standard way to measure the virulence of *Leishmania* is to compare strains to each other or to an experimental control in an assay normally based on macrophage cell culture or infection of a mammalian model organism. In either case the Parasitic Load in the cells is observed in *in vitro* culture or in a sample of tissue isolated from the lesions or the body of the model and is compared to a control.

The measurement of the virulence characteristic can be qualitative or quantitative.

The elimination of the function of the candidate *Leishmania* virulence factor can be achieved via gene replacement or site directed mutagenesis, knock in and knock out techniques and iRNA, as well as any other method known in the art to affect the function of a gene.

According to a third aspect of the present disclosure, there is provided a method for identifying substances which modulate the effects of at least one candidate *Leishmania* virulence factor (L_{f1}) identified by a method according to the current invention, comprising the steps:
a) measurement of at least one characteristic of L_{f1} ;
b) exposure of L_{f1} to at least one candidate substance and the measurement of said at least one characteristic of L_{f1}.
c) comparison of the measurements of said at least one characteristic of step a) and step b), and wherein these differ identifying a substance which specifically affects said L_{f1}.

In the present disclosure the characteristic of the *Leishmania* virulence factor (L_{f1}), which is measured can be any structural, biochemical, physiological or other measurable characteristic. In particular when the *Leishmania* virulence factor is an enzyme this characteristic can be a specific measurement of enzyme catalysis. Alternatively, *in vivo* or a cell free immunological assay could also be a measurement of the virulence characteristic of the whole Leishmania organism or an extract thereof, for instance a pfu (plaque forming units), ELISA or a viability assay.

Preferably the method comprises the further steps of:
d) measurement of at least one characteristic of a human orthologue (Hn)ofLn;
e) exposure of H_{f1} to at least one candidate substance and the measurement of said at least one characteristic of H_{f1};
f) comparison of the measurements of said at least one characteristic of steps d) and f); wherein when said measurements of steps a) and b) differ and said measurements of steps c) and d) do not differ, identifying a substance which specifically modulates the effects of said Ln.

In this further elaboration of this method, substances which specifically affect the *Leishmania* virulence factor or more generally have a greater effect upon the *Leishmania* virulence factor than the human orthologue can be identified. Such substances would be expected to have little effect upon humans hence minimising the detrimental side effects of the selected substance whilst retaining activity against the *Leishmania* virulence factor.

Preferably this method may be *performed in vitro.*

Alternatively this method may be performed *in vivo.*

According to a fourth aspect of the present disclosure, there is provided a method for identifying substances specific for at least one candidate *Leishmania* virulence factor (L_{f2}) identified by a method according to the present invention, using a mixture of nucleic acids, wherein said method comprises at least the following steps:
(a) bringing a mixture of nucleic acids which form a combinatorial library into contact with L_{f2};
(b) recovering nucleic acids which bind to L_{f2}, in step (a);
(c) amplifying said nucleic acids with high affinity for L_{f2}, so as to obtain a mixture of nucleic acids, enriched in nucleic acids having a high affinity for said L_{f2}, and
(d) identifying aptamers specific for L_{f2}, from the mixture obtained in (e).

Preferably this method comprises the further replacement steps of:
(al) bringing a mixture of nucleic acids which form a combinatorial library into contact with the human orthologue (H_{f2}) of L_{f2};
(b1) recovering a first subset S 1 of nucleic acids which do not bind to H_{f2}, in step (a);
(c1) bringing the first subset S 1 into contact with L_{f2};
(d1) recovering the nucleic acids which exhibit a high affinity with respect to L_{f2};
(e1) amplifying said nucleic acids with high affinity for L_{f2}, so as to obtain a mixture of nucleic acids, enriched in nucleic acids having a high affinity for said L_{f2}, and
(f1) identifying aptamers specific for L_{f2}, from the mixture obtained in (e).

This further elaboration of the method allows the selection of aptamers which have affinity to the Leishmania protein but not to the human orthologue. Such aptamers would be particularly advantageous as they would be expected to have little if any detrimental effects upon humans but to be very useful as diagnostic reagents and/or therapeutics against the *Leishmania* virulence factor.

Aptamers can be created by the combinatorial method for aptamer generation called SELEX (Systematic Evolution of Ligands by EXponential enrichment) (Tuerk and Gold, 1990, Ellington and Szostak, 1990).

Briefly, the principle of the SELEX method involves the selection, from a mixture of nucleic acids comprising random sequences, and by successive reiterations of binding, separation and amplification steps, of nucleic acid molecules (aptamers) exhibiting a defined binding affinity and a defined specificity for a given target. Thus, starting from a mixture of random nucleic acids, the SELEX method comprises more specifically the following steps:
- bringing the mixture of nucleic acids into contact with the target element (natural or synthetic polymers: proteins, polysaccharides, glycoproteins, hormones, receptors, antigens, antibodies, cell surfaces; small molecules: medicament, metabolites, cofactors, substrates, transition state analogs; tissues and in particular whole cells; viruses, etc.), under conditions which promote binding,
- separating the unbound sequences,
- dissociating the nucleic acid-target element complexes,
- amplifying the dissociated nucleic acids, so as to obtain a mixture enriched in ligands (aptamers), and
- repeating the binding, separation, dissociation and amplification steps, for the desired number of times.

High-affinity and high specificity aptamers are selected from a vast random sequence oligonucleotide library (normally 10¹⁴ initial molecules). Evolution is directed by the reduction of initial pool complexity during reiterative cycles of physical separation of target-binding sequences from inactive variants, followed by their reamplification. Aptamers can theoretically be developed to any target, from a pure single molecule to complex systems like a whole cell (K.N. Morris, et al 1998, M. Blank, et al, 2001, Daniels, et al, 2003, Cherchia et al, 2005). The binding of an aptamer strongly depends on the conformational state of its target (Tuerk and Gold 1990).

Recently subtractive SELEX has proved to be a useful tool in finding ligands to specific biological markers that distinguish a subtype of cells from cells of homologous origin. This strategy was used for the isolation of aptamers capable of distinguishing differentiated PC 12 cells from normal PC12 cells (Wang J et al. 2003), and tumour vasculature from those of the normal brain (Blank et al, 2001).

In the present disclosure *Leishmania* virulence factor Ln and *Leishmania* virulence factor L_{f2} can be the same or different molecules such as proteins or fragments thereof.

According to a further aspect of the present disclosure said L_{f1} or said L_{f2} comprises a nucleic acid fragment encoded by a sequence selected from the group: SEQ ID NO: 25, SEQ ID NO: 27, SEQ ID NO: 29, SEQ ID NO: 31, SEQ ID NO: 33; or a portion of any one thereof or wherein said L_{f1} or said L_{f2} comprises a peptide sequence selected from the group: SEQ ID NO: 26, SEQ ID NO: 28, SEQ ID NO: 30, SEQ ID NO: 32, SEQ ID NO: 34, or of any one thereof.

According to a fifth aspect of the present disclosure there is provided the use of an isolated or purified nucleic acid comprising a sequence selected from the group: SEQ ID NO: 25, SEQ ID NO: 27, SEQ ID NO: 29, SEQ ID NO: 31, SEQ ID NO: 33; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43 or a fragment thereof, for the manufacture of a medicament, diagnostic reagent or vaccine for the treatment and/or detection of *Leishmania* infection.

According to a sixth aspect of the present disclosure there is provided the use of an isolated or purified polypeptide comprising a sequence selected from the group: SEQ ID NO: 26, SEQ ID NO: 28, SEQ ID NO: 30, SEQ ID NO: 32, SEQ ID NO: 34; or a fragment thereof, for the manufacture of a medicament, diagnostic reagent or vaccine for the treatment and/or detection of *Leishmania* infection.

According to a seventh aspect of the present disclosure there is provided the use of an isolated or purified nucleic acid comprising a sequence selected from the group: SEQ ID NO: 25, SEQ ID NO: 29, or a fragment thereof; or an isolated or purified peptide comprising a sequence selected from the group: SEQ ID NO: 26, SEQ ID NO: 30, or a fragment thereof; for the detection of differentiated promastigotes of *Leishmania infantum.*

According to an eighth aspect of the present disclosure there is provided the use of an isolated or purified nucleic acid comprising a sequence selected from the group: SEQ ID NO: 27, or a fragment thereof; or an isolated or purified peptide comprising a sequence selected from the group: SEQ ID NO: 28, or a fragment thereof; for the detection of visceral parasites of *Leishmania infantum* and/or iscerisation of Leishmania infantum.

According to a ninth aspect of the present disclosure there is provided the use of an isolated or purified nucleic acid comprising a sequence selected from the group: SEQ ID NO: 31, SEQ ID NO: 33 or a fragment thereof; or an isolated or purified peptide comprising a sequence selected from the group: SEQ ID NO: 32, SEQ ID NO: 34 or a fragment thereof; for the detection of cutaneous parasites of *Leishmania infantum.*

According to a tenth aspect of the present disclosure, there is provided a diagnostic kit to perform a method of detecting differentiated promastigotes of *Leishmania infantum,* to detect visceral parasites of *Leishmania infantum* and/or viscerisation of *Leishmania infantum* or to detect cutaneous parasites of *Leishmania infantum,* the kit comprises:
- a substrate coated in at least one peptide selected from the group SEQ ID NO: 26, SEQ ID NO: 28, SEQ ID NO: 30, SEQ ID NO: 32, SEQ ID NO: 34;
- at least one anti-human immunoglobulin antibody;
- reagents to detect said at least one anti-human immunoglobulin antibody;
- instructions for use.

According to this aspect of the present disclosure there is provided a diagnostic kit for use with a patient, comprising a substrate coated in one of the peptide virulence factors identified by the inventors. The kit also comprises an anti-human immunoglobulin antibody such that following exposure of the substrate to serum antibodies or another antibodies source from the patient, any patient antibodies attached to the peptide coated substrate can be visualised using the reagents also included in this kit, allowing a diagnosis to be made.

For a better understanding of the invention and to show how the same may be carried into effect, there will now be shown by way of example only, specific embodiments, methods and processes according to the present invention with reference to the accompanying drawings in which:
**Fig. 1****.** RT-PCR of gene expression in PNA + and PNA - forms of visceral (LV 50) and cutaneous (Drep 14) *L. infantum* isolates.
**Fig. 2****.** shows an alignment of the protein sequences of E2-BCKD from various organisms.
**Fig. 3****.** shows an alignment of the protein sequences of MTAP from various organisms.
**Fig. 4****.** shows an alignment of the protein sequences of NR from various organisms.
**Fig. 5****.** shows an alignment of the protein sequences of LT7 from various organisms.
**Fig. 6****.** shows an alignment of the protein sequences of 517 from various organisms.
**Fig. 7****.** shows an alignment of the protein sequences of the hypothetical conserved protein LinJ35_V3.3420 and homologues from various organisms..
**Fig. 8****.** shows an alignment of the protein sequences of Dynein light chain protein and homolgues from various organisms..
**Fig. 9****.** shows an alignment of the protein sequences of the hypothetical protein LinJ05_V3.0820 and homologues from various organisms.
**Fig. 10****.** shows an alignment of the protein sequences of LAT Dihydrolipoamide branched chain transacyclase (LinJ05_V3.0180) and homologues from various organisms
**Fig. 11****.** Average parasitic density of isolates LV and LLC of *L*.
   *infantum* according to time.
**Fig. 12****.** Average densities parasitic (10⁶) of PNA- forms resulting from isolates LV and LLC of *L*. *infantum* according to time.
**Fig. 13****.** Average percentages of PNA - forms resulting from isolates LV and LLC of *L. infantum* according to time.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### Example 1. Material and methods

### 1.1. Parasite cultures

Two different *L. infantum* isolates from Tunisian VL and CL patients, selected from a cryobank on the basis of molecular studies (Barhoumi et al., unpublished results) were used: MHOM/TN/94/LV50 from a VL patient and MHOM/TN/96/DREP14 and from a CL patients, respectively that belong to the zymodemes MON-01 and MON-24, also described in other Mediterranean countries (Gramiccia, 2003; Pratlong et al., 2004; Baptista-Fernandes et al., 2007).

MHOM/TN/94/LV50 is a code issued by the World Health Organization (WHO). It indicates host/country of isolement/year of insulation/code of the isolate.

Upon thawing of the parasites, the parasites were cultivated on Coagulated Rabbit Serum (CRS) medium (Belkaid et al., 1996) at 26°C and were then transferred for mass culture to Schneider's Insect Medium (SIM) (Sigma) enriched with 10 % of Heat Inactivated Fetal Calf Serum (FCS) (GibcoBRL) supplemented with 2 mM L-Glutamine, 100 U/ml penicillin and 100 µg/ml streptomycin.

They were plated at a density of 3x10⁶ parasites/ml as PNA + parasites and collected at precise time points. The inventors focused on day 4-parasites, which correspond to the early stationary phase and where both isolates presented peaks of PNA - parasites.

The kinetics of growth was defined starting from the daily counting of parasitic density of the two isolates LV (LV49 and LV50) and two isolates LLC (DREP 05 and DREP 14) of *L. infantum.* The cultures were initiated with 3 x 10⁶ parasites/ml from the fourth day of culture where the parasites are in stage of active division. With an aim of homogenizing the parasitic cultures, a test PNA was carried out on these, during which PNA - forms were eliminated from the preparation and PNA+ forms were cultivated with the same starting density.

Thus, two sets of selection were conducted on these cultures. Initiated at 3 x 10⁶ parasites/ml of PNA+ forms, growth was followed by daily counting of the parasitic density in triplicate. In parallel, the density of PNA - forms as that of the PNA + forms was evaluated after a PNA test and daily counting.

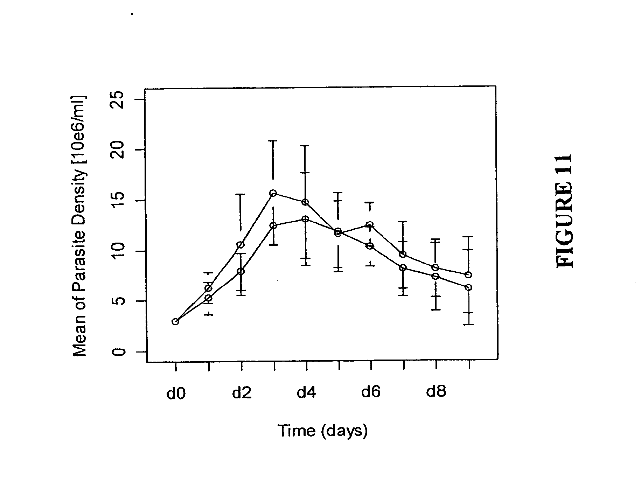

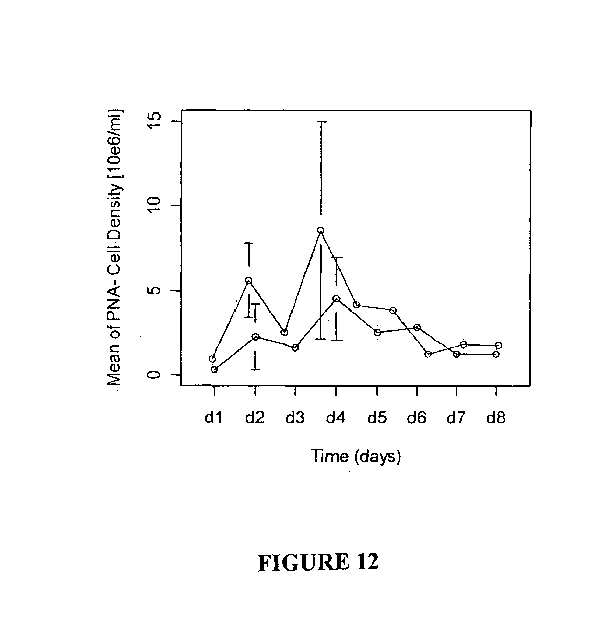

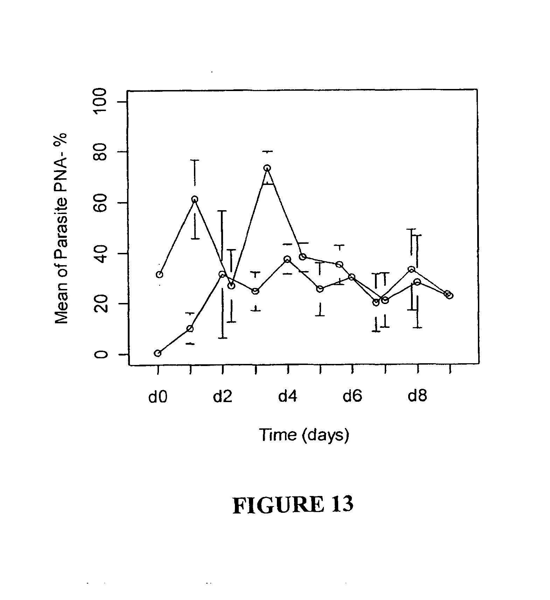

The results of this counting made it possible to plot the curve which translates the median value of the parasitic density of the 2 categories of isolates LV and LLC (Figure 11). The results of the counting of forms PNA- and PNA + of the two types of isolates also made it possible to plot the curves which translate the average density (Figure 12) as well as the percentages (Figure 13) of PNA - forms (PNA-/PNA- + PNA+) resulting from the 2 categories of isolates LV and LLC of *L*. *infantum.*

These curves show that the maximum of PNA - forms was reached at the fourth day of growth. Other peaks appear at days 2, 6 and 8. These peaks probably represent the sequential process of differentiation which generates the differentiated morphotypes described by Rogers et al., 2002 and Gossages et al., 2003.

To compare mRNA expression levels among the isolates at different developmental stages, the inventors reverse-transcribed and amplified (RT-PCR) total RNA extracted from PNA + and PNA - forms purified from stationary phase cultures of the isolates. Differentiated parasites were negatively selected by peanut agglutinin (PNA) as previously described (Louassini et al., 1998). Parasites were collected and resuspended at a density of 1- 2x10⁸/ml, with 50 µg/ml peanut agglutinin in SIM-FCS for 30 min at room temperature. Agglutinated PNA + forms were separated by centrifugation for 5 min at 1100 rpm in the pellet from the free, non-agglutinated PNA - cells in the supernatant. PNA - forms were recovered in the pellet after centrifugation at 3500 rpm for 10 min. Both parasite pellets were washed twice with phosphate buffered saline solution, immediately resuspended in Trizol^{™} reagent (Invitrogen) and stored at -80°C for further analysis.

### 1.2. Total RNA extraction, mRNA purification and cDNA synthesis

Total RNA was isolated using Trizol^{™} (Invitrogen) following the manufacturer instructions. 2x10⁸ *L. infantum* PNA + and PNA - promastigotes from early stationary phase were resuspended in 1 ml Trizol reagent. Extracted RNAs were treated with (40 UI) DNase I-RNase free (Boehringer Mannheim, Germany) in (6 mM MgCl2, 40 mM Tris-HCl pH 8, and 200 mM KCl) for 1 h at 37°C. The RNA was then phenol/chloroform extracted and DNAse treatment was assessed by PCR using "α-tubulin" primers.

No amplification was observed at this stage. The quantity of RNA was assessed using a gene-quant spectrophotometer (Amersham) and its quality by gel electrophoresis for the presence of the three distinct ribosomal bands (18S, 24Sa and 24Sβ). Messenger RNAs were then purified from the DNase I-treated RNA using mRNA purification kit according to the manufacturer's instructions (Amersham, Pharmacia Biotech) and used for cDNA synthesis. The reaction mixture contained 1 X Reverse Transcription (RT) buffer (Invitrogen), 40 UI RNasin (Promega) as RNase inhibitor, 200 µM each dNTP, 0,5 µg/µl of oligo (dT)₁₅ primer (Promega) and 200 UI/µl of SuperScript II^{™} Rnase H⁻ Reverse Transcriptase enzyme (Invitrogen) in a final volume of 20 µl. After 1 h incubation at 42°C, heating for 15 min at 70°C stopped the reactions. Quality of the synthesized cDNA was assessed using 1 µl to amplify an 800 bp fragment of the constitutively expressed α-tubulin gene in promastigotes using "α-tubulin" primers (see **Table I** for primer sequences). 10 µl of cDNAs were used to generate labelled expressed probes for library screening.

### 1.3. Screening of chromosome 5-enriched plasmid library

The screened library was constructed from a clone of the French isolate, LEM1317 (MHOM/FR/-82/LEM356-c11317) (Ravel et al., 1995a; Wincker et al., 1996; Wincker et al., 1997; Britto et al., 1998; Ravel et al.; 1999; Tamar et al., 2000) considered at the time of the study as a reference for defining linkage groups and chromosomal assignment of probes. This parasite clone was further characterized by the relative simplicity of its karyotype and the clear separation of chromosomes, particularly chromosome 5.

In brief, chromosome 5 was separated by PFGE on agarose gels, purified from the gels and randomly sheared by sonication into about 1 kb size fragments. The fragments were repaired and were blunt-end cloned into the EcoRV site of pBluescript II SK plasmids. The ligation products were transformed into competent XL1 Blue *E.coli* cells and the library was plated on LB agar medium containing 50 µg ml⁻¹ ampicillin, 50 µg ml⁻¹ X Gal and 100 mM IPTG for white/blue selection. 2400 recombinant clones obtained were transferred to 96 well microtiter plates to generate arrayed colonies on nylon filters in replicate, which were used for hybridization.

Following assumption of Akopyants et al., (2001), the inventors considered that 1/5^{th} of the library would be enough to cover the expression of chromosome 5 (500 kb). So, the cDNAs from two *L. infantum* isolates taken at logarithmic phase were labelled with [α-³²P] dCTP (3000 Ci/mmole, Amersham - Greece) with the Multiprime DNA labelling kit according to manufacturer's instructions (Amersham - Greece) and were used to probe 480 colonies (5 filters each). Hybridisation and washings were performed according to the manufacturer's instructions (Amersham) at 65°C.

Specifically hybridization was performed over night at 65°C in 6X SSC, 5X Denhart, 0,5 % SDS and 50 ng of sonicated single stranded salmon sperm DNA. Five post-hybridization washes were performed at 65°C; twice with 2X SSC for 15 min, then once for 30 min with successively 2X SSC, 0,1 % SDS; 1X SSC, 0,1 % SDS and finally 0,1X SSC, 0,1 % SDS. Filters were exposed to X ray sensitive films for autoradiography (X OMAT-LS and XOMAT AR5, Kodack) at - 80 °C.

### 1.4. Plasmid analysis

Positive colonies were identified and grown in LB-ampicilline medium. The plasmid DNAs were isolated according to the alkaline lysis method (Sambrook et al., 1989). A double enzymatic digestion using PstI and XhoI that have sites flanking the cloning EcoRV site, determined size of the inserts. When internal restriction sites were observed, the size of the insert was confirmed by PCR using the vector primers. 2 µg of each recombinant plasmid DNA were then sequenced using 80 pmol of either T3 or T7 primer with the *Big Dye TM Terminator Cycle Sequencing Ready Reaction* kit (Perkin Elmer) on ABI prism 377 automated DNA sequencer.

### 1.5. In silico. characterization

For nucleotide sequence homology searches, mapping and annotation of the insert sequences, the inventors used different bioinformatic programs and servers. Searches were done on BLAST at NCBI site (http://www.ncbi.nlm.nih.gov/blast/Blast.cgi) and Omniblast servers (http://www.sanger.ac.uk/projects/l.major/toolkit/blast-server.shtlm) on genedb database (http://www.genedb.org/). The inventors first predicted the location of the sequences on the *L. major* Friedlin chromosomes and with progress of *L. infantum* genome, actual positions were mapped using the artemis annotations of the chromosomes downloaded from the Sanger center website. The inventors also used the *Artemis Comparative Tool* (ACT) (Carver et al., 2005) to compare these sequences (L. *infantum* LEM 1317) with those in databases of *L. infantum* clone JPCM5 (MCAN/98/LLM-877) and *L. major* Friedlin MHOM/IL/81/Friedlin (LmjF). Sequence alignment was analysed by the clustal W program. The function of the predicted proteins was extracted from genedb database. The last update of *in silico* sequence analysis was done in September 2007 using the version 3 of *L. infantum* genome data.

### 1.6. Primers and PCR reactions

Two µg of total RNA were used for each 20 µl RT reaction. PCR reactions on cDNA were done in duplicate in 50 µl using Go Taq DNA polymerase (Promega, Biogene- Tunis). The constitutively expressed α-tubulin gene (LinJ13_V3.0330), and a gene preferentially expressed in metacyclic stage, Meta 1 (LinJ17_V3.0990) were used as controls for RT-PCR analysis. Eight genes corresponding to the first 6 tags analysed were selected to assess expression by RT-PCR in both isolates. Primers listed in Table I, were designed using the program genefisher (http://bibiserv.techfak.uni-bielefeld.de/genefisher/) based on the *L. major* gene sequences (the only ones then available).

**Table I. List of forward and reverse primer sets used for RT-PCR reactions.**

| | | | | | | | | |
|---|---|---|---|---|---|---|---|---|
| LinJ35_V3. 3420 | 15F5 | 3547F/R | 600 | 1398-1998 | | | 62 | 61 |
| LinJ05_V3. 0180 | 14B9 | LAT F/R | 500 | 313-813 | | | 64 | 64 |
| LinJ05_V3. 0170 | 14B9 | 517 F/R | 500 | 1292-1792 | | | 64 | 66 |
| LinJ05_V3. 0420 | 14D9 | 542 F/R | 600 | 49-649 | | | 64 | 64 |
| LinJ05_V3 0820 | 12D5 | 582 F/R | 600 | 1072-1672 | | | 64 | 64 |
| LinJ05_V3. 0660 | 19C5 | NR F/R | 700 | 152-852 | | | 63 | 63 |
| LinJ04_V3. 0080 | 12F8 | LT7 F/R | 627 | 14-641 | | | 60 | 60 |
| LinJ05_V3. 0830 | 12D5 | MAP F/R | 498 | 327-825 | | | 60 | 56 |
| LinJ13_V3. 0330 | α-Tubutin | TubF/R | 808 | 1-809 | | | 54 | 60 |
| LinJ17_V3. 0990 | Meta I | MetF/R | 319 | 16-335 | | | 57 | 55 |

Eight primer pairs (3547, LAT, 517, 542, 582, NR, LT7 and MAP) were designed to amplify sequences of a selection of 8 genes associated with expressed tags identified in this study; Primers Tub and met were designed within α-Tubulin gene that is constitutively expressed in *Leishmania* promastigotes, and Meta 1 gene that was shown to be preferentially expressed in metacyclic stage of different *Leishmania* species, respectively. Code of targeted genes was extracted from genedb Database of *L. infantum.* Product size was calculated according to primer positions. TmF and TmR correspond to melting temperature of forward and reverse primers, respectively.

The adequacy of the primers designed to amplify the targeted genes, was confirmed by sequence alignment at the nucleotide level. Few nucleotide differences were observed. Conservation of the spacing between the primers was also confirmed. Finally, the inventors verified by ePCR on *L. infantum* sequence database using a local bioinformatics resource LBTx ("*Leishmania* Bioinformatic's ToolboX" Chakroun et al., 2007), to ensure that only the targeted genes would be amplified by the selection of primer pairs.

Amplification of *L. infantum* DNA yielded products at the expected size with the respective primers. The 50 µl PCR reactions contained 1µl of cDNAs obtained from PNA+ or PNA- parasites, or 50 ng of genomic DNA (as control), 200 µM of each dNTP, 5X Go Taq buffer (Promega - Tunis), 30 pmol of forward and reverse primers and 1,5 Units of Go Taq DNA polymerase (Promega - Tunis).

After the initial denaturation for 3 min at 94°C, the cycle conditions were: 25X [1 min at 94°C, 30 S at 60°C, 2 min at 72°C] and 2 min final elongation at 72°C for primers pairs (3547, LAT, 517, 582, MAP and LT7); and 25X [1 min at 94°C, 30 S at 58°C, 2 min at 72°C] and 5 min final elongation at 72°C for the two remaining primer pairs (NR and 542).

α-tubulin was amplified as follows: after an initial 5 min at 94°C, 25X [1 min at 94°C, 30 S at 55°C, 1 min at 72°C] and 5 min final elongation at 72°C. For Meta 1 primers, the inventors used 2 min at 94°C, 25X [1 min at 94°C, 1 min at 50°C, 1 min at 72°C] and 5 min final elongation at 72°C.

To be able to analyse the PCR results before endpoint, all PCR reactions were run for 25 cycles. The PCR products were analysed by electrophoresis on 1.5 % agarose gels containing ethidium bromide (0.5 µg/ml). Analysis was conducted in duplicate as 2 sets of experiments using different cDNA products from the same parasite pellets. Each set of experiments included all PCR reactions. α-tubulin amplification was done multiple times to assure steady quality of the cDNAs during a set of assays.

### 1.7. Estimates of the signal intensities and statistical analysis

Polaroids taken from the agarose gels of all experiments were scanned and signal intensity was analysed using NIH image J program (version 1.24) (http://www.rsb.info.nih.gov/iii/). The relative peak areas for targeted and control α-tubulin genes were estimated following the program instructions and were used to represent the relative product yield. Student t-test was used to compare the logarithm of the adjusted band intensity between groups. No correction for multiple testing was used.

### Example 2. Results

### 2.1. Screening a chromosome 5- enriched genomic library with cDNAs from promastigotes of L. infantum isolates resulted in selection and sequencing of 23 inserts

Five filters of the library were hybridized with radiolabelled log-phase cDNAs from the VL and CL isolates under study. The autoradiography revealed 23 colonies as follows: 8 hybridized to the CL cDNA, 14 to the VL cDNA and one reacted with both. The mean size of the inserts that ranged from 500 to 1100 bp was 800 bp. Sequences were determined entirely or partially at one or both ends using plasmid primers and were analysed using bioinformatic tools and resources after purification from vector sequences.

Using Blast, sequence homologies were first searched for in the different public releases of *L. major* database and then in the *L. infantum* releases, as these occurred. All sequences searched generated hits. Homology percentage with sequences in databases ranged from 95 % to 100 % in case of *L. infantum* JPCM5 clone (LinJ) while it varied from 84 % to 96 % in case of *L. major* Friedlin (LmjF). The inventors decided to focus on the first six tags that were identified based on the early releases of the databases (Table II).

**Table II. Features of 6 expressed tags identified by the screening of a chromosome 5-enriched plasmid library using cDNA from promastigote cultures of 2 L. infantum isolates causing VL or CL.**

| **Insert ^{a}** | **Reacted with LV 50/DREP 14^{b}** | **Insert size (bp) ^{c}** | **Mapping** | | **Remarks^{f}** |
|---|---|---|---|---|---|
| | | | **Significant hit on chromosome^{d}** | **Tag position^{g} on *L. infantum^{e}*** | |
| 12D5 | +/- | 700 | 5 | 300667-301512 **LinJ05_V3.0830** | Sequence overlapping a CDS |
| 19C5 | -/+ | 800 | 5 | 221895-222514 | Intergenic sequence |
| 14B9 | -/+ | 600 | 5 | 53414-53852 | Intergenic sequence |
| 14D9 | -/+ | 1000 | 5 | 138503-138384 | Intergenic sequence |
| 12F8 | +/- | 800 | 4 | 30106-30971 **LinJ04_V3.0080** | Sequence overlapping a CDS |
| 15F5 | +/- | 1000 | 35 | 1373311-1373894 **LinJ35_V3.3420** | Sequence overlapping a CDS |

| | | | | | |
|---|---|---|---|---|---|
| Table II lists features relative to 6 tags identified by 6 inserts selected from the screening of a chromosome 5-enriched plasmid library with cDNAs from actively growing promastigotes of two *L. infantum* isolates, MHOM/TN/94/LV50 and MHOM/TN/96/DREP14, respectively obtained from VL and CL cases. ^{a} indicates the code of the plasmid colony. ^{b} summarizes results of the library screening with transcriptome probes of the two isolates LV50 (MHOM/TN/94/LV50) and DREP14 (MHOM/TN/96/DREP14), where (+) means positive signal and (-) means negative signal. ^{c} corresponds to the size of insert that was defined by double digestion and/or PCR. ^{d} indicates chromosome assignment by omniblast on *Leishmania* databases. Significance is defined as a percent of homology ranging between 80 % and 100 % and e-value ≤ 10⁻⁶. ^{e} corresponds to position on version 3 of *L. infantum* genome overlapped by sequence reads of the inserts. They are here considered as expressed genomic tags and were assigned the code of the corresponding inserts. When the tag overlaps a coding sequence, the gene identifier is indicated in bold. ^{f} annotation related to the genomic site of the tag. ^{g} the positions are defined compared to *L. infantum* clone JPCM5 (MCAN/98/LLM-877). | | | | | |

To validate this screening approach, a selection of 8 genes associated with 6 genomic expressed tags were used to analyse relative expression to α-tubulin by RT-PCR in PNA + and PNA - forms of the two isolates, which derived from early stationary phase, when PNA - counts were shown to peak for both isolates see example 1.1 above. Statistical analysis of the signal intensities observed in RT-PCR reactions at 25 cycles confirmed differences in expression in promastigote forms within and between isolates. This highlighted variability in gene expression among isolates. Polymorphic expression patterns were used to identify potential markers for differentiation, virulence or pathogenicity of *L. infantum.*

### 2.2. Homology- based analysis allowed assignment of the 6 inserts to 8 chromosomes and to at least 11 genomic sites

Besides homology percentage, the blast search analysis allowed chromosomal assignment of the inserts based on the annotation provided by the *Leishmania* genome projects. Interestingly, the 6 inserts mapped to a total of 3 chromosomes; 4 were located on chromosome 5, the remaining were on chromosomes 4 (N = 1) and 35 (N = 1) (Table II). 5 inserts corresponded to unique loci. In the case of 14D9, initial searches identified a tag on chromosome 5 but with the progress of genome projects, 5 additional significant hits were also obtained on chromosomes 36, 27, 1, 17 and 15 in order of increasing *p-value* (see Table III), bringing loci corresponding to the 6 inserts to a total of at least 11 on 8 chromosomes.

**Table III p-value of 14D9**

| **Tag^{a}** | **Position of tag^{b}** | **Chromosome^{c}** | **Percentage identity^{d}** | **e-value^{e}** | **Score hit^{f}** |
|---|---|---|---|---|---|
| 14D9 | 138503-138384 | 5 | 100.00 | 5.8e-063 | 238.4 |
| 14D9 | 1047672-1047791 | 36 | 99.17 | 1.4e-060 | 230.4 |
| 14D9 | 94124-94005 | 27 | 99.17 | 1.4e-060 | 230.4 |
| 14D9 | 60240-60359 | 1 | 98.33 | 3.4e-058 | 222.5 |
| 14D9 | 619727-619620 | 17 | 100.00 | 8.4e-056 | 214.6 |
| 14D9 | 331701-331818 | 15 | 98.32 | 3.3e-055 | 212.6 |

| | | | | | |
|---|---|---|---|---|---|
| ^{a}. Tag name. ^{b}. Position of tag 14D9 on the 6 chromosomes defined by comparison to *L. infantum* JPCM5 (MCAN/98/LLM-877). ^{c}. indicates the chromosomal localization of the tag 14D9. ^{d}. indicates the percentage identity of the tag 14D9 with the sequences of *L. infantum* JPCM5 (MCAN/98/LLM-877). ^{e}. indicates the statistical score non-dependent upon the size of the hit which gives a measure of similarity. ^{f}. indicates the statistical zcore dependent on the size of the hit and giving a measure of similarity. | | | | | |

The tags on chromosomes 5, 4 and 35 were further characterized as regards annotation provided by the *L. major* and *L. infantum* genome projects. Tags 12D5, 12F8 and 15F5 were found overlapping coding sequences (CDS) encoding for a methylthioadenosine phosphorylase (LinJ05_V3.0830) and for hypothetical conserved proteins (LinJ04_V3.0080 and LinJ35_V3.3420), respectively. The remaining tags mapped within intergenic positions (19C5, 14B9 and 14D9) on chromosome 5 between an upstream and a downstream genes, all transcribed from the same strand (Tables II and IV).

**Table IV. List of genes associated to a selection of 6 expressed tags.**

| **Tags** | **Upstream gene** | **CDS overlapped by the tag** | **Downstream gene** |
|---|---|---|---|
| | **LinJ05_V3.0820** | **LinJ05_V3.0830** | **LinJ05_V3.0840** |
| **12D5** | Hypothetical conserved protein (582) | Methylthioadenosine phosphorylase (MAP) | Hypothetical protein |
| | **LinJ05_V3.0650** | | **LinJ05_V3.0660** |
| **19C5** | Hypothetical conserved protein | - | Nitroreductase-like protein (NR) |
| | **LinJ05_V3.0170** | | **LinJ05_V3.0180** |
| **14B9** | Hypothetical conserved protein (517) | - | Dihydrolipoamide branched chain transacyclase (LAT) |
| | **LinJ05_V3.0420** | | **LinJ05_V3.0430** |
| **14D9** | Dynein light chain protein (542) | - | Hypothetical conserved protein |
| | **LinJ04_V3.0070** | **LinJ04_V3.0080** | **LinJ04_V3.0090** |
| **12F8** | DNA Topoisomerase IB-type small sub-unit | Hypothetical conserved protein (LT7) | Hypothetical conserved protein |
| | **LinJ35_V3.3430** | **LinJ35.3420** | **LinJ35_V3.3410** |
| **15F5** | Hypothetical conserved protein | Hypothetical conserved protein (3547) | Hypothetical conserved protein |

Genomic environment of the tags was deduced from annotations on version 3 of public release of *L. infantum* genome. Three tags were found within coding sequences while the others were intergenic. Genes upstream and downstream the tags in the transcription units are listed on the Table IV. Of the 15 genes listed, 8 were randomly selected for RT-PCR expression analysis. Code of corresponding primers used in RT-PCR experiments appears in parenthesis.

### 2.3. GO analysis showed that most genes associated to the tags encode for enzymatic activities implicated in different metabolic or cellular pathways

In total, 15 genes were associated to the 6 tags. This includes genes overlapping the tags and genes identified upstream and downstream in the transcription units. The inventors considered their gene ontology (GO) annotation as defined by *L. major* genedb taking into account biological process, cellular component and molecular function. Furthermore, assignment to super families based on presence of domains encoded by the genes was also considered (Table V).

**Table V. Gene ontology annotations of the 15 genes associated to the 6 tags deduced from gene db.**

| **Gene product** | **Identifier** | **Biological process** | **Cellular component** | **Molecular function** | **Super family domain** |
|---|---|---|---|---|---|
| Hypothetical protein, conserved | LinJ05_V3.0820 | | | | |
| Methylthioadenosine phosphorylase, Putative | LinJ05_V3.0830 | | | S-methyl-5-thioadenosine phosphorylase activity | |
| | | Amino acid salvage | Cytoplasm | GO : 0017061 | Purine and uridine phosphorylases |
| | | GO: 0043102 | GO : 0005737 | transferase activity, transferring pentosyl groups | SSF53167 |
| | | | | GO: 0016763 | |
| Hypothetical protein Unknown function | LinJ05_V3.0840 | | | | |
| Hypothetical protein, conserved | LinJ05_V3.0650 | Biological process | | | |
| | | GO : 0008150 | Cellular_component | Protein binding GO: 0005515 | RNI-like |
| | | Proteolysis | GO : 0005775 | Subtilase activity GO: 0004289 | SSF52047 |
| | | GO: 0006508 | | | |
| Nitroreductase-like protein | LinJ05_V3.0660 | Electron transport | Cellular_component | Oxidoreductase activity acting on NADH or NADPH | FMN-dependent nitroreductase-like |
| | | GO: 0006118 | GO : 0005575 | nitrogenous group as acceptor | SSF55469 |
| | | | | GO: 0016491 | |
| hypothetical conserved protein | LinJ05_V3.0170 | | | | |
| | | acetyl-CoA biosynthetic process | Mitochondrial alpha-ketoglutarate | Acyltransferase activity GO: 0008415 | Single hybrid motif |
| | | | | | SSF51230 |
| Dihydrolipoamide branched chain | LinJ05_V3.0180 | GO: 0006085 | dehydrogenase complex | Alpha-ketoacid dehydrogenase | Peripheral subunit-bindingdomain of |
| transacylase, putative | EC 2.3.1.- | Metabolic process | GO : 0005947 Mitochondrion | activity GO : 0003826 | |
| | | GO: 0008152 | | protein binding | 2-oxo aciddehydrogenase complex |
| | | | GO : 0005739 | GO: 0005515 | |
| | | | | | SSF47005 |
| Dynein-light chain-protein, putative | LinJ05_V3.0420 | Microtubule-based process | Microtubule associated complex | Microtubule motor activity | |
| | | GO: 0007017 | GO: 0005875 | GO: 0003777 | |
| Hypothetical protein, conserved | LinJ05_V3.0430 | | | | Metallo-dependent phosphatases |
| | | | | | SSF56300 |
| DNA Topo-isomerase IB-type small | LinJ04_V3.0070 | DNA topological change | Chromosome | DNA binding GO: 0003677 | DNA breaking-rejoining enzymes |
| subunit | EC 5.99.1.2 | GO: 0006265 | GO: 0005694 | DNA topoisomerase type 1 activity GO: 0003917 | SSF56349 |
| Hypothetical conserved protein | LinJ04_V3.0080 | | | | S-adenosyl-L-methionine-dependent |
| | | | | | methyltransferases |
| | | | | | SSF53335 |
| Hypothetical conserved protein | LinJ04_V3.0090 | | | | |
| Hypothetical conserved protein | LinJ3S_V3.3430 | mRNA capping | | mRNA (nucleoside-2'-o) methyltransferaseactivity | S-adenosyl-L-methionine-dependent |
| | | GO: 0006370 | | | |
| | | mRNA processing | | | methyltransferases |
| | | | | GO: 0004483 | SSF53335 |
| | | GO: 0006397 | | | |
| Hypothetical conserved protein | LinJ35_V3.3420 | | | ATP binding GO: 0005524 | |
| | | | | Helicase activity GO: 0004386 | |
| | | ATP synthesis coupled proton | Proton-transporting two-sector | Hydrogen ion transporting ATP synthase activity, | |
| | | transport | ATPase complex | rotational mechanism GO: 0046933 | |
| | | GO: 0015986 | GO: 0016469 | | |
| | | | | Hydrogen ion transporting ATPase activity, rotational | |
| | | | | Mechanism GO: 0046961 | |
| Hypothetical conserved protein | LinJ35_V3.3410 | | | | ARM repeat SSF48371 |

Table V lists GO (Gene Ontology) annotation provided by genedb for corresponding genes in *L. major,* as regards biological process, cellular component, molecular function and attribution to super family. In 8 cases, the GO annotation linked the genes to biological processes like amino acid salvage (GO: 0043102), proteolysis (GO: 0006508), electron transport (GO: 0006118), ATP synthesis coupled proton transport (GO: 0015986), acetyl CoA biosynthetic process (GO: 0006085), microtubule- based process (GO: 007017), mRNA capping (GO: 0006370), and DNA topological change (GO: 0006265). In addition for 3 other genes, the predicted proteins had domains assigning them to super families of metallo-dependent phosphatases (SSF56300), S-adenosyl-L- methionine dependent transferases (SSF53335) or ARM repeat (SSF48371). Finally, no annotation was provided in case of the remaining 4 genes. Of the 15 genes, 10 are identified as hypothetical proteins with or without GO annotation.

In total, 9 genes encode for enzymes or proteins bearing enzymatic domains. Of particular interest, gene LinJ05_V3.0180 (tag 14B9) which encodes for a dihydrolipoamide branched chain transacylase has been described as under positive selection in a three *Leishmania* species comparative genomic analysis (Peacock et al., 2007). Two genes belong to the S-adenosyl-L- methionine dependent transferases super family (SSF53335) but are likely involved in different molecular functions (LinJ04_V3.0080 and LinJ35_V3.3430).

### 2.4. Differential expression of 8 genes associated to the tags in PNA + and PNA-promastigote forms in L. infantum isolates

Expression of 8 randomly selected genes associated with the 6 tags was assessed in duplicate experiments by RT-PCR in the two isolates DREP 14 and LV 50, in PNA + and PNA - parasites taken at the early stationary phase when PNA - counts peaked. The genes overlap tags, or correspond to upstream or downstream CDS in the transcription units (Table IV).

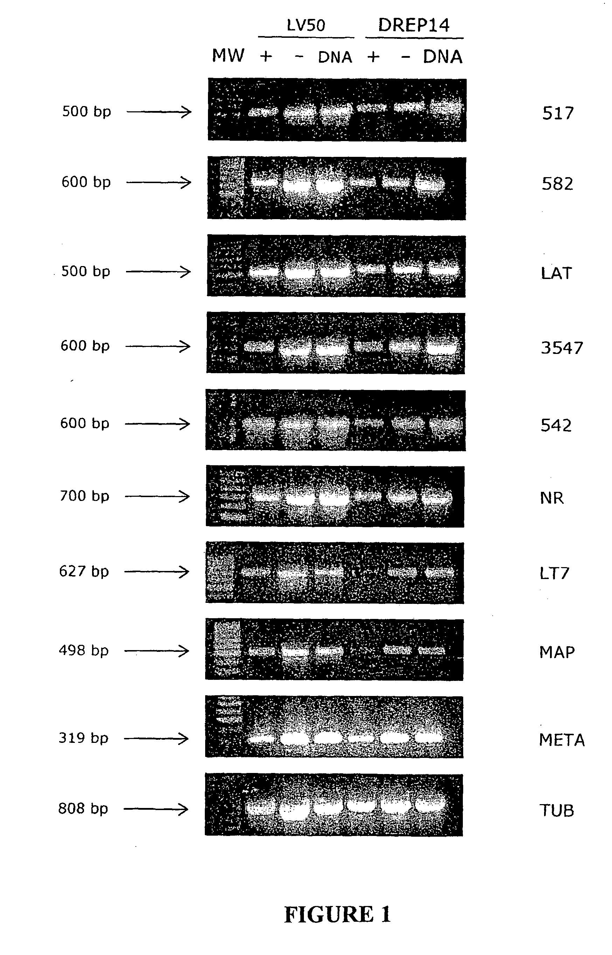

They encode for proteins having unknown functions (LinJ05_V3.0170, LinJ05_V3.0820), for a Dynein light protein (LinJ05_V3.0420), for enzymes like methylthioadenosine phosphorylase (LinJ05_V3.0830), a potential nitroreductase (LinJ05_V3.0660), a dihydrolipoamide branched chain transacylase (LinJ05_V3.0180), an enzyme involved in ATP synthesis coupled proton transport (LinJ35_V3.3420), or for a protein assigned to S-adenosyl- L- methionine- dependent methyltransferases super family (LinJ04_V3.0080) (Table IV and VII). The study also assessed expression of Meta 1, a gene preferentially expressed in metacyclics of different *Leishmania* species (Uliana et al., 1999). As seen in figure 1, the primers developed amplified efficiently and in a comparable way the DNAs of both isolates yielding products at the expected sizes. However, differences in intensities were observed for the different cDNAs indicating variations in gene expression levels in association with differentiation and/or according to the isolates (Figure 1).

In figure 1 RT-PCR analyses were performed using comparable amounts of cDNAs synthesized from promastigotes taken at early stationary phase of promastigote cultures of isolates LV50 and DREP14. Lanes (+), (-) and (DNA) correspond to PNA + cDNA, PNA - cDNA and genomic DNA of the isolates. Panels A to H correspond to amplification products of transcripts or genes LinJ05_V3.0170, LinJ05_V3.0820, LinJ05_V3.0180, LinJ35_V3.3420, LinJ05_V3.0420, LinJ05_V3.0660, LinJ04_V3.0080 and LinJ05_V3.0830 amplified by primer pairs 517, 582, LAT, 3547, 542, NR, LT7 and MAP, respectively. Control amplifications for constitutive expression in promastigotes (α tubulin gene) is represented on panel I. Panel J corresponds to amplification of Meta 1 products described in previous studies as preferentially expressed in metacyclics. Genomic DNAs of the isolates constituted the internal controls of amplification.

### 2.5. Statistical analysis reveals different mRNA expression patterns between and within L. infantum isolates and allows selection of candidate virulence genes.

With the hypothesis that the amplification at 25 cycles did not reach end point, and as target DNA was amplified similarly in both isolates, the inventors measured and compared the signal intensities observed for the PNA - and PNA + cDNAs relative to the constitutively expressed α-tubulin gene in promastigotes (Purdy et al., 2005a; Srividya et al., 2007) (Table VI).

**Table VI.** Measured band intensity of targeted and control α-tubulin genes obtained by RT-PCR of mRNA of visceral (LV 50) and cutaneous (DREP 14) *L. infantum* PNA+ and PNA- stages.

| Code of target genes^{a} | LV50 (+)^{b} | LV50 (-)^{c} | drep 14 (+)^{d} | drep14 (-)^{e} |
|---|---|---|---|---|
| 517 (1) | 10,18 | 21,16 | 7,3 | 9,35 |
| 517 (2) | 12,87 | 22,79 | 4,57 | 12,3 |
| 517 (3) | 12,36 | 19,46 | 8,31 | 13,19 |
| 517 (4) | 12,68 | 19,66 | 6,67 | 13,35 |
| 542 (1) | 16,23 | 20,84 | 5,43 | 17,39 |
| 542 (2) | 13,99 | 20,84 | 6,37 | 18,88 |
| 542 (3) | 16,04 | 23,48 | 19,42 | 13,73 |
| 542 (4) | 17,12 | 24,81 | 18,05 | 12,71 |
| 582 (1) | 10,97 | 20,47 | 4,98 | 10,86 |
| 582 (2) | 10,11 | 14,84 | 7,76 | 14,79 |
| 582 (3) | 11,71 | 14,28 | 6,81 | 13,56 |
| 582 (4) | 13,5 | 15,84 | 7,12 | 14,67 |
| NR (1) | 22,08 | 16,11 | 4,14 | 15,11 |
| NR (2) | 15,28 | 15,73 | 4,99 | 18,04 |
| NR (3) | 20,45 | 16,06 | 4,76 | 15,96 |
| NR (4) | 14,1 | 15,77 | 5,88 | 17,2 |
| LAT (1) | 17,23 | 17,98 | 6,58 | 16,66 |
| LAT (2) | 16,84 | 15,34 | 10,19 | 16,98 |
| LAT (3) | 19,03 | 17,96 | 6,59 | 15,82 |
| LAT (4) | 14,81 | 15,27 | 10,85 | 17,03 |
| 3547 (1) | 3,72 | 36,91 | 3,17 | 7,96 |
| 3547 (2) | 14,33 | 19,35 | 6,16 | 13,49 |
| 3547 (3) | 3,99 | 35,75 | 2,89 | 7,66 |
| 3547 (4) | 14,99 | 19,71 | 5,58 | 13,17 |
| LT7 (1) | 7,19 | 25,98 | 3,32 | 19,25 |
| LT7 (2) | 9,6 | 27,06 | 2,02 | 18,08 |
| LT7 (3) | 7,77 | 26,39 | 3,84 | 19,51 |
| LT7 (4) | 10,06 | 24,71 | 2,34 | 18,53 |
| MAP (1) | 6,08 | 26,27 | 2,88 | 20,09 |
| MAP (2) | 10,3 | 27,68 | 3,68 | 20,64 |
| MAP (3) | 6,67 | 25,47 | 3,01 | 20,25 |
| MAP (4) | 9,36 | 29,76 | 2,56 | 20,34 |
| Meta (1) | 14,1 | 20 | 10,72 | 22,84 |
| Meta (2) | 14,04 | 19,9 | 10,34 | 18,55 |
| Meta (3) | 12,11 | 20,54 | 10,31 | 23,79 |
| Meta (4) | 14,31 | 20,44 | 10,24 | 18,53 |
| Tub (1) | 17,91 | 22,08 | 13,65 | 15,2 |
| Tub (2) | 17,5 | 21,23 | 13,01 | 15,65 |
| Tub (3) | 11,34 | 17,44 | 15,99 | 18,8 |
| Tub (4) | 14,31 | 19,69 | 13,85 | 19,65 |
| Tub (5) | 16,47 | 22,17 | 14,25 | 15,55 |
| Tub (6) | 18,96 | 19,7 | 12,47 | 15,59 |
| Tub (7) | 12,2 | 18,56 | 14,79 | 18,88 |
| Tub (8) | 15,3 | 19,95 | 13,22 | 19,14 |

| | | | | |
|---|---|---|---|---|
| ^{a}517, 542, 582, NR, LAT, 3547, LT7, MAP and Meta 1 correspond to code of primers that amplified the following genes (see Table I), LinJ05_V3.0170, LinJ05_V3.0420, LinJ05_V3.0820, LinJ05_V3.0660, LinJ05_V3.0180, LinJ35.3420, LinJ04_V3.0080 and LinJ17_V3.0990, respectively. Tub is the code primer of the internal control corresponding to α-tubulin gene that is constitutively expressed in *Leishmania* promastigotes. Expression analysis was done as 2 sets of RT- PCR amplification, each including all genes; while expression of all genes was tested once during each set, transcripts of α-tubulin gene were amplified twice at the beginning and at the end to assess steady quality of the cDNA over the time of experiments. Two sets of measures were taken for each amplification by two different readers: (1-4 for Tub; 1-2 for the rest) for the first reader and (5-8 for Tub; 3-4 for the rest) for the second. ^{b, c, d, e} indicate band intensities measured by NIH image J program of target and control genes obtained by RT-PCR analysis of PNA+ (+) and PNA- (-) promastigote stages of visceral (LV 50) and cutaneous (DREP 14) *L. infantum* isolates. | | | | |

Interestingly, of the 9 genes evaluated, 8 were shown to have differential expression either between PNA + or PNA - promastigotes of the same isolate or among isolates for each form (Table VII).

**Table VII. Statistical analysis of stage- and isolate- wise gene expression levels in LV 50 and DREP 14 isolates and in PNA + and PNA - promastigote stages.**

| ***Primers Code*** | ***Gene identifier*** | ***Gene encoding for*** | *LV 50 PNA (+)*/*PNA* (-) | | *DREP 14 PNA (+)*/*PNA* (-) | | *PNA* (+) *LV 50*/ *DREP 14* | | PNA (-) LV 50/ DREP 14 | |
|---|---|---|---|---|---|---|---|---|---|---|
| | | | **Log (relative Expression) ±Standard Error *(p-value)*** | **Expression phenotype** | **Log (relative Expression) ±Standard Error *(p-value)*** | **Expression phenotype** | **Log (relative Expression) ±Standard Error *(p-value)*** | **Expression phenotype** | **Log (relative Expression) ±Standard Error *(p-value)*** | **Expression phenotype** |
| 517 | LinJ05_V3. 0170 | Hypothetical conserved protein | 0.2801382±0 1831 1 (0.0955) | -- | 0.38384±0 2644 2 (0.0657) | -- | 0.5±0.532 (**0.046**) | Up | 0.399±0.234 (**0.0058**) | Up |
| 542 | LinJ05_V3. 0420 | Dynein light chain protein | -0.081398±0.088 (0.432) | -- | 0.17371±0.8736 (0.647) | -- | 0.31±0.6777 (0.412) | -- | 0.21799±0.337 (0.1337) | -- |
| 582 | LinJ05_V3. LinJ05_V3. 0820 | Hypothetical protein | -0.0727±0.25149 (0.5756) | -- | -0.49453±0.112 (**0.0016**) | Down | 0.45995±0.1666 9 (**0.0123**) | Up | 0.03813±0.22 (0.65) | -- |
| 3547 | LinJ35_V3. 3420 | Hypothetical conserved protein | -0.996646±1.2276 (0 0886) | -- | 0.66999±0.1495 (**0.0134**) | Down | 0.47967±0.6 (0.3732) | -- | 0.8±0.4776 (**0.0035**) | Up |
| LAT | LinJ05_V3. | Dihydrolipoamide 0180 branched chain (0.0044) Up transacyclase | 0.2882±0.131271 (**0.0044**) | Up | -0.477±0.32268 (**0.0088**) | Down | 0.61±0.194 (**0.0035**) | Up | -0.1551±0.0832 (**0.0236**) | Down |
| LT7 | LinJ04_V3. 0080 | Hypothetical conserved protein | -0.84181±0.2665 (**0.0024**) | Down | -1.696459±0.2 (**0.0001**) | Down | 1.02±0.664 (**0.0051**) | Up | 0.169936±0.226 (0.1) | -- |
| NR | LinJ05 V3. 0660 | Nitroreductase-like protein | 0.372734±0.12233 (**0.0003**) | Up | -1.0±0.1635 (**0**) | Down | 1.1822±0.2119 (**0**) | Up | -0.1916±0.10 (0.0031) | Down |
| MAP | LinJ05 V3. 0830 | Methylthioadenosine phosphorylase | -0.96839±0.3013 (**0.0047)** | Down | -1.6968±0.151 (**0**) | Down | 0.868±0.4422 (0.0077) | Up | 0.1396±0.2412 (0.1542) | -- |
| Meta 1 | 0990 | LinJ17_V3. Meta 1 protein | -0.1270±0.172 (0.319) | -- | -0.4779±0.1979 (**0.0107**) | down | 0.1698±0.2787 (**0.194**) | -- | -0.1811±0.3194 (**0.218**) | -- |

Expression of the genes relative to α-tubulin was evaluated by analysing the signal intensities using NIH image J program. The relative intensities were transformed in log values and were statistically compared between PNA + and PNA - forms for each *L. infantum* isolate (stage-wise comparison) and between the two isolates for PNA + or PNA - forms (isolate-wise comparison)." Up", "down" or "- -" indicate over-, under-, or steady expression respectively. For instance in PNA +/PNA - column, "down" indicates that the gene is under-expressed in PNA+ as compared to PNA-.

The comparison of PNA + and PNA - parasites of the same isolate showed significant over expression of 7 out of 9 genes in PNA - parasites of DREP 14 (CL) isolate; while in the case of the LV 50 (VL) isolate, only 4 of these showed differential expression: 2 (LinJ04_V3.0080 and LinJ05_V3.0830) being over expressed in the PNA - parasites and 2 (LinJ05_V3.0180 and LinJ05_V3.0660) over expressed in PNA + parasites (**Table**VII). For each isolate, no differences between expression in PNA - and PNA + parasites were observed for the two genes (LinJ05_V3.0170 and LinJ05_V3.0420). Interestingly, difference in expression of Meta 1 gene was observed in PNA + and PNA - parasites of DREP 14, but not in case of LV 50 isolate.

The pairwise comparison of PNA + parasites showed up-regulation in 6 out of the 8 genes assessed in the VL isolate as compared to the CL. However, in case of isolate- wise comparison of PNA - parasites, 2 genes appeared up-regulated in the VL isolate (LinJ05_V3.0170 and LinJ35_V3.3420) and 2 others in the CL one (LinJ05_V3.0180 and LinJ05_V3.0660). No statistical differences were observed for the other genes in these forms. This highlights a variable gene expression not only when parasites undergo differentiation but also among isolates.

Considering up, down or steady expression phenotypes observed by stage and isolate, 7 different expression patterns could be defined. In pattern (A), genes LinJ04_V3.0080 and LinJ05_V3.0830 (encoding for methylthioadenosine phosphorylase and a hypothetical conserved protein assigned to S-adenosyl- L-methionine dependent methyltransferase superfamily, respectively), the inventors had comparable over-expression in PNA- forms of the 2 isolates, while expression is stronger in PNA+ forms of VL isolate than in the CL ones. In another pattern (B), gene LinJ05_V3.0170 coding for a conserved hypothetical protein appears more expressed in PNA - and in PNA + forms of the VL isolate than in the CL one with comparable expression levels between stages within each isolate. In pattern (C), 2 genes were more expressed in PNA - forms of the CL isolate than in the VL ones. Interestingly, in the VL isolate these genes have relative over- expression in the PNA + forms, while in the CL isolate they are over expressed in the PNA - forms. They encode for a dihydrolipoamide branched chain transacylase (LinJ05_V3.0660) and a nitroreductase- like protein (LinJ05_V3.0180), respectively.

In Pattern D (LinJ05_V3.0420) correspond to a gene that is constitutively expressed in the promastigote stages of both isolates.

In pattern E (LinJ05_V3.0820) and F (LinJ35_V3.3420) differences are observed between PNA + or PNA- stages of the isolates, while in pattern G (LinJ17_V3.0990) the only difference observed relates to a difference in expression of the gene of DREP14.Finally, 4 genes (LinJ05_V3.0820 & LinJ05_V3.0830, and LinJ05_V3.170 & LinJ05_V3.180) map to the same transcription unit of chromosome 5 and correspond to two pairs of tandem genes, the expression patterns observed did not show any physical correlation confirming previous observations (Saxena et al., 2003; 2007).

In total, there is a clear difference in expression patterns of the selection of genes under study between and within the two isolates in PNA + and PNA - forms. The variable expression patterns highlighted here could reflect important intrinsic differences in biological processes like parasite differentiation, virulence, or pathogenicity.

### 2.6 Identification in silico of inhibitors/activators targeting unique areas of the target molecule.

Starting from the alignment of the peptide sequences of the various genes identified and resulting from parasites of the family of trypanosomatides and the vertebrate host (*Homo sapiens*) and while taking as a starting point the degree of homology, it is now possible to identify the insertion points and/or deletions between the various sequences.

On this basis, a new strategy of targeting related but distinct parasite proteins identified as virulence factors which carry deletions and/or insertions has been adopted with the aim of developing new anti-pathogenic molecules.

To validate this approach, of identifying or designing molecules of which bind selectively to protein of the parasite without affecting its human orthologue a range of techniques involving the modeling *"in silico"* of target proteins and the design methods using computer-assisted drug design, the inventors wish to develop selective inhibitors/activators of parasite virulence factors without affecting the endogenous orthologue.

A comparison of the Leishmania, Trypanosome and Mammalian protein sequences for the putative virulence factors identified is shown below in Table VIII, IX and X below.

**Table VIII. Uniprot code and percentage homology for the different protein sequences of the putative virulence factors identified in the present invention.**

| **Organism** | **Protein LT7 (LinJ04_V3.0080)** | | | **Protein 517 (LinJ05_V3.0170)** | | | **Methylthioadenosine phosphorylase (MTAP)** | | | **Dihydrolipoamide branched chain transacylase (E2 BCKD)** | | | **Nitroreductase (NR)** | | |
|---|---|---|---|---|---|---|---|---|---|---|---|---|---|---|---|
| | **Uniprot Code** | **Homology* (Uniprot)** | **Homology* (NCBI)** | **Uniprot Code** | **Homology (Uniprot)** | **Homology (NCBI)** | **Uniprot Code** | **Homology (Uniprot)** | **Homology (NCBI)** | **Uniprot Code** | **Homology (Uniprot)** | **Homology (NCBI)** | **Uniprot Code** | **Homology (Uniprot)** | **Homolog y (NCBI)** |
| *L. infantum* | A4HS03 | 100 % | 100 % | A4HSD2 | 51 % | 100 % | A4HSK5 | 100% | 100 % | Q4QJ15 | 76 % | Not found | A4HS188 | 96% | 100 % |
| | A4IC32 | 22% | | | | | | | | | | | | | |
| | A4HSN2 | 21 % | | | | | | | | | | | | | |
| | Q7JQ59 | 26% | | | | | | | | | | | | | |
| *L.donovani* | Q27426 | 26% | Not found | Not found | Not found | Not found | Not found | Not found | Not found | Not found | Not found | Not found | Not found | Not found | Not found |
| *L. major* | Q95Z88 | 93 % | 93 % | Q4QJ16 | 49 % | 87 % | Q4GJB9 | 96% | 97 % | A4H464 | 71% | 93 % | Q4QJD6 | 91 % | 94 % |
| | Q4FVV9 | 22% | | | | | | | | | | | | | |
| | Q4QJ92 | 21 % | | | | | | | | | | | | | |
| *L. braziliensis* | A4H3S8 | 86% | 86 % | A4H463 | 34 % | 68 % | A4H4C5 | 83 % | 84 % | Q4D8Z1 Q4DDM3 | 40 % | 81% | A4H4BO | 79 % | 81 % |
| | A4H4F5 | 22% | | | | | | | | | 39 % | | | | |
| *T. cruzi* | Q4DUC7 | 45 % | 45 % | Not found | Not found | 35 % | Q4DZW1 Q7YWE5 Q4DQ97 | 60% | 60% | Q57Z16 | 38 % | 40-41 % | Q4DCW9 Q4D8D9 | 56% | 57% |
| | | 23 % | | | | | | 60% | | | | | | | |
| | Q4DZX7 | | | | | | | 60% | | | | | | | |
| T. *brucet* | Q38EP1 | 45 % | 45 % | Not found | Not found | 39 % | Q57XS4 | 58 % | 59 % | Q57Z16 | 38 % | Not found | Q57XU3 | 57 % | 50 % |
| *Homo sapiens* | O60344-4 | 26% | 24-31 % (endothelin converting enzym 2) | Not found | Not found | Not found | Q6FHT1 | Not found | Not found | P11182 Q5VVL8 Q5VVL7 | | 40% | Not found | Not found | Not found |
| | A8MUP2 | 24 % | | | | | | | | | 38 % | | | | |
| | Q8N6RO-1 | 25 % | | | | | | | | | 38 % | | | | |
| | A8K6S5 | 25 % | | | | | | | | | 36 % | | | | |
| | Q8N6RO | 25 % | | | | | | | | | | | | | |
| | Q8N6RO-4 | 25 % | | | | | | | | | | | | | |
| *Mus musculus* | Q80260-3 | 28 % | 26-28 % (endothelin converting enzym 2) | Not found | Not found | Not found | Q9CQ65 | Not found | Not found | Q3TMF5 Q7TND9 P53395 | | 39-40 % | Not found | Not found | Not found |
| | Q80260-4 | 26 % | | | | | | | | | | | | | |
| | Q80Z60 | 28 % | | | | | | | | | 38 % | | | | |
| | Q80Z60-2 | 28 % | | | | | | | | | 37 % | | | | |
| | Q91YR5 | 25% | | | | | | | | | 37 % | | | | |
| | Q3TRF3 | 25 % | | | | | | | | | | | | | |
| | Q91YR5-1 | 25% | | | | | | | | | | | | | |
| | Q91YR5-2 | 25% | | | | | | | | | | | | | |
| *Rattus norvegicus* | Not found | Not found | 25 % | Not found | Not found | Not found | Q7TP15 | Not found | Not found | Not found | Not found | 39 % | Not found | Not found | Not found |

| | | | | | | | | | | | | | | | |
|---|---|---|---|---|---|---|---|---|---|---|---|---|---|---|---|
| * corresponds to the percentage of homology defined compared to the sequence published of protein in question. The data and the codes of proteins in Table VIII, of each quoted organization come from the Uniprot databases and NCBI. The percentages of homology were given compared to the protein sequence of L. infantum by means of the Blast program using the UNIPROT and NCBI databases. The absence of homology was checked using Blastp on site NCBI databases NR. The absence of homology was confirmed in addition by aligning by means of the program Clustal X the sequences of the 2 proteins NR and MTAP of L. infantum to those annotated as such at the Human one. | | | | | | | | | | | | | | | |

**Table IX- Code and function of the various fields present identified by Interpro, pfam and prosite.**

| **Protein** | **Interpro** | **pfam** | ***Prosite*** |
|---|---|---|---|
| Methylthioadénosine phosphorylase (MTAP) | IPR010044 : methylthioadenosine phosphorylase | PF00896 : phosphorylase family 2 | *PS01240 : purine and other phosphorylase family 2 signature* |
| | IPR001369 : purine phosphorylase, family 2 | | |
| Dihydrolipoamide branched chain transacylase (E2-BCKD) | IPR003016 : 2-oxo acid dehydrogenase, lipoyl binding site | PF02817 : e3 binding domain | *PS00189 : 2-oxo acid dehydrogenoses* |
| | IPR004167: E3 binding | PF00198 : 2-oxo acid dehydrogenases acyltransferase (catalytic domain) | |
| | IPR000089 : biotinlipoyl attachment | | *acyltransferase* |
| | IPR001078 : catalytic domain of components of various dehydrogenase complexes | PF00364 : biotin requiring enzyme | *component lipoyl binding site* |
| | IPR011057 : 2-oxo acid dehydrogenase acyltransferase component lipoyl binding site | | |
| *Nitroreductase like-protein (NR)* | *IPR000415* : *nitroreductase* | *PF00881 : nitroreductase family* | - |

| | | | |
|---|---|---|---|
| All the data concerning the code of the various fields and the significance of each one were collected from genedb (HTTP: /www.genedb.org). | | | |

**Table X Code, position, sequence and function of the various fields identified by Prosite and HMM library.**

| **Protein** | **Code Prosite** | **Position** | **Sequence in *L. infantum*** | **Function or domain** |
|---|---|---|---|---|
| Methylthioadenosine phosphorvlase (MTAP) | PS01240 | 58-98 | LPRHGPHHQYNPSEINYRAN.ICALKQMGVRY ILAINAVGSL (SEQ ID NO 21) | purine and other phosphorylase family 2 signature |
| Dihydrolipoamide branched chain transacylase (E2-BCKD) | PS50968 | 48-121 | | Biotinyl_lipoyl |
| | PS00189 | 72-101 | | 2-oxo acid dehydrogenases acyltransferase component lipoyl binding site |
| Nitroreductase (NR) | | 93-296 | | Nitroreductase |
| LT7 (LinJ04_V3.0080) | SSF53337 (HMM library) | 11-189 | | Appartenance à la superfamille des S-adenosyl-L-methionine acetyltransferase |

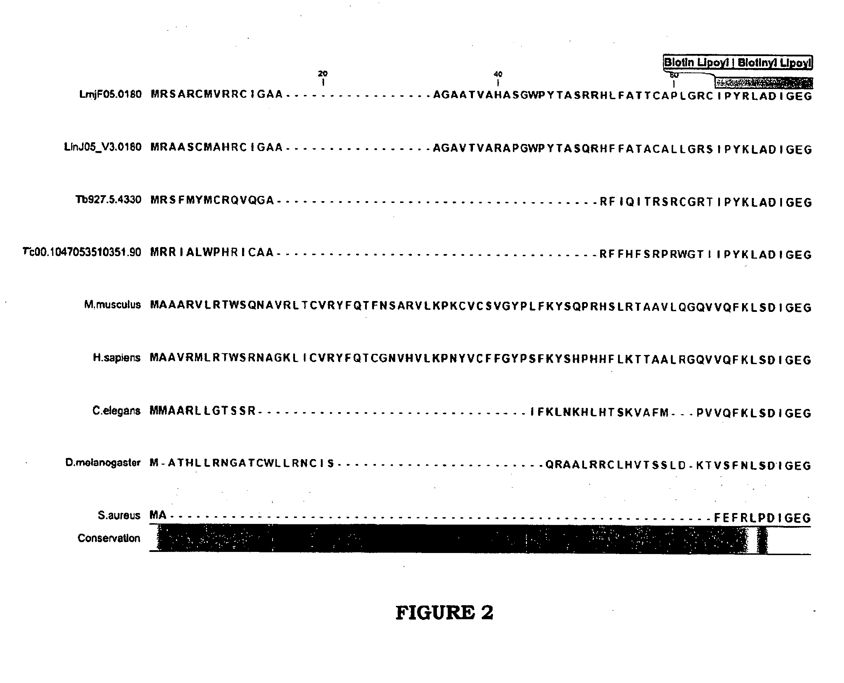

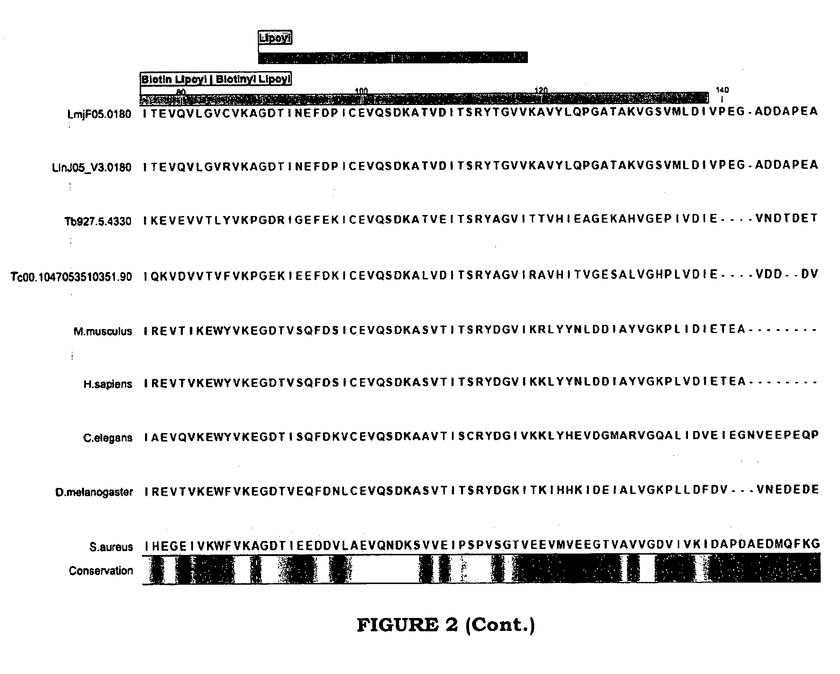

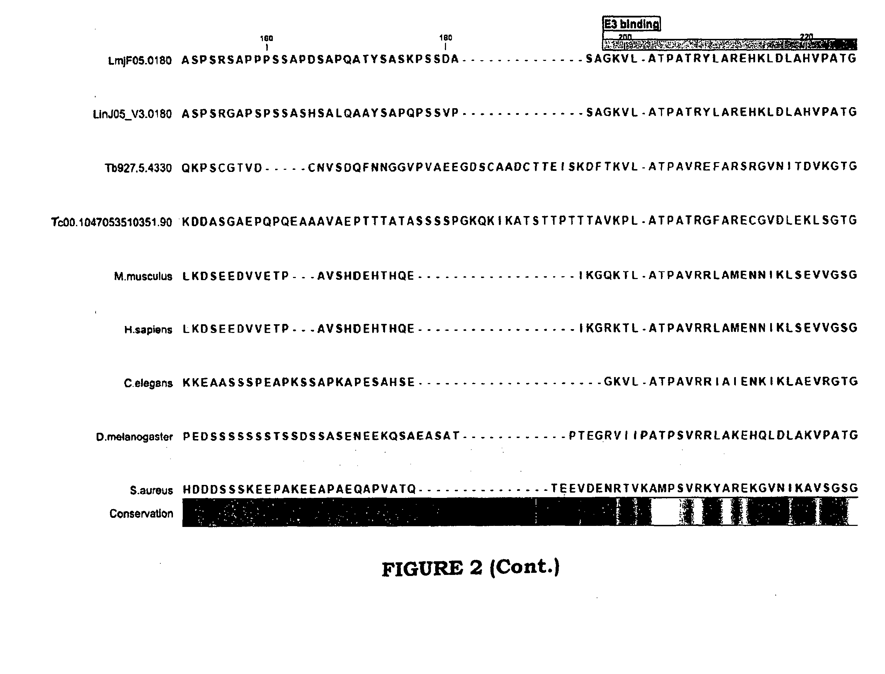

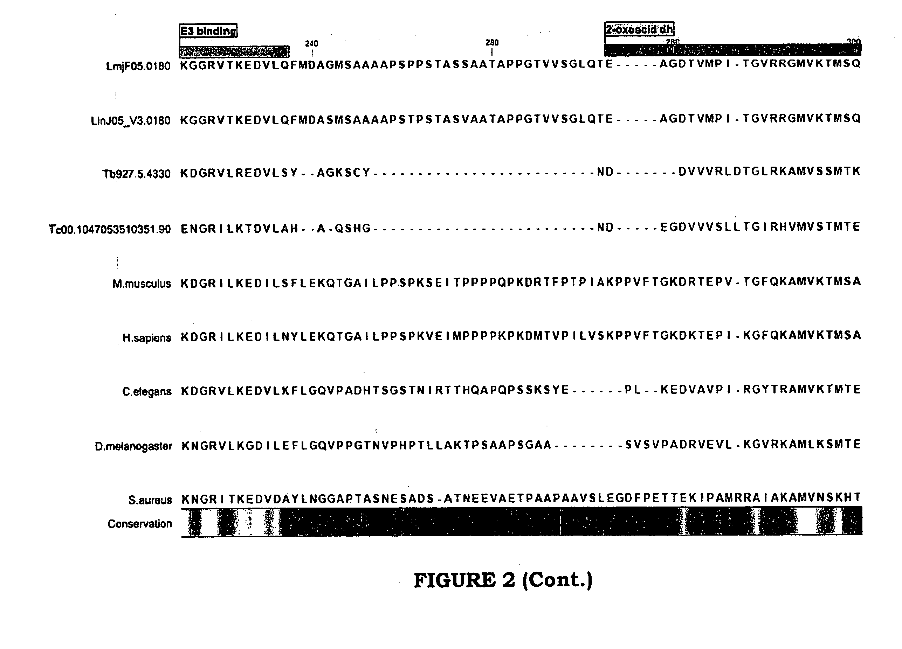

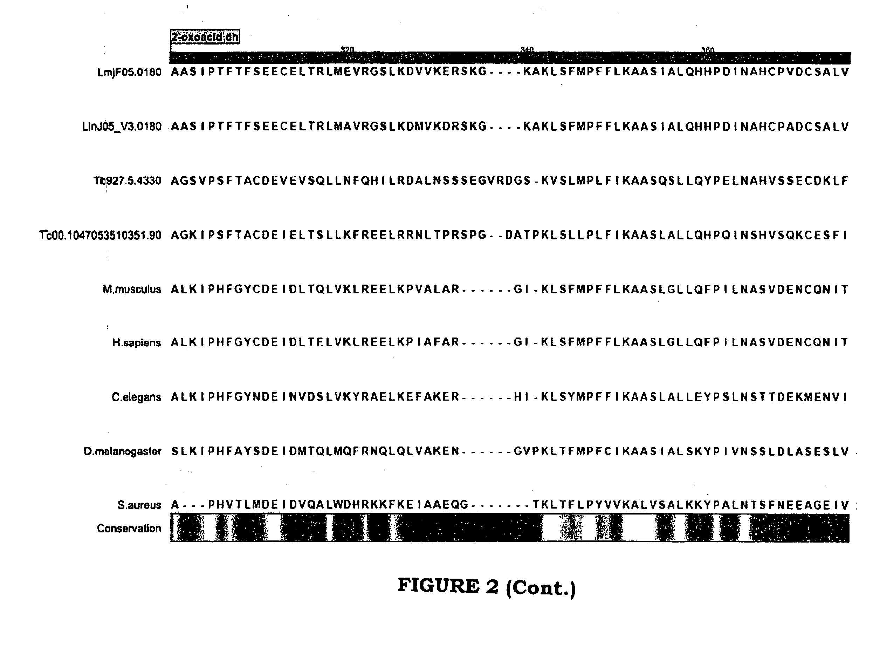

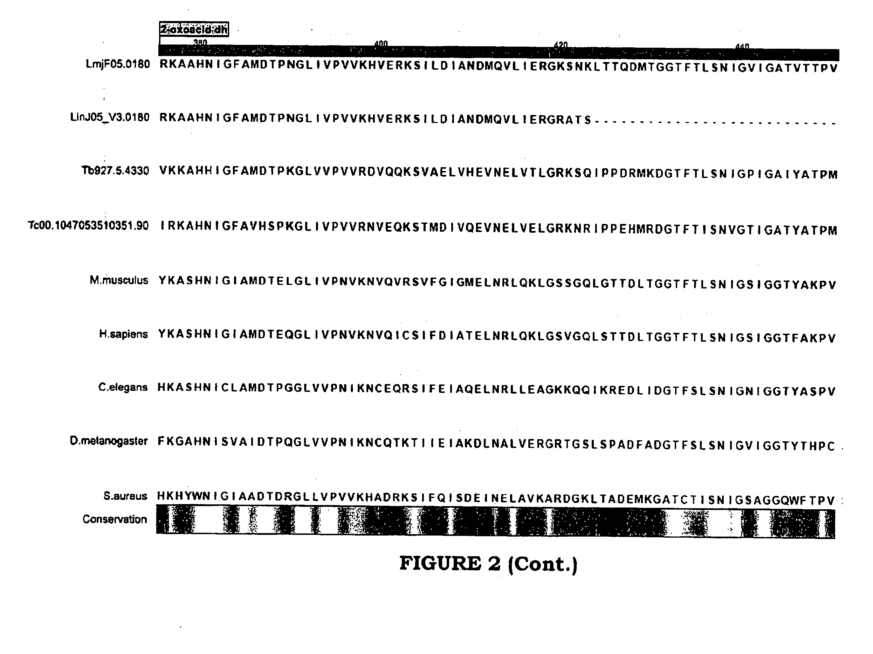

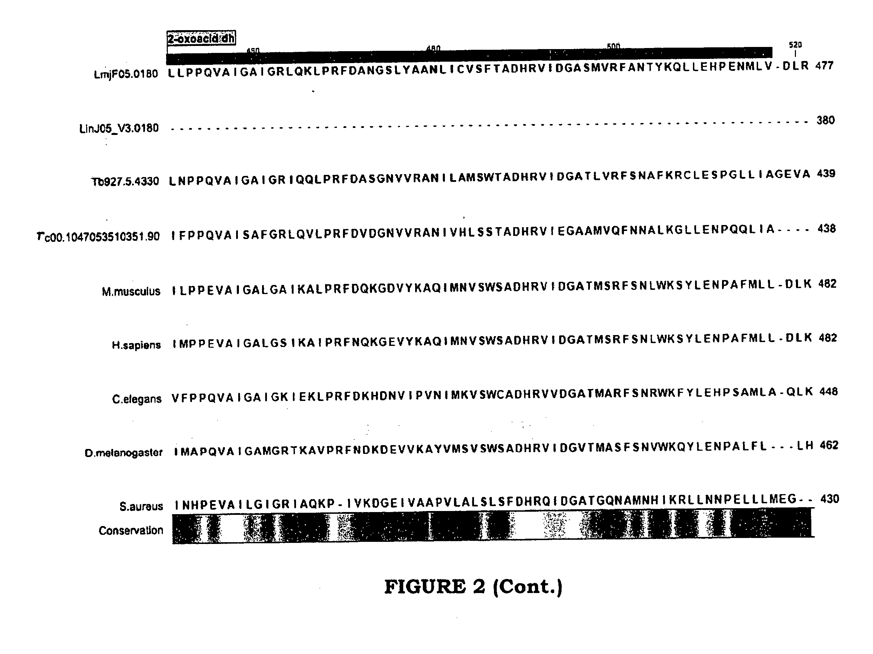

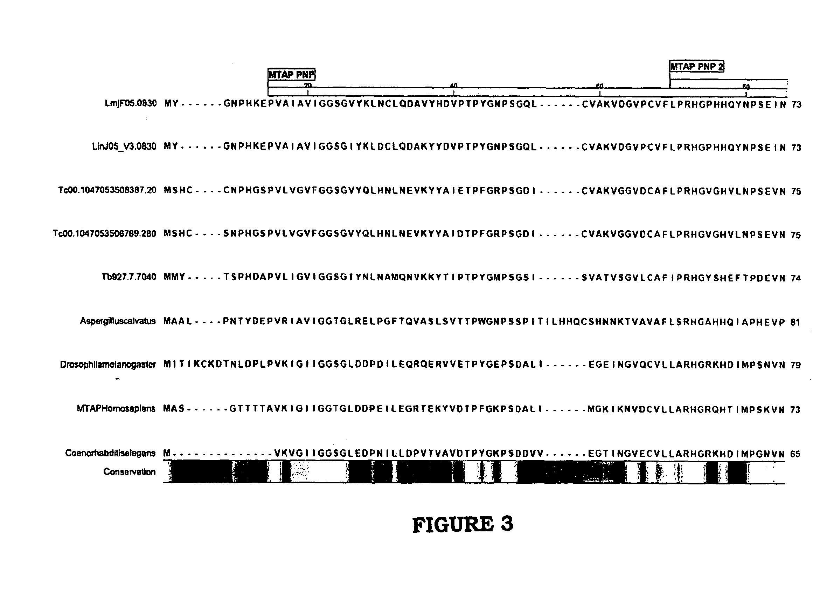

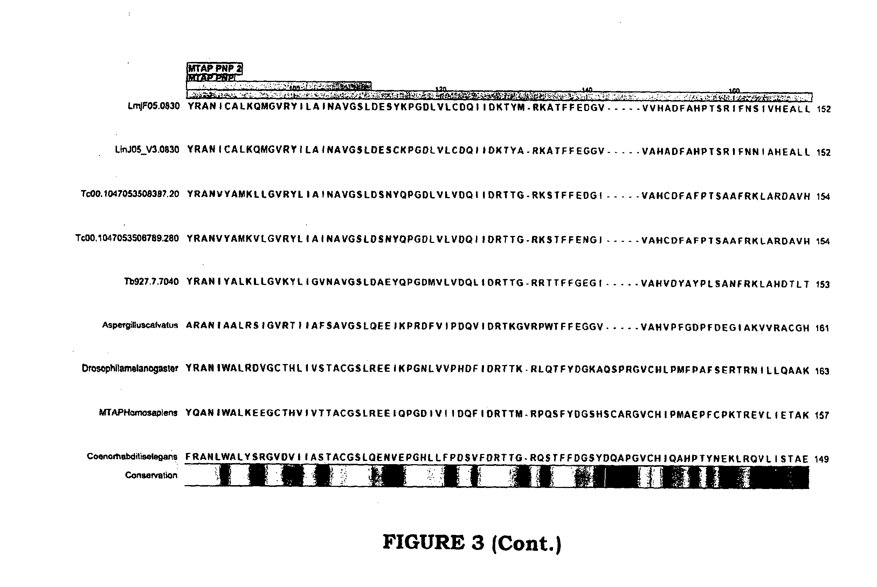

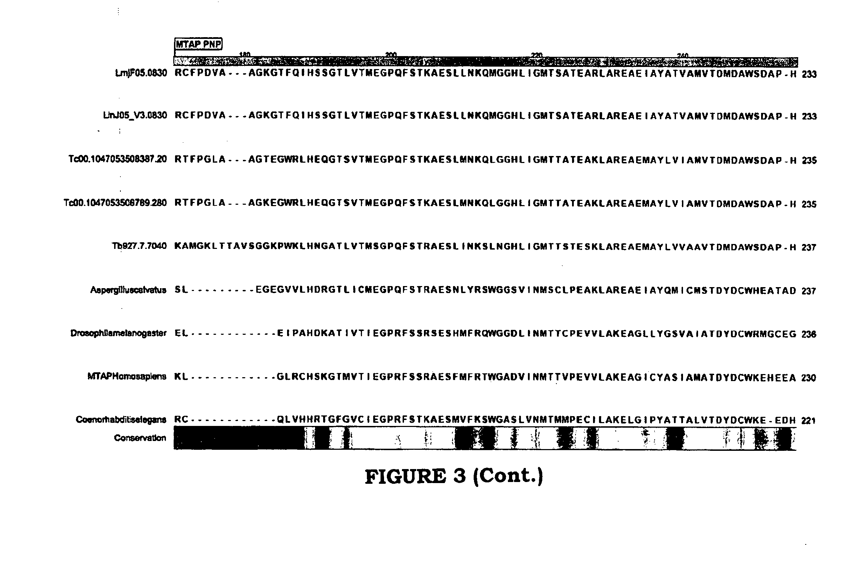

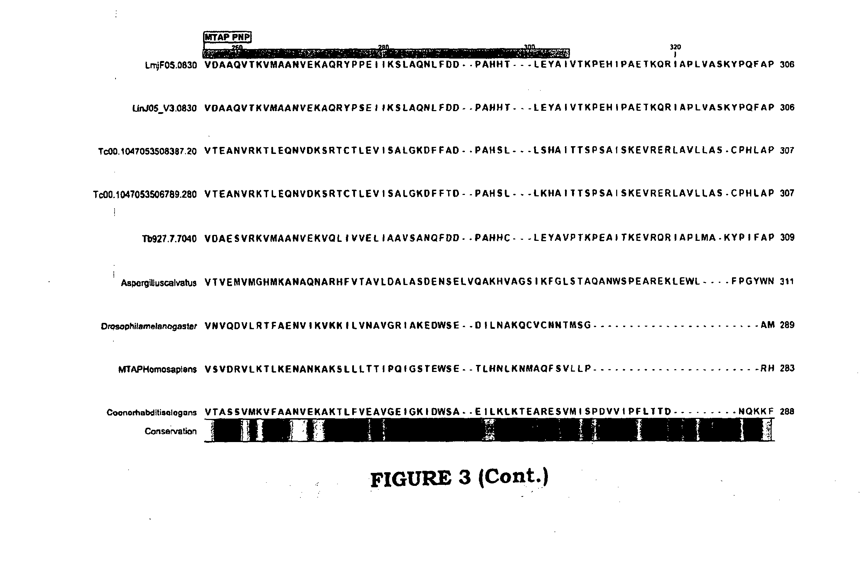

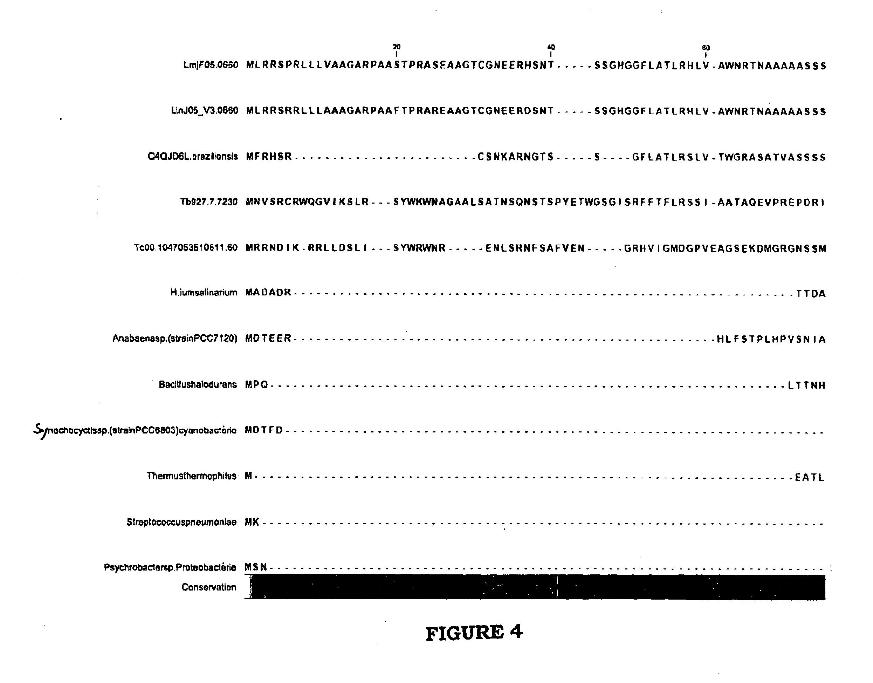

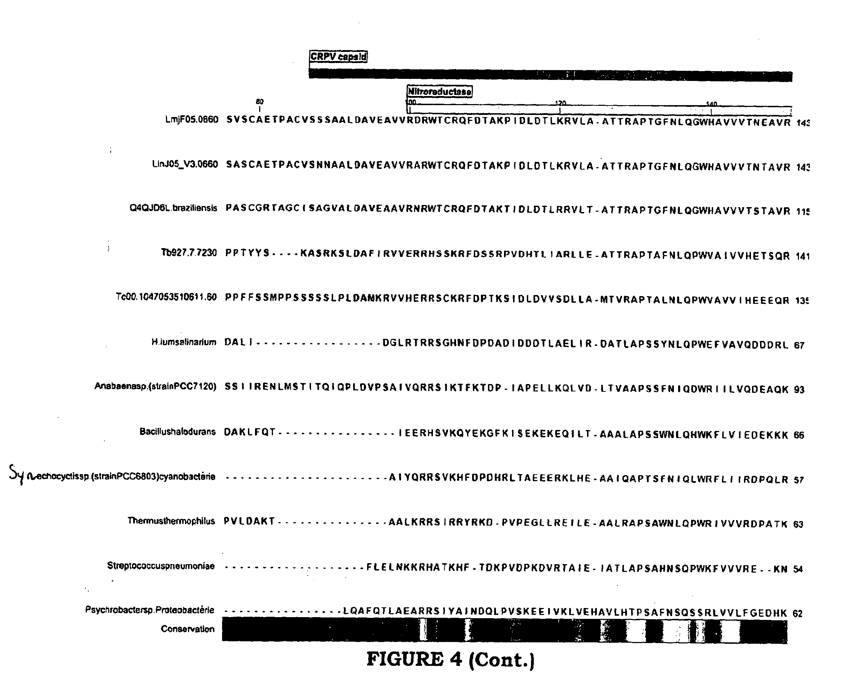

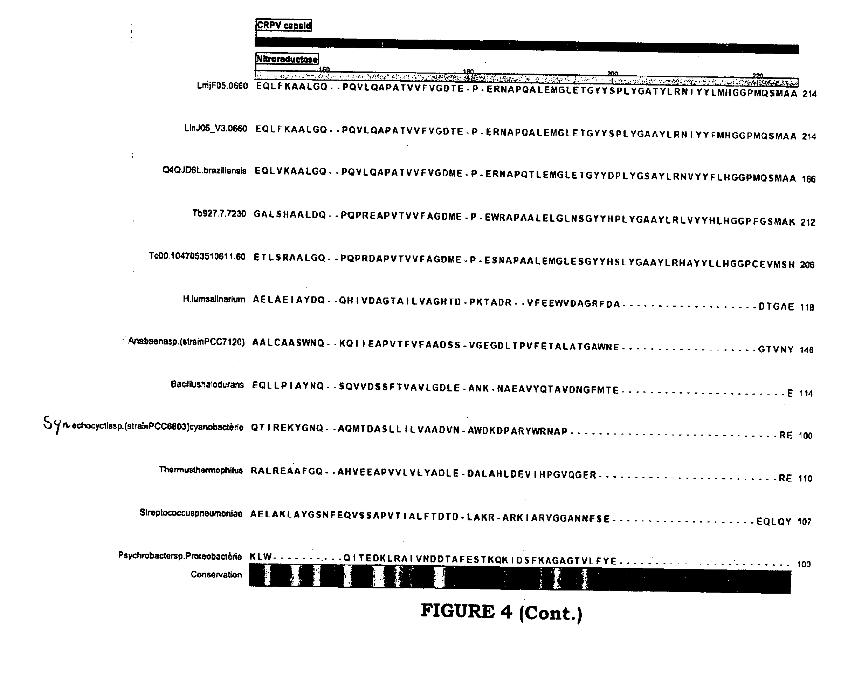

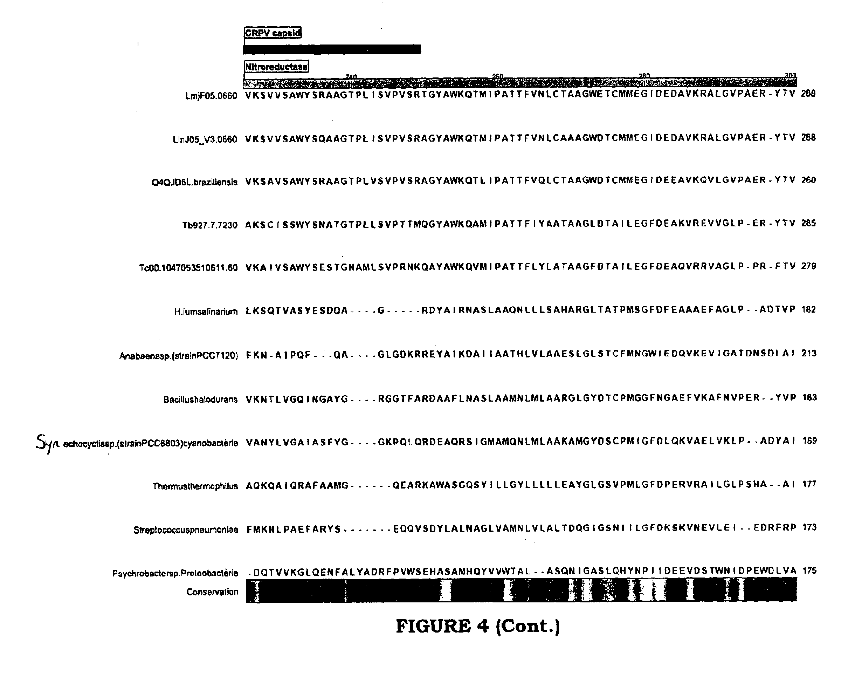

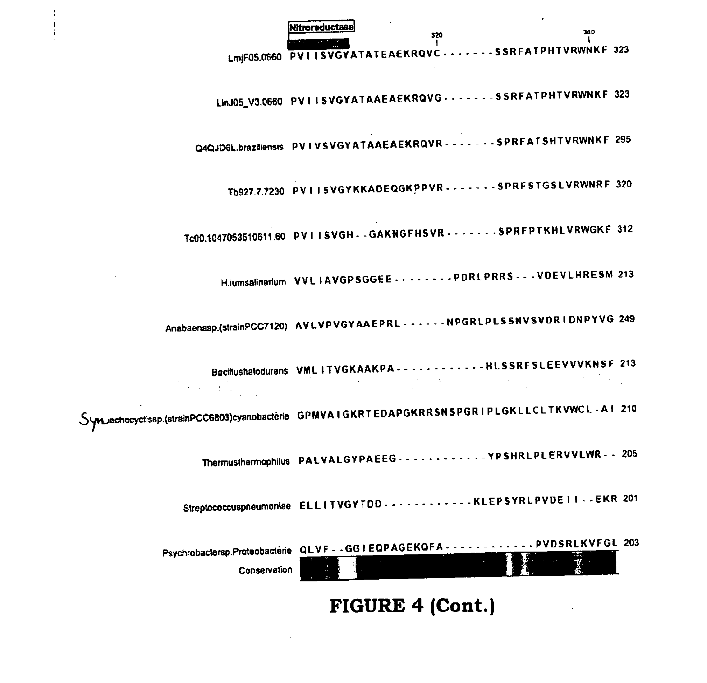

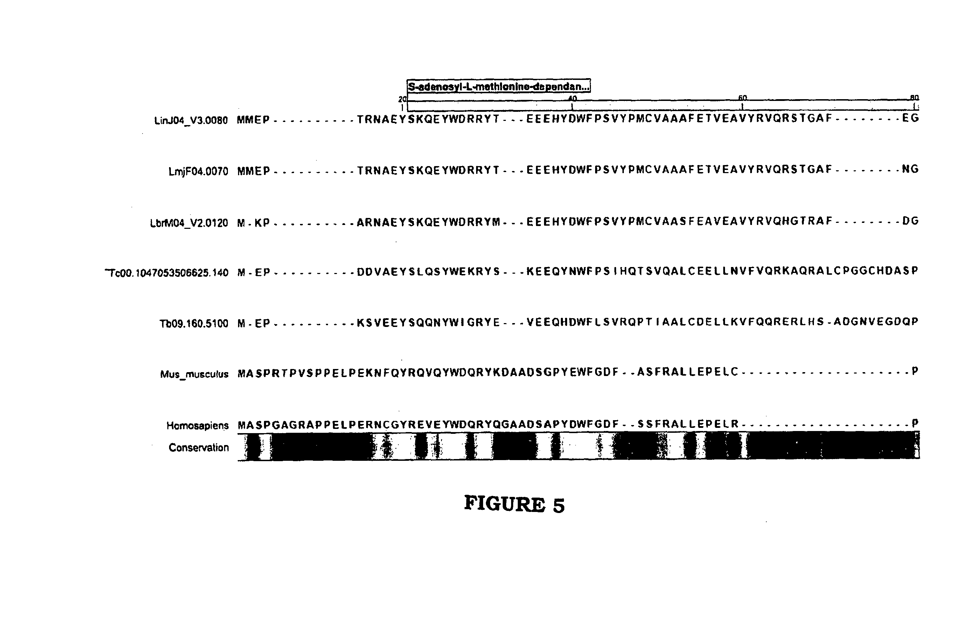

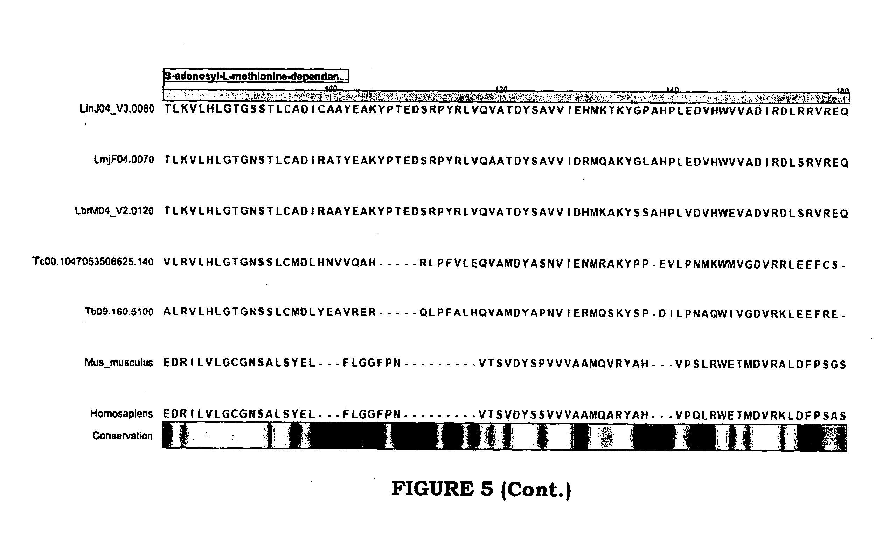

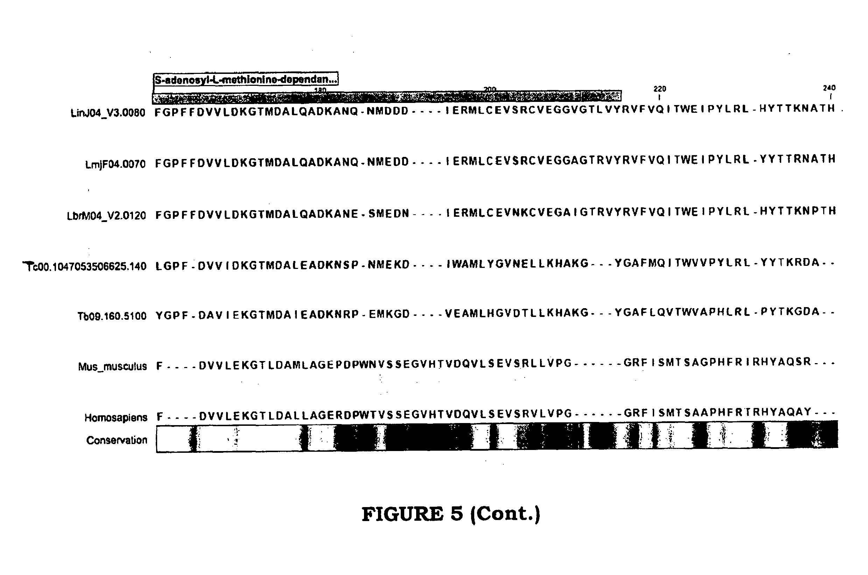

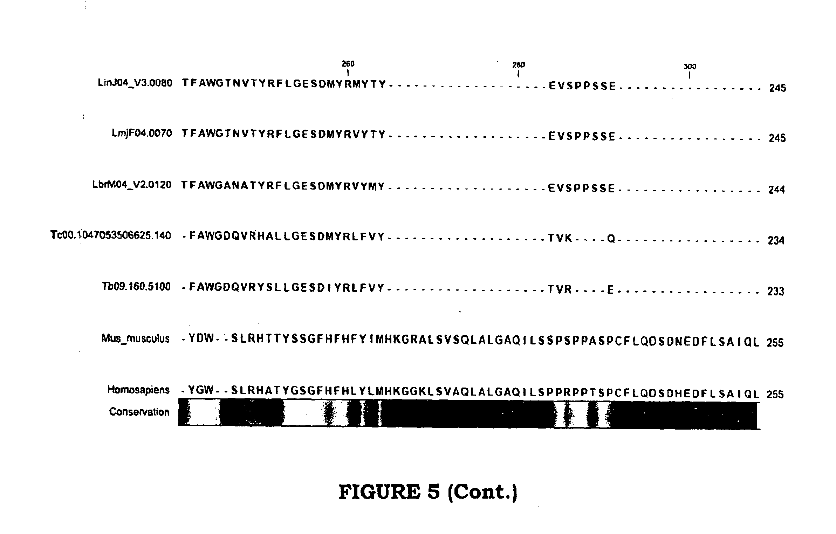

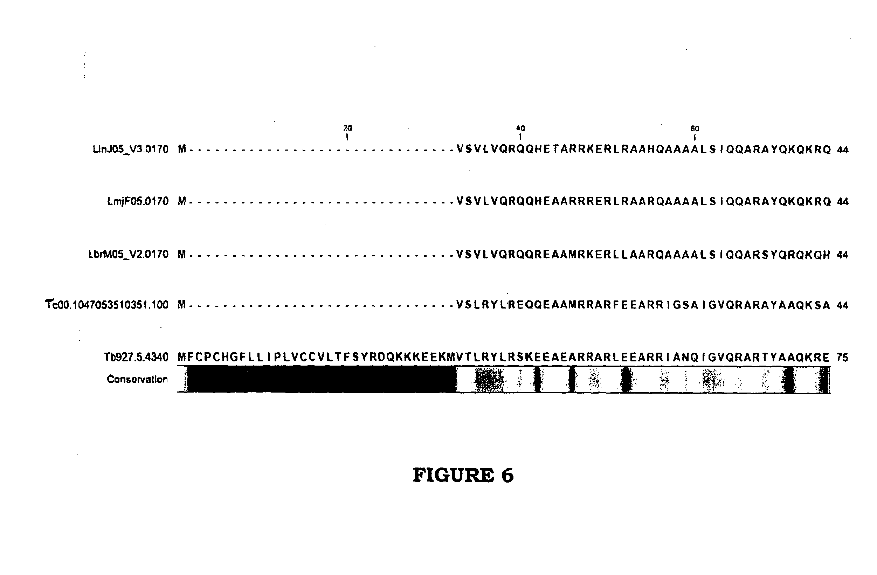

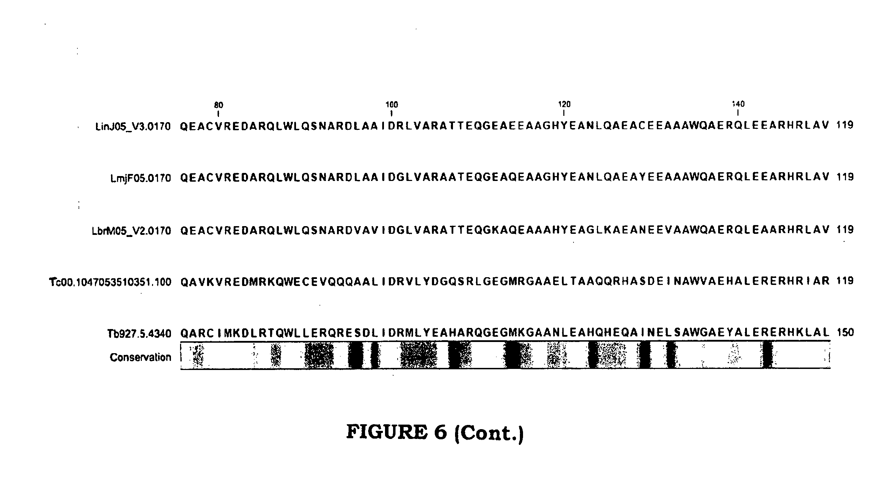

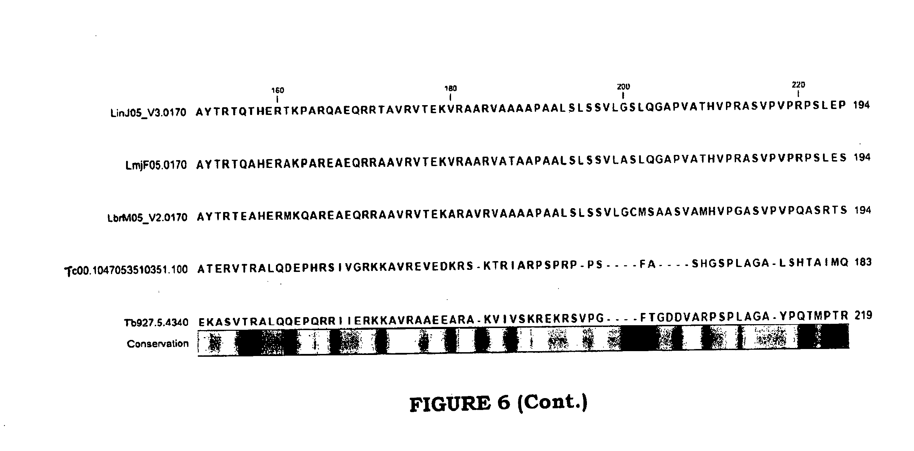

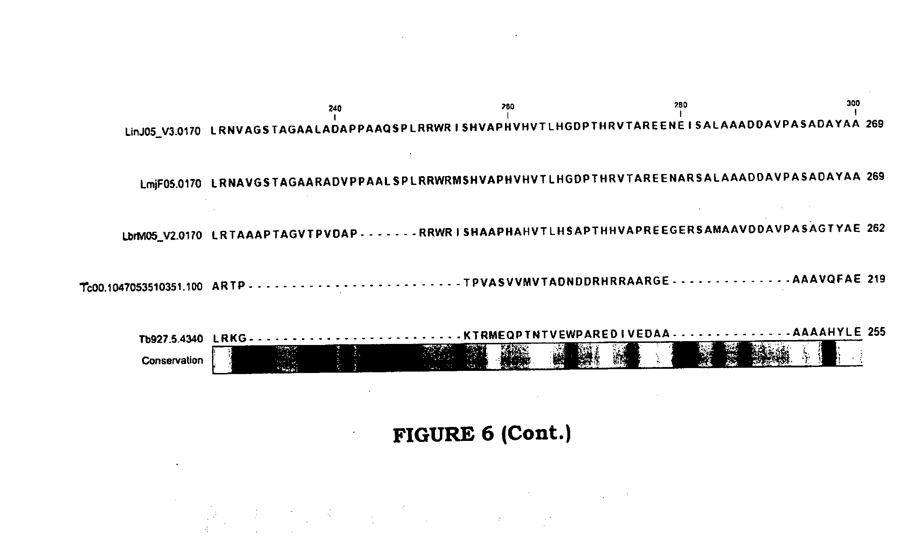

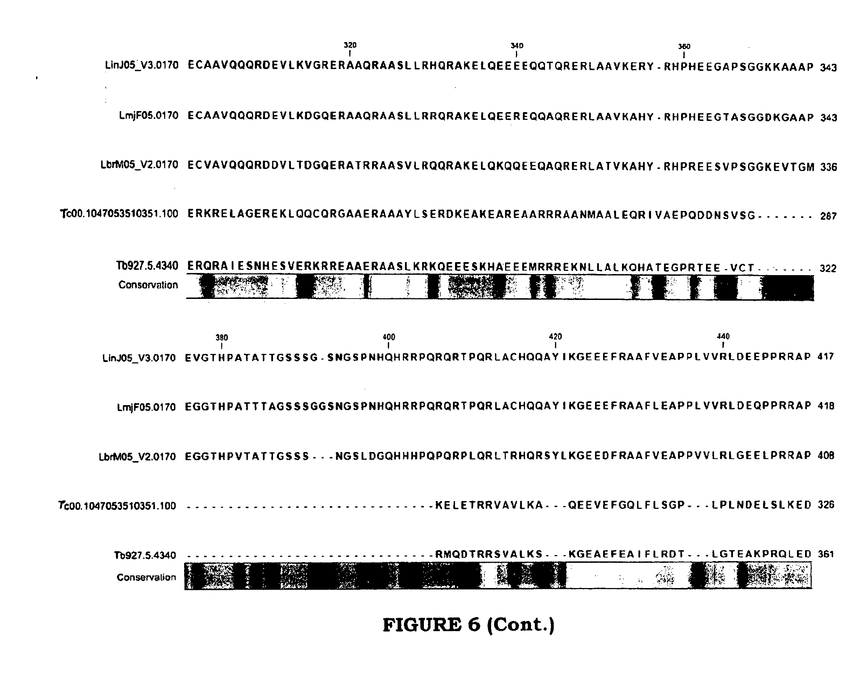

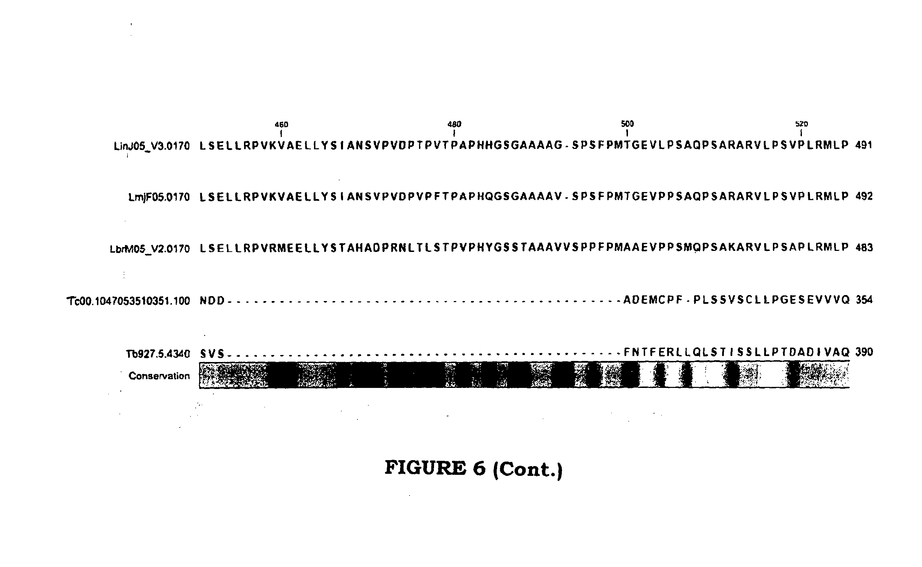

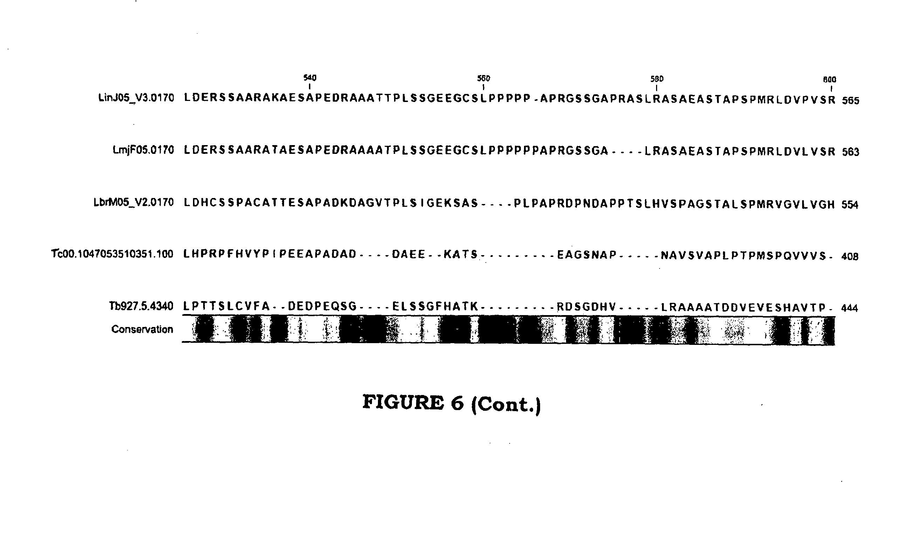

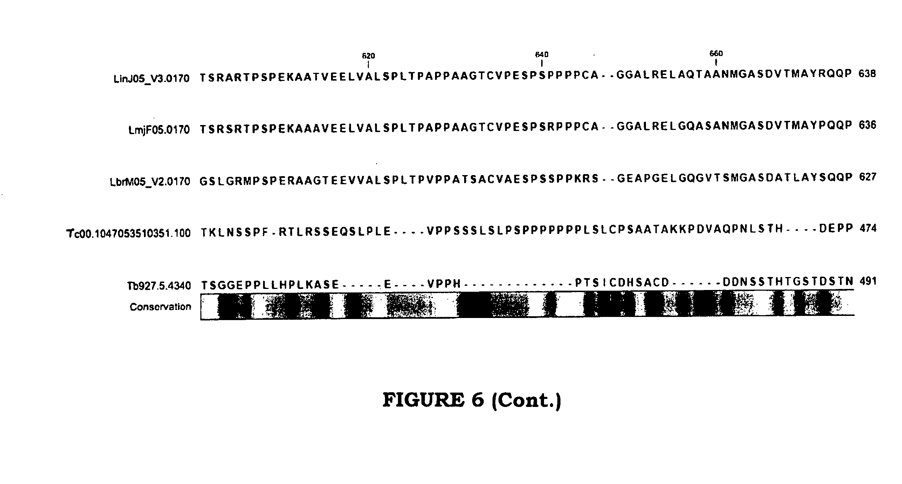

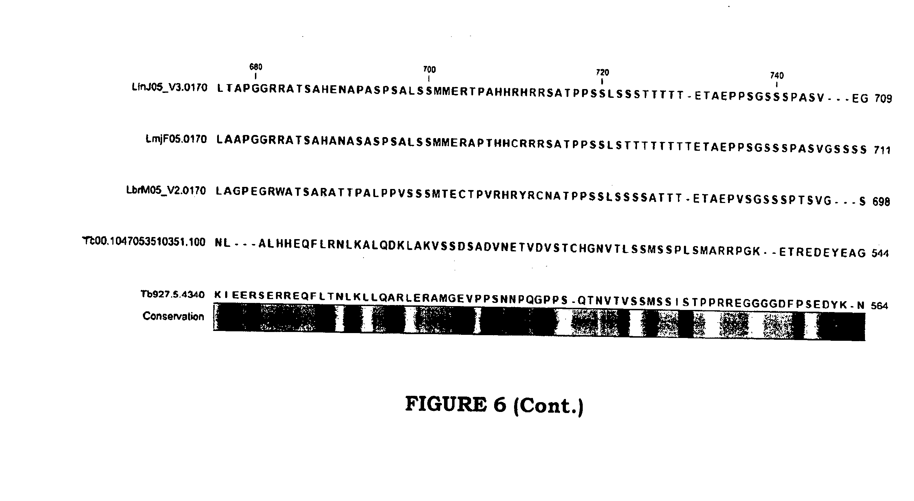

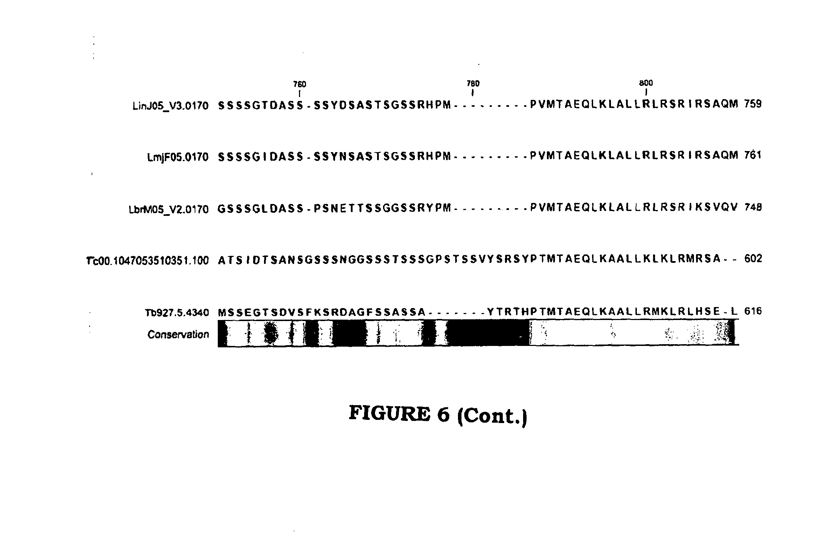

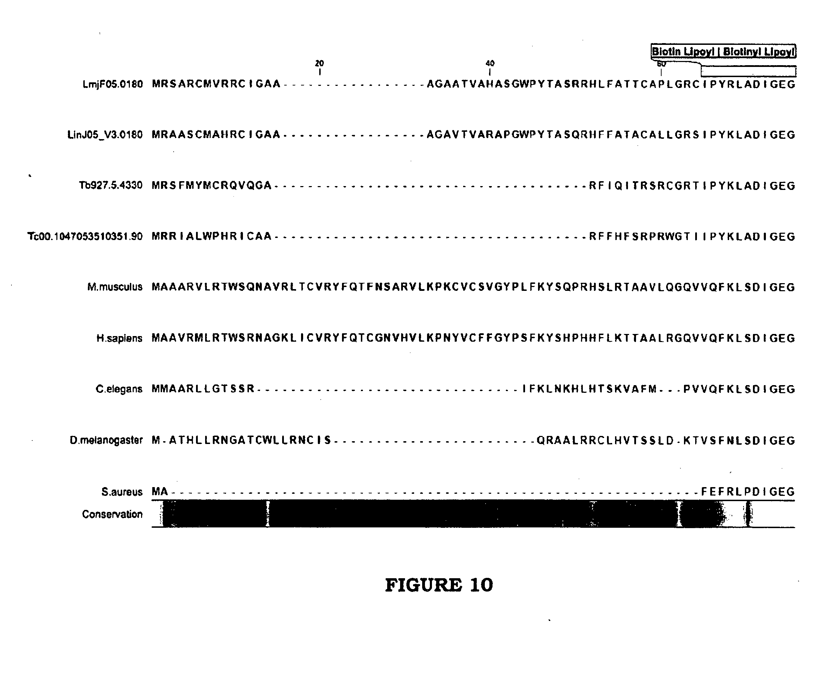

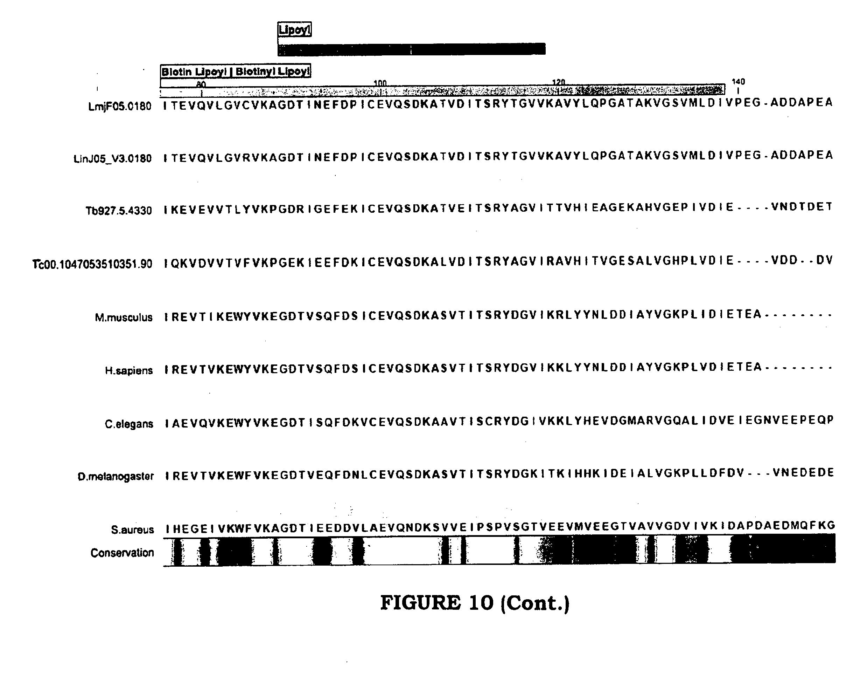

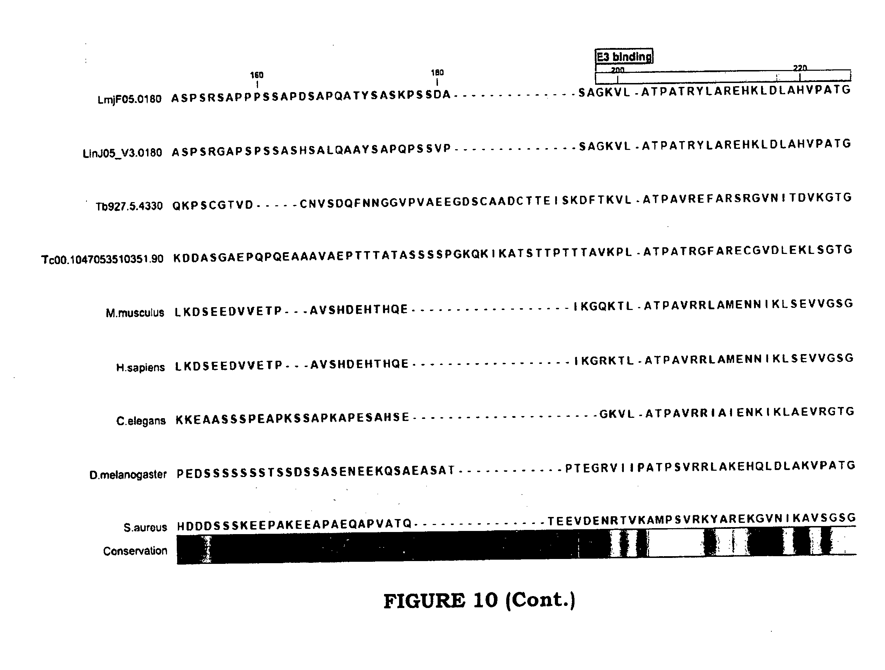

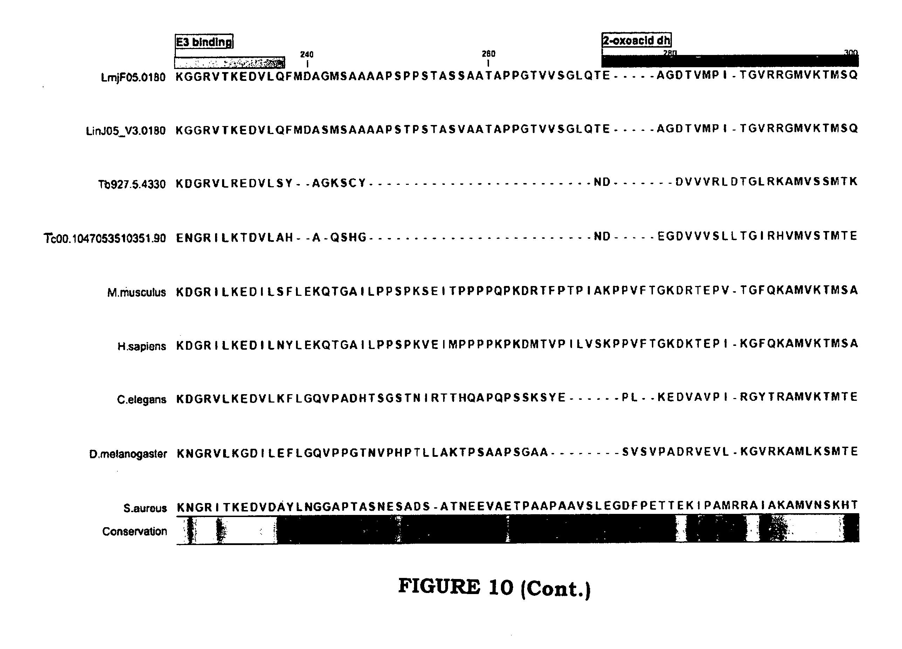

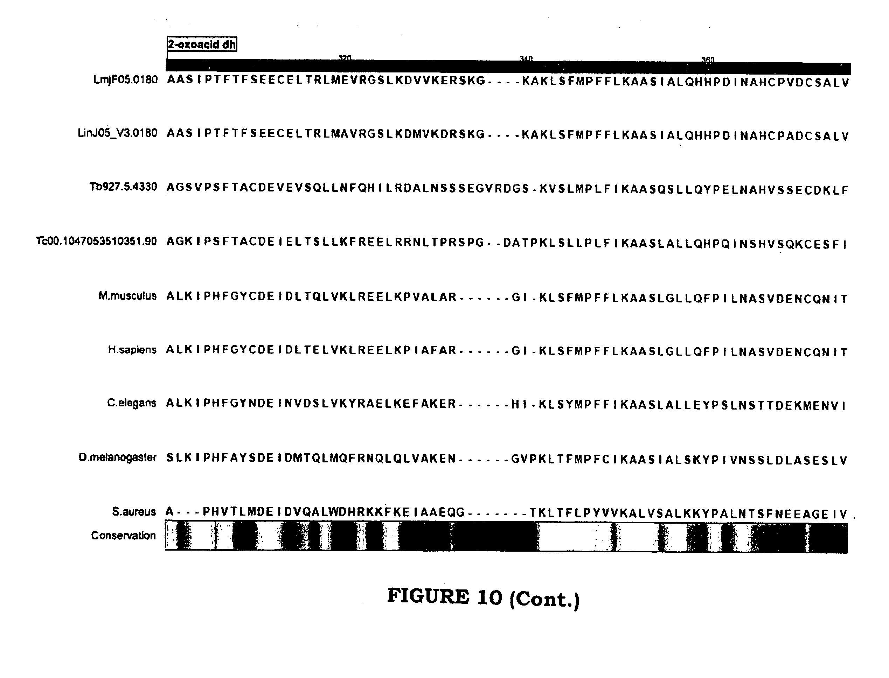

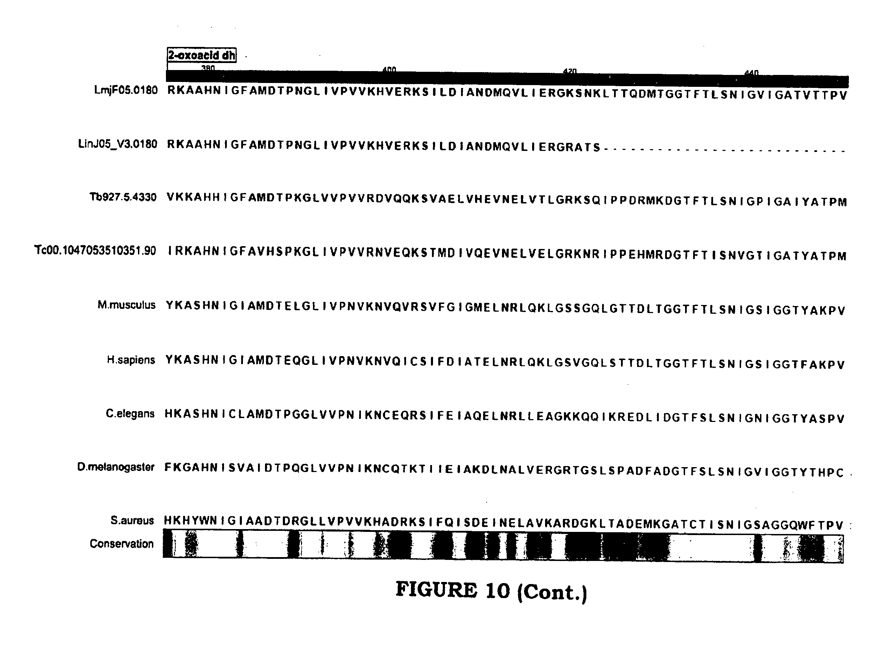

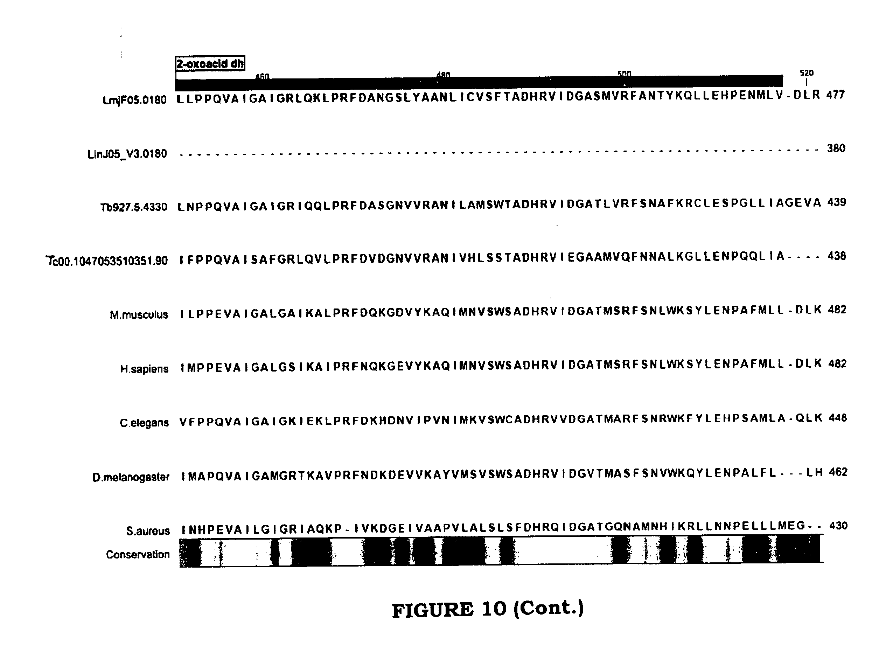

Alignment of proteins including the structures 3D emphasizing the active and interactive sites identified are shown in figures 2 E2-BCKD-alignment, figure 3 MTAP-alignment and figure 4 NR-alignment, figure 5 LT7-alignment figure 6 517-alignment and figure 10.

These figures show the alignment of the protein sequences resulting from various organisms as well as the contents of the various sequences. Various alignments were carried with the program CLC.

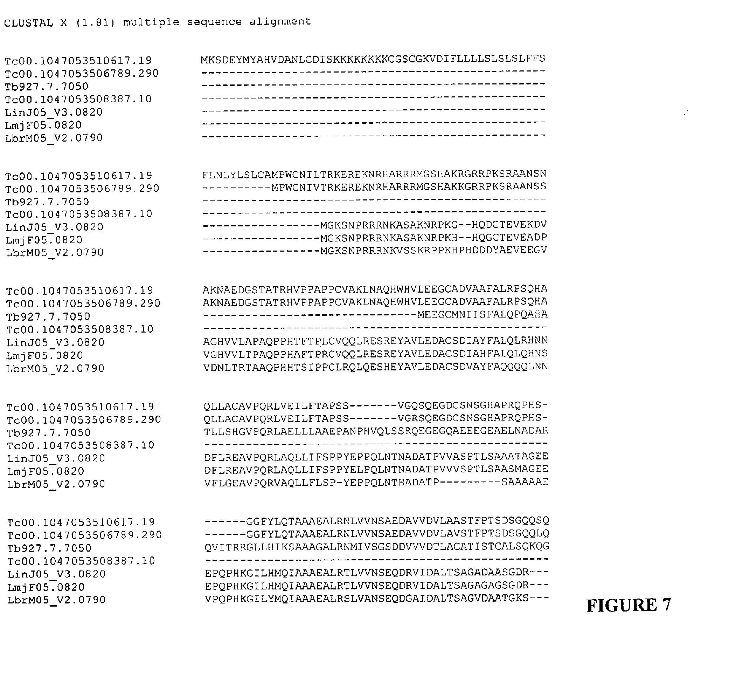

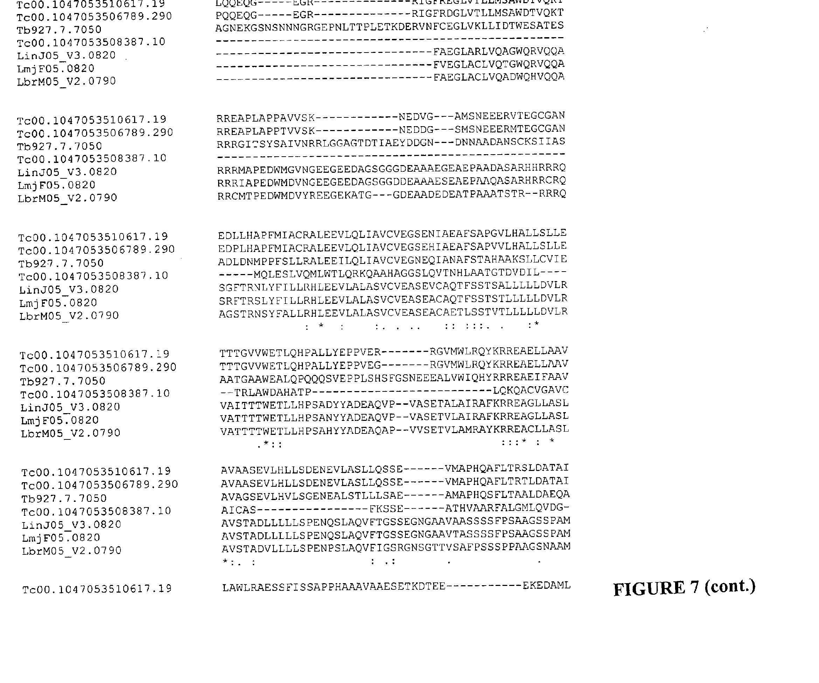

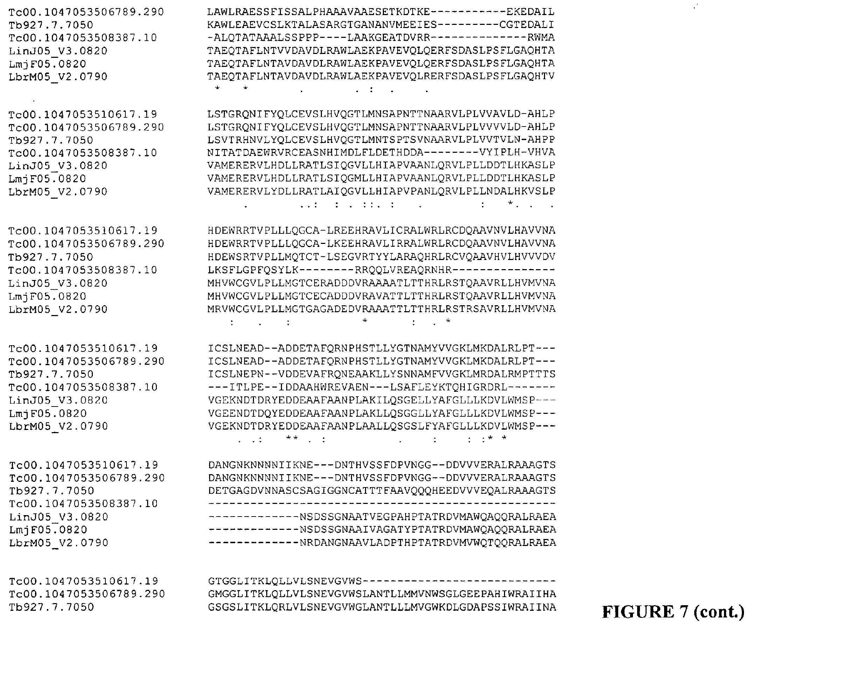

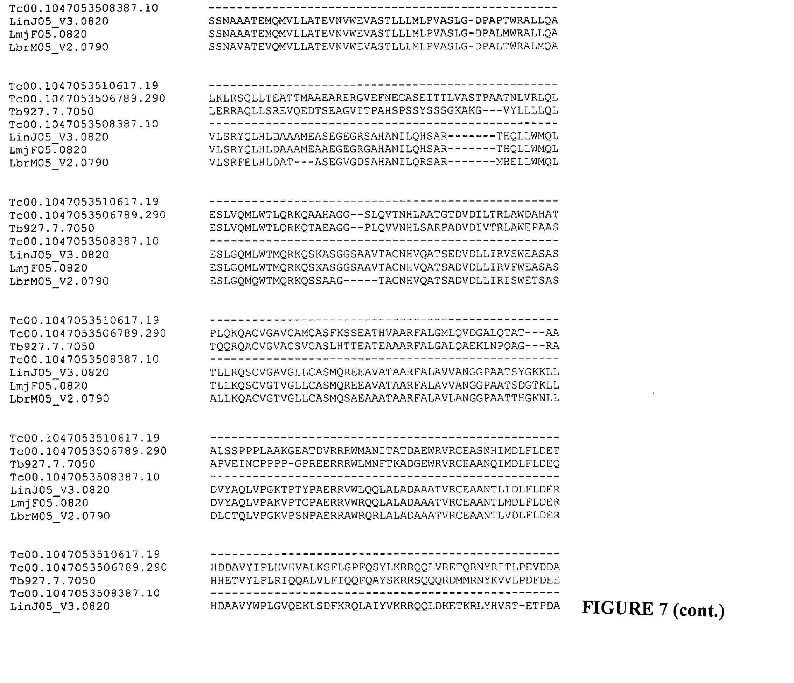

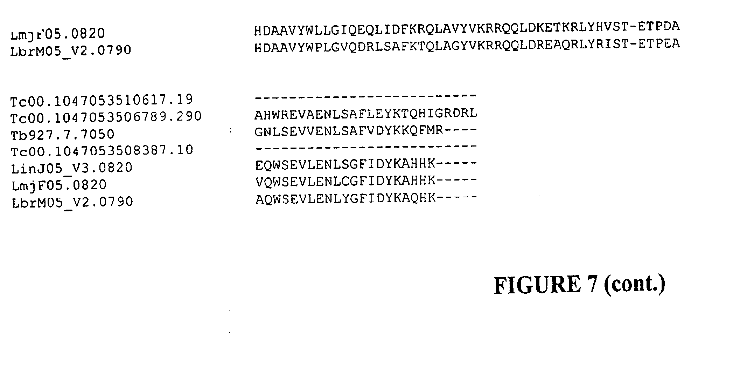

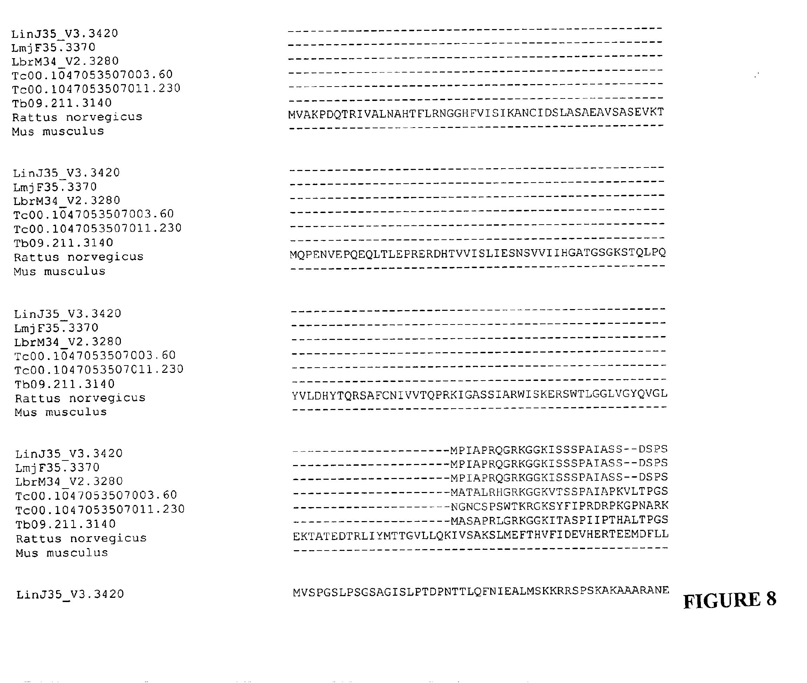

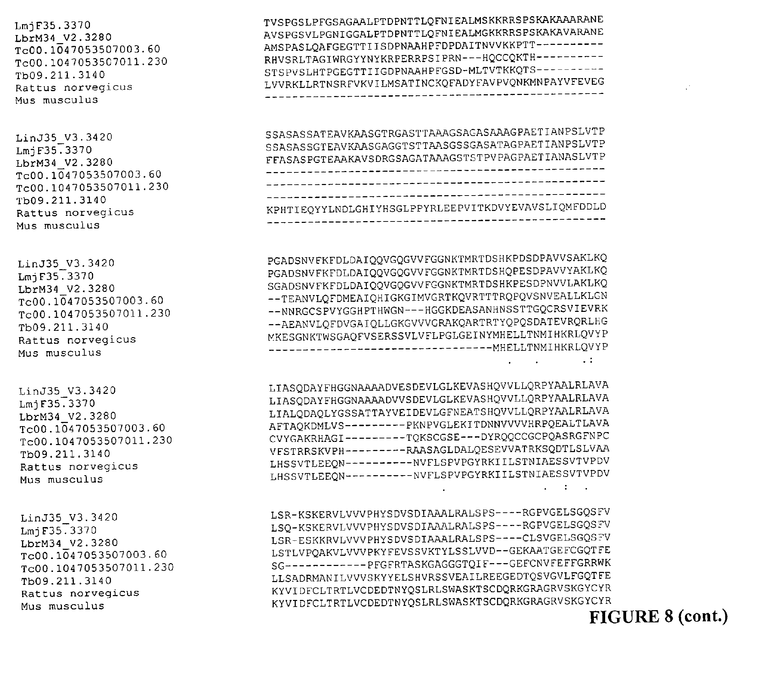

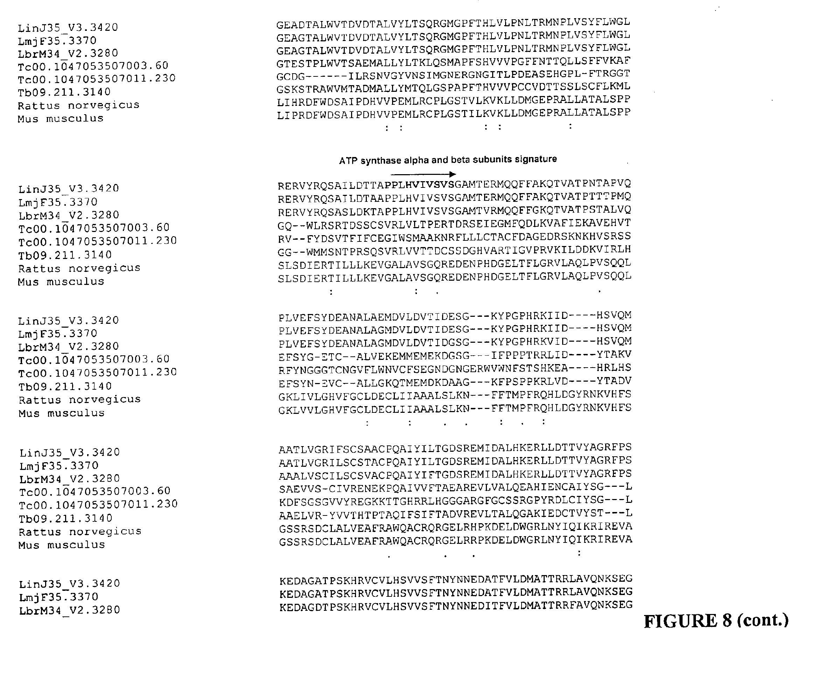

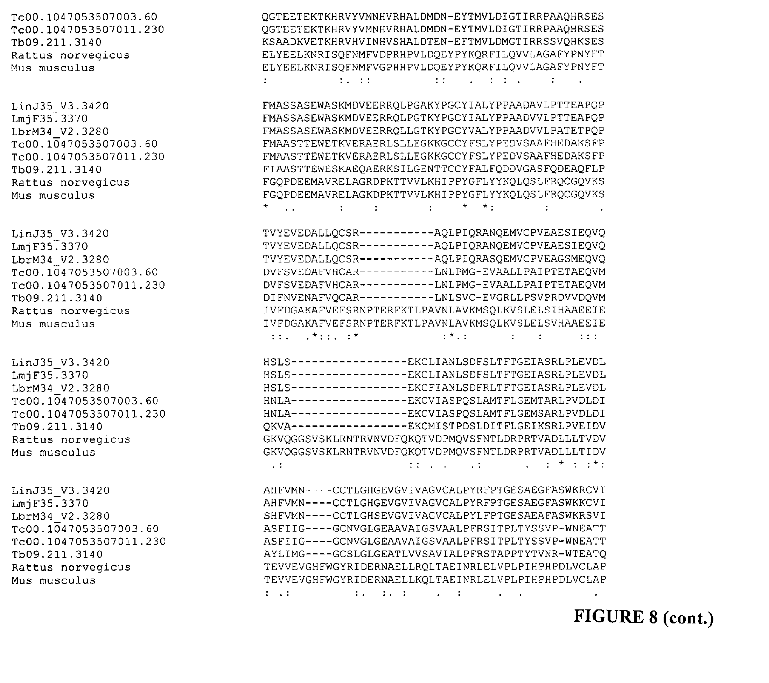

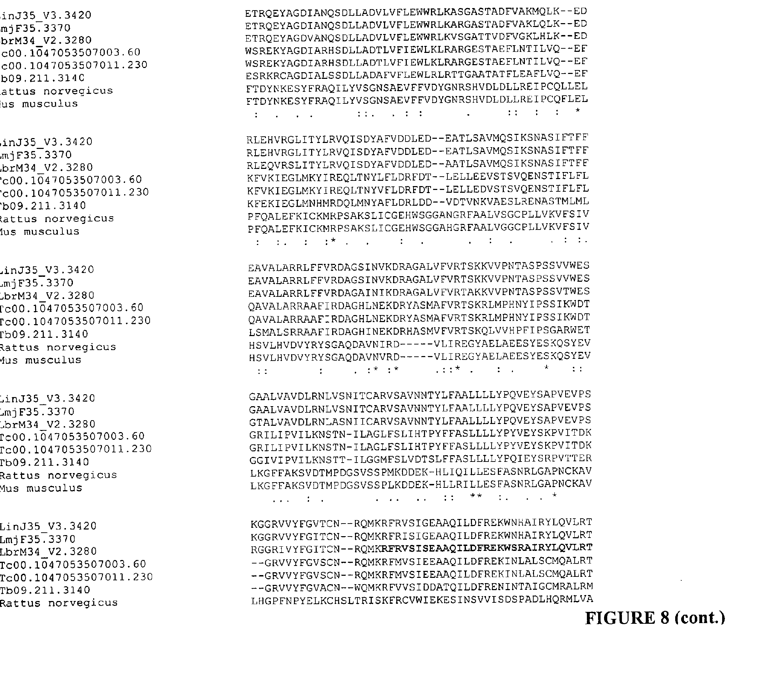

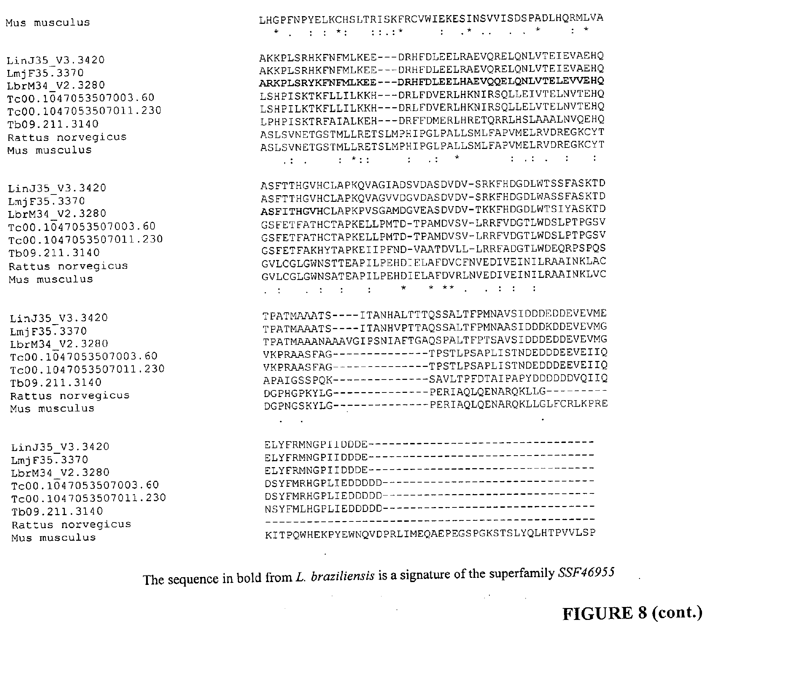

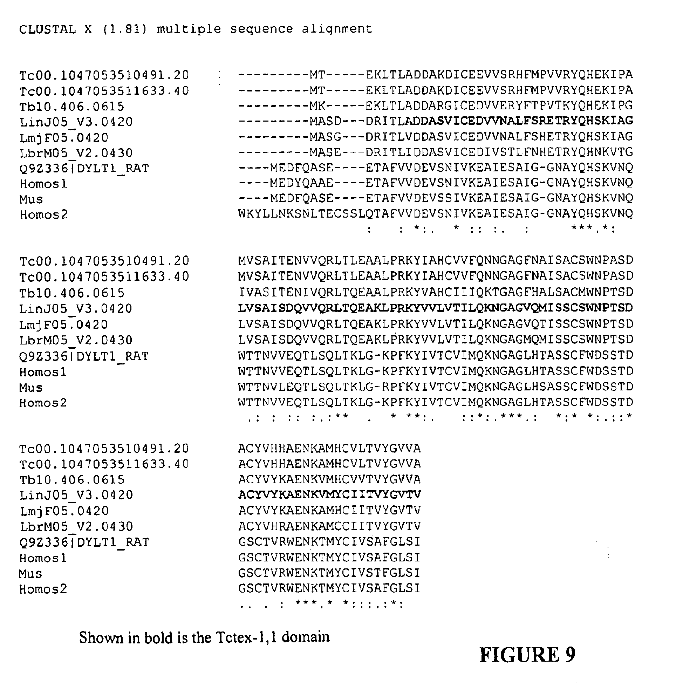

In figure 7 the hypothetical conserved protein LinJ35_V3.3420, figure 8 dynien light chain protein and figure 9 hypothetical conserved protein LinJ05_V3.820 compares these various proteins from *Leishmania infantum* with those the various species.

The results shown in figures 2 to 10, revealed the presence of sequences specific to the species *Leishmania ,* as well as those specific to the family of trypanosomes and others which are shared between all the described organisms. These *Leishmania* or trypanosome specific sequences are candidates for the generation of specific inhibitors and/or activators of the relevant putative virulence factors and could form the basis for novel therapeutics, diagnostic reagents and/or vaccines.

### 2.7 Use of putative virulence factors to sift inhibitors and/or activators for use as anti-Leishmania agents, including chemical molecules, natural substances, designed or selectively evolved polymeric biological substances.

According to a first experimental protocol, methods to identify compounds which affect the activity of the putative virulence factors involve the steps:
1) Cloning into a suitable expression vector such as a bacterial plasmid, the coding sequence of a putative or established virulence factor. Preferably this bacterial plasmid comprises a detectable tag, such as 6xHis, FLAG, GFP which is fused with the cloned coding sequence at the N or C terminus.
2) Expression of the cloned protein in soluble form or which is solubalised following expression and purification if necessary. Purification is preferably made using the detectable tag for instance using an anti-tag antibody or by other suitable means.
3) The enzymatic activity or another recordable characteristic of the protein is recorded. Preferably this is done in a high through put format, for example in a 96 well plate and the enzymatic activity or other characteristic is measured quantitatively.
4) The screening of the candidate compounds is performed by the incubation of the reactional mixtures with the candidate extracts/molecules/substances and detecting alterations in the quantitative characteristic being measured.

According to a second experimental protocol the selection of small inhibiting or activating RNAs made using SELEX or other means of generating specific Aptamers. A significant advantage of this approach is that it does not require knowledge of the activity of the candidate virulence factor and so no experimental protocols need be developed or optimised for a particular candidate virulence factor. It exploits the natural affinity of some RNAs for proteins.

Such methods involve:
1) The expression of the candidate virulence factor.
2) The construction or purchase of a SELEX library.
3) Screening of the SELEX library against the expressed candidate protein.
4) The collection of the interacting RNA molecules.
5) The random mutation of this pool of interacting RNA molecules and the screening of this first generation SELEX library against the candidate protein.
6) Further rounds of screening and mutagenesis as are necessary to evolve suitably specific RNA aptamers.

In both of the above experimental protocols the resulting molecules/aptamers can in a further set of experiments be monitored for activity against the orthologue protein from humans and if found to have an effect thereon be discarded or subjected to modification and/or further evolution as appropriate so as to lose the ability to interact with the ortholgue whilst retaining the ability to affect the parasite protein.

### 2.8 Use of identified virulence factors in diagnostic, therapeutic and vaccine applications

The Inventors have for the first time identified and characterized in *silico,* expressed genomic tags by differential screening of a *L. infantum*-chromosome enriched library with cDNAs from late-log phase promastigotes of two Tunisian *L. infantum* isolates, LV50 and DREP14, isolated from VL and CL patients, respectively.

The *Leishmania* virulence model classifies parasite virulence factors into invasive/ evasive, patho-antigenic and protective/cure determinants, and assumes that such molecular determinants regulate virulence, some of them acting as virulence suppressors or as enhancers (Chang and McGuire, 2002). Differences in gene expression in axenic promastigote forms would potentially relate to host-vector interactions or to the infection process of mammals during its early stages. It is also now well accepted that differentiation process is preparatory and adaptative to a change in host environment (Bates, 2006). Therefore, significant differences on stage-or isolate- expression would point to potential intrinsic intra-specific differences in gene expression, here assumed to be among the mechanisms interfering with the complex host-parasite-environment interplay. They could also reveal novel drug target candidates.

Using a relative semi quantitative estimation, most of the genes analysed showed relative differential expression according to the isolate or the stages. More genes were found overexpressed in PNA - forms of the CL isolate than in the VL isolate. However, isolate-wise comparison of expression levels within PNA + or PNA - forms showed striking differences between the isolates. Comparison of PNA + forms in the 2 isolates suggests a tendency for overexpression of genes in the VL isolate. The observations emphasize on importance of gene expression at the isolate level and tend to indicate that gene expression might not be similarly regulated in the two isolates. As pointed earlier, VL parasites could have presumably a more vigorous metabolism (Guerbouj et al, 2001) and could be under more complex interaction patterns than CL parasites of the same species.

A variable expression pattern between isolates and stages has revealed different phenotypes of which 3 were particularly interesting considering the predictions of the model of Chang and McGuire (2002).

Pattern A supports the hypothesis that the genes (LinJ04_V3.0080 (SEQ ID NO:25) and LinJ05_V3.0830 (SEQ ID NO: 29)) could constitute markers of differentiated stages of *L. infantum* promastigotes.

In pattern B, phenotypes observed suggest that gene LinJ05_V3.0170 (SEQ ID NO: 27) could constitute a marker for VL parasites or for visceralization.

While pattern C seems to favour a potential implication of two other genes (LinJ05_V3.0180 (SEQ ID NO: 31) and LinJ05_V3.0660 (SEQ ID NO: 33)) in reduced virulence / pathogenicity of the cutaneous *L. infantum* parasites.

Interestingly, pattern of Meta 1 gene is in discrepancy with previously described preferential expression in stationary phase promastigotes (assimilated to metacyclics) of *L. major, L. donovani* and *L. mexicana* (Uliana et al., 1999), possibly reflecting differences among *Leishmania* species or isolates. Indeed, genes assessed in the course of differentiation process could be under transient or permanent up-down regulation (Saxena et al., 2007; Sirividya et al., 2007), therefore the expression of the genes here identified still need to be determined against time course in culture.

Of particular interest, the predicted function of 3 genes selected as candidate virulence factors on basis of expression patterns is compatible with roles suggested here, which renders some tentative conclusions about these genes very attractive and likely. More importantly such genes shown to be key players in pathologic processes or in detoxification deserve a special attention as potential drug targets against *Leishmania* parasites. In mammalian cells, the 5' methylthioadenosine phosphorylase (MTAP) is a key enzyme in the catabolism of methylthioadenosine (MTA), a powerful inhibitory by-product of polyamine biosynthesis (Nobori et al., 1996). MTA is known to interfere with various cell processes including regulation of gene expression, proliferation, differentiation and apoptosis. It is hypothesized that MTA could play a regulatory role in the cell (Berasain et al., 2004). MTAP activity is therefore important avoiding accumulation of MTA. MTAP gene acts as a tumour suppressor gene (Tang et al., 2000), inhibiting cell proliferation. This is compatible with it (LinJ05_V3.0830) being a marker for differentiated stages of promastigotes, which do not multiply.

In mammalian cells, the dihydrolipoamide branched chain transacylase or E2 subunit is a part of the mitochondrial branched-chain α-ketoacid dehydrogenase (BCKD) complex that have a central role catalyzing the irreversible oxidative decarboxylation of the branched-chain α-ketoacid deriving from leucine, isoleucine, and valine (Brosnan and Brosnan, 2006). Of particular interest, leucine regulates the activity of this complex and its limitation is known to induce autophagy, a mechanism recently shown associated to *Leishmania* differentiation (Besteiro et al., 2006). Defect in this enzyme or in domains interacting with this enzyme in the BCKD complex are responsible for Maple Syrup Urine Disease, a genetic disorder in humans (Chuang et al., 2006). This enzyme was also shown to be involved in circulating immune complexes in cardiomyopathies or in hepatic diseases (Ansari et al., 1994; Nishioka et al., 1997). Coincidentally, in *Leishmania* the gene (LinJ05_V3.0180) is under positive selection possibly due to immune pressure (Peacock et al., 2007) and its role in parasite survival deserves more attention.

Nitroreductases are a group of enzymes that catalyse the conversion of aromatic nitro compounds to amines that also often exhibits toxic, mutagenic or carcinogenic activity. These proteins have recently raised enormous interest because of their central role in mediating nitroaromatic toxicity (Homma-Takeda et al., 2002; Padda et al., 2003; Sarlauskas et al., 2004), their potential use in bioremediation (Hannink et al., 2001), biocatalysis (Kaiyala et al., 2003) and their utility in activating prodrugs in directed anticancer therapies (Denny, 2002; Hu et al., 2003; Searle et al., 2004). It has been shown also that these enzymes have chromate reduction activity (Kwak et al., 2003). In *Leishmania,* this gene is closer to bacterial genes than to those of fungi, other lower eukaryotes, suggesting their lateral transfer from bacteria (de Oliveira et al., 2007). In *Mycobacterium tuberculosis,* another intramacrophagic pathogen, nitroreductase gene is overexpressed in response to low oxygen conditions within macrophages (Purkayastha et al., 2002). In *Leishmania* parasites, it is interesting to note that it (LinJ05_V3.0660) is over- expressed in the PNA - parasites, as part of a process where they prepare to invade macrophages. The role of this gene in intracellular survival within macrophages or under hypoxic conditions will need to be determined.

Based upon these findings of the inventors these the isolated nucleotide sequences SEQ ID NO: 25, SEQ ID NO: 27, SEQ ID NO: 29, SEQ ID NO: 31, SEQ ID NO: 33 are all potentially useful as therapeutics for instance interference RNA, as diagnostic reagents for instance as part of a nucleic acid hybridisation based kit to detect *Leishmania* DNA and/or as a DNA vaccine.

Likewise the peptide sequences SEQ ID NO: 26, SEQ ID NO: 28, SEQ ID NO: 30, SEQ ID NO: 32, SEQ ID NO: 34 are all potentially useful as vaccines and as components in a diagnostic kit such as to determine the presence of anti-serum antibodies which would be indicative of *Leishmania* infection.In particular kits and/or reagents for the detection of these antigens in urine or saliva samples are considered to be a very useful new diagnostic reagent.

The nucleotide and peptide sequences of the identified putative virulence factors are listed below in Table XI.

**Table XI - Nucleotide and Peptide Sequences of differentially expressed Leishmania putative virulence factors**

| Name | Sequence | SEQ ID NO |
|---|---|---|
| LinJ04_V3.00 80 (LT7) Nucleotide | | 25 |
| LinJ04_V3.0080 (LT7) Peptide | | 26 |
| LinJ05_V3.0170 (517) Nucleotide | | 27 |
| | | |
| LinJ05_V3.0170 (517) Peptide | | 28 |
| LinJ05_V3.0830 | | 29 |
| Nucleotide | | |
| LinJ05_V3.0830 Peptide | | 30 |
| LinJ05_V3.0180 Nucleotide | | 31 |
| LinJ05_V3.0180 Peptide | | 32 |
| | | |
| LinJ05_V3.0660 Nucleotide | | 33 |
| LinJ05_V3.0660 Peptide | | 34 |
| LinJ35_V3.3420 Nucleotide | | 35 |
| | | |
| LinJ05_V3 0420 Nucleotide | | 36 |
| | | |
| LinJ05_V3.0820 Nucleotide | | 37 |
| | | |
| LiN05_V3.0840 Nucleotide | | 38 |
| | | |
| LinJ05_V3.0650 Nucleotide | | 39 |
| | | |
| LinJ05_V3.0430 Nucleotide | | 40 |
| | | |
| LinJ04_V3.0070 Nucletide | | 41 |
| | | |
| LinJ04_V3.0090 Nucleotide | | 42 |
| LinJ35_V3 3430 Nucleotide | | 43 |

### References

Akopyants, N.S., Clifton, S.W., Martin, J., Pape, D., Wylie, T., Li, L., Kissinger, J.C., Roos, D.S., Beverley, S.M., 2001. A survey of the Leishmania major Friedlin strain V1 genome by shotgun sequencing: a resource for DNA microarrays and expression profiling. Mol. Biochem. Parasitol. 113, 337-340.
Ansari, .A.A., Neckelmann, N., Villinger, F., Leung, P., Danner, D.J., Brar, S.S., Zhao, S., Gravanis, M.B., Mayne, A., Gershwin, M.E., 1994. Epitope mapping of the branched chain alpha-ketoacid dehydrogenase dihydrolipoyl transacylase (BCKD-E2) protein that reacts with sera from patients with idiopathic dilated cardiomyopathy. J Immunol. 153, 4754-4765.
Antoniazi, S., Lima, H.C., Cruz, A.K., 2000. Overexpression of miniexon gene decreases virulence of Leishmania major in BALB/c mice in vivo. Mol. Biochem. Parasitol. 107, 57-69.
Baptista-Fernandes, T., Marques, C., Roos Rodrigues, O., Santos-Gomes, G.M., 2007. Intra-specific variability of virulence in Leishmania infantum zymodeme MON-1 strains. Comp Immunol Microbiol Infect Dis. 30, 41-53.
Bates, P.A., 2006. Housekeeping by Leishmania. Trends Parasitol. 22, 447-448.
Bates, P.A., Rogers, M.E., 2004. New insights into the developmental biology and transmission mechanisms of Leishmania. Cur. Mol. Med. 4,601-609.
Belhadj, S., Pratlong, F., Hammami, M., Kallel, K., Dedet, J.P., Chaker, E., 2003. Human cutaneous leishmaniasis due to Leishmania infantum in the Sidi Bourouis focus (Northern Tunisia): epidemiological study and isoenzymatic characterization of the parasites. Acta. Trop. 85, 83-86.
Belkaid, P.M., Harrat, Z., Hamrioui, B., Thellier, M., Datry, A., Danis, M., 1996. A simple media for isolation and culture of Leishmania. Bull. Soc. Pathol. Exot. 89, 276-277.
Ben Said, M., Guerbouj, S., Saghrouni, F., Fathallah-Mili. A., Guizani, I., 2006. Occurrence of Leishmania infantum cutaneous leishmaniasis in central Tunisia. Trans. R. Soc. Trop. Med. Hyg. 100, 521-526.
Berasain, C., Hevia, H., Fernandez-Irigoyen, J., Larrea, E., Caballeria, J., Mato, J.M., Prieto, J., Corrales, F.J., Garcia Trevijano, E.R., Avila, M.A., 2004. Methylthioadenosine phosphorylase gene expression is impaired in human liver cirrhosis and hepatocarcinoma. Biochim. Biophys. Acta. 1690, 276-284.
Besteiro, S., Williams, R.A., Morrison, L.S., Coombs, G.H., Mottram, J.C., 2006. Endosome sorting and autophagy are essential for differentiation and virulence of Leishmania major. J Biol Chem. 281, 11384-11396.
M. Blank, et al., Systematic evolution of a DNA aptamer binding to rat brain tumor microvessels. Selective targeting of endothelial regulatory protein pigpen, J. Biol. Chem. 276 (19) (2001) 16464-16468.
Britto, C., Ravel, C., Bastien, P., Blaineau, C., Pages, M., Dedet, J.P., Wincker, P., 1998. Conserved linkage groups associated with large-scale chromosomal rearrangements between Old World and New World Leishmania genomes. Gene. 222, 107-117.
Brosnan, J.T., Brosnan, M.E., 2006. Branched-chain amino acids: enzyme and substrate regulation. J Nutr. 136, 207-211.
Campino, L., Pratlong, F., Abranches, P., Rioux, J.A., Santos-Gomes, G., Alves-Pires, C., Cortes, S., Ramada, J., Cristovao, J.M., Afonso, M.O., Dedet, J.P., 2006. Leishmaniasis in Portugal: enzyme polymorphism of Leishmania infantum based on the identification of 213 strains. Trop. Med. Int. Health. 11, 1708-1714.
Carver, T.J., Rutherford, K.M., Berriman, M., Rajandream, M.A., Barrell, B.G., Parkhill, J., 2005. ACT: the Artemis Comparison Tool. Bioinformatics. 21, 3422-3423.
Chakroun, A. S. Turki, L. Kebaier, H. Barhoumi, M. Saadi, Y. Smeti, I. Sahli, A. Guerbouj, S. Lahé madi, R. Ben Fadhel, M. Driss, M. Kaabi, B. Ajroud, K. Chemkhi, J. Ben Abderrazak, S. Guizani., I. 2007. LB Tx: A Tool Box for the Selection and Analysis of Potential Leishmania Targets for Intervention and Disease Control. International Conference on the Bioinformatics of African Pathogens and Disease Vectors» à Nairobi (Kenya) 22-01 Juin 2007, «www.lirmm.fr/france_afrique/Nairobi2007/Nairobi_program.pdf».
Chang, K.P., Akman, L., Nielsen, J.S., 1999. Leishmania virulence and genetic heterogeneity. Clin. Dermatol. 17, 269-273.
Chang, K.P., McGwire, B.S., 2002. Molecular determinants and regulation of Leishmania virulence. Kinetoplastid. Biol. Dis. 1, 1.
Chang, K.P., Reed, S.G., McGwire, B.S., Soong, L., 2003. Leishmania model for microbial virulence: the relevance of parasite multiplication and pathoantigenicity. Acta. Trop. 85, 375-390.
Cerchia L, Duconge F, Pestourie C, Boulay J, Aissouni Y, Gombert K, Tavitian B, de Franciscis V, Libri D. Neutralizing aptamers from whole-cell SELEX inhibit the RET receptor tyrosine kinase. PLoS Biol. 2005 Apr;3(4):e123. Epub 2005 Mar 22.Chuang, D.T., Chuang, J.L., Wynn, R.M., 2006. Lessons from genetic disorders of branched-chain amino acid metabolism. J. Nutr. 136, 243S-249S.
de Oliveira, I.M., Henriques, J.A., Bonatto, D., 2007. In silico identification of a new group of specific bacterial and fungal nitroreductases-like proteins. Biochem Biophys Res Commun. 355, 919-925.
D.A. Daniels, et al., A tenascin-C aptamer identified by tumor cell SELEX: systematic evolution of ligands by exponential enrichment, Proc. Natl. Acad. Sci. USA 100 (26) (2003) 15416-15421.
Denny, W.A., 2002. Acridine derivatives as chemotherapeutic agents. Curr. Med. Chem. 9, 1655-1665.
Denise H., Poot J, Jiménez M, Ambit A, Herrmann DC, Vermeulen AN, Coombs GH, Mottram JC. Studies on the CPA cysteine peptidase in the Leishmania infantum genome strain JPCM5. BMC Mol Biol. 2006 Nov 13; 7:42.
Dillon, D.C., Day, C.H., Whittle, J.A., Magill, A.J., Reed, S.G., 1995. Characterization of a Leishmania tropica antigen that detects immune responses in Desert Storm viscerotropic leishmaniasis patients. Proc. Natl. Acad. Sci. U S A. 92, 7981-7985.
Ellington AD, Szostak JW. In vitro selection of RNA molecules that bind specific ligands. Nature. 1990 Aug 30;346(6287):818-22.
Gramiccia, M., 2003. The identification and variability of the parasites causing leishmaniasis in HIV-positive patients in Italy. Ann Trop Med Parasitol. 97, 65-73.
Guerbouj, S., Guizani, I., Speybroeck, N., Le Ray, D., Dujardin, J.C., 2001. Genomic polymorphism of Leishmania infantum: a relationship with clinical pleomorphism?. Infect. Genet. Evol. 1, 49-59.
Hannink, N., Rosser, S.J., French, C.E., Basran, A., Murray, J.A., Nicklin, S., Bruce, N.C., 2001. Phytodetoxification of TNT by transgenic plants expressing a bacterial nitroreductase. Nat. Biotechnol. 19, 1168-1172.
Holzer, T.R., McMaster, W.R., Forney, J.D., 2006. Expression profiling by whole-genome interspecies microarray hybridization reveals differential gene expression in procyclic promastigotes, lesion-derived amastigotes, and axenic amastigotes in Leishmania mexicana. Mol. Biochem. Parasitol. 146, 198-218.
Homma-Takeda, S., Hiraku, Y., Ohkuma, Y., Oikawa, S., Murata, M., Ogawa, K., Iwamuro, T., Li, S., Sun, G.F., Kumagai, Y., Shimojo, N., Kawanishi, S., 2002. 2,4,6-trinitrotoluene-induced reproductive toxicity via oxidative DNA damage by its metabolite. Free. Radic. Res. 36, 555-566.
Honore, S., Garin, Y.J., Sulahian, A., Gangneux, J.P., Derouin, F., 1998. Influence of the host and parasite strain in a mouse model of visceral Leishmania infantum infection. FEMS. Immunol. Med. Microbiol. 21, 231-239.
Hu, L., Yu, C., Jiang, Y., Han, J., Li, Z., Browne, P., Race, P.R., Knox, R.J., Searle, P.F., Hyde, E.I., 2003. Nitroaryl phosphoramides as novel prodrugs for E. coli nitroreductase activation in enzyme prodrug therapy. J. Med. Chem. 46, 4818-4821.
Ivens, A.C., Peacock, C.S., Worthey, E.A., Murphy, L., Aggarwal, G., Berriman, M., Sisk, E., Rajandream, M.A., Adlem, E., Aert, R., Anupama, A., Apostolou, Z., Attipoe, P., Bason, N., Bauser, C., Beck, A., Beverley, S.M., Bianchettin, G., Borzym, K., Bothe, G., Bruschi, C.V., Collins, M., Cadag, E., Ciarloni, L., Clayton, C., Coulson, R.M., Cronin, A., Cruz, A.K., Davies, R.M., De Gaudenzi, J., Dobson, D.E., Duesterhoeft, A., Fazelina, G., Fosker, N., Frasch, A.C., Fraser, A., Fuchs, M., Gabel, C., Goble, A., Goffeau, A., Harris, D., Hertz-Fowler, C, Hilbert, H, Horn, D, Huang, Y, Klages, S, Knights, A, Kube, M, Larke, N, Litvin, L, Lord, A., Louie, T., Marra, M., Masuy, D., Matthews, K., Michaeli, S., Mottram, J.C., Muller-Auer, S., Munden, H., Nelson, S., Norbertczak, H., Oliver, K., O'neil, S., Pentony, M., Pohl, T.M., Price, C., Purnelle, B., Quail, M.A., Rabbinowitsch, E., Reinhardt, R., Rieger, M., Rinta, J., Robben, J., Robertson, L., Ruiz, J.C., Rutter, S., Saunders, D., Schafer, M., Schein, J., Schwartz, D.C., Seeger, K., Seyler, A., Sharp, S., Shin, H., Sivam, D., Squares, R., Squares, S., Tosato, V., Vogt, C., Volckaert, G., Wambutt, R., Warren, T., Wedler, H., Woodward, J., Zhou, S., Zimmermann, W., Smith, D.F., Blackwell, J.M., Stuart, K.D., Barrell, B., Myler, P.J., 2005. The genome of the kinetoplastid parasite, Leishmania major. Science. 309, 436-442.
Kaiyala, K.J., Thiele, T.E., Watson, C.H., Ramsay, D.S., 2003. Nitrous oxide-induced c-Fos expression in the rat brain. Brain. Res. 967, 73-80.
Kallel, K., Ammari, L., Kaouech, E., Belhadj, S., Anane, S., Kilani, B., Chaker, E., 2007. Asymptomatic bearing of Leishmania infantum among tunisian HIV infected patients. Pathol. Biol. 19, 1-4.
Kamhawi, S., 2006. Phlebotomine sand flies and Leishmania parasites: friends or foes? Trends. Parasitol. 22, 439-445.
Kwak, Y.H., Lee, D.S., Kim, H.B., 2003. Vibrio harveyi nitroreductase is also a chromate reductase. Appl. Environ. Microbiol. 69, 4390-4395.
Leifso, K., Cohen-Freue, G., Dogra, N., Murray, A., McMaster, W.R., 2007. Genomic and proteomic expression analysis of Leishmania promastigote and amastigote life stages: the Leishmania genome is constitutively expressed. Mol. Biochem. Parasitol. 152, 35-46.
Louassini, M., Adroher, F.J., Foulquie, M.R., Benitez, R., 1998. Investigations on the in vitro metacyclogenesis of a visceral and a cutaneous human strain of Leishmania infantum. Acta. Trop. 70, 355-368.
Louassini, M., Foulquie, M., Benitez, R., Adroher, F.J., 1999a. Citric-acid cycle key enzyme activities during in vitro growth and metacyclogenesis of Leishmania infantum promastigotes. J. Parasitol. 85, 595-602.
Louassini, M., Foulquie, M.R., Benitez, R., Adroher, F.J., 1999b. Activity of key enzymes in glucose catabolism during the growth and metacyclogenesis of Leishmania infantum. Parasitol. Res. 85, 300-306.
K.N: Morris; et al., High affinity ligands from in vitro selection: complex targets, Proc. Natl. Acad. Sci. USA 95 (6) (1998) 2902-2907.
Murray, A., Fu, C., Habibi, G., McMaster, W.R., 2007. Regions in the 3' untranslated region confer stage-specific expression to the Leishmania mexicana a 600-4 gene. Mol. Biochem. Parasitol. 153, 125-132.
Murray, H.W., Berman, J.D., Davies, C.R., Saravia, N.G., 2005. Advances in leishmaniasis. Lancet. 366, 1561-1577.
Nishioka, M., Morshed, S.A., Parveen, S., Kono, K., Matsuoka, H., Manns, M.P., 1997. Antibodies to P450IID6, SLA, PDH-E2 and BCKD-E2 in Japanese patients with chronic hepatitis. J Gastroenterol Hepatol. 12, 862-868.
Nobori, T., Takabayashi, K., Tran, P., Orvis, L., Batova, A., Yu, A.L., Carson, D.A., 1996. Genomic cloning of methylthioadenosine phosphorylase: a purine metabolic enzyme deficient in multiple different cancers. Proc. Natl. Acad. Sci. U.S.A. 93, 6203-6208.
Padda, R.S., Wang, C., Hughes, J.B., Kutty, R., Bennett, G.N., 2003. Mutagenicity of nitroaromatic degradation compounds. Environ. Toxicol. Chem. 22, 2293-2297.
Pages, M., Bastien, P., Veas, F., Rossi, V., Bellis, M., Wincker, P., Rioux, J.A., Roizes, G., 1989. Chromosome size and number polymorphisms in Leishmania infantum suggest amplification/deletion and possible genetic exchange. Mol. Biochem. Parasitol. 36, 161-168.
Peacock, C.S., Seeger, K., Harris, D., Murphy, L., Ruiz, J.C., Quail, M.A., Peters, N., Adlem, E., Tivey, A., Aslett, M., Kerhornou, A., Ivens, A., Fraser, A., Rajandream, M.A., Carver, T., Norbertczak, H., Chillingworth, T., Hance, Z., Jagels, K., Moule, S., Ormond, D., Rutter, S., Squares, R., Whitehead, S., Rabbinowitsch, E., Arrowsmith, C., White, B., Thurston, S., Bringaud, F., Baldauf, S.L., Faulconbridge, A., Jeffares, D., Depledge, D.P., Oyola, S.O., Hilley, J.D., Brito, L.O., Tosi, L.R., Barrell, B., Cruz, A.K., Mottram, J.C., Smith, D.F., Berriman, M., 2007. Comparative genomic analysis of three Leishmania species that cause diverse human disease. Nat. Genet. 39, 839-847.
Pratlong, F., Rioux, J.A., Marty, P., Faraut-Gambarelli, F., Dereure, J., Lanotte, G., Dedet, J.P., 2004. Isoenzymatic analysis of 712 strains of Leishmania infantum in the south of France and relationship of enzymatic polymorphism to clinical and epidemiological features. J. Clin. Microbiol. 42, 4077-4082.
Pratlong, F., Rioux, J.A., Marty, P., Faraut-Gambarelli, F., Dereure, J., Lanotte, G., Dedet J.P., 2004. Isoenzymatic analysis of 712 strains of Leishmania infantum in the south of France and relationship of enzymatic polymorphism to clinical and epidemiological features. J Clin Microbiol. 42, 4077-4082.
Purdy, J.E., Donelson, J.E., Wilson, M.E., 2005a. Leishmania chagasi: the alpha-tubulin intercoding region results in constant levels of mRNA abundance despite protein synthesis inhibition and growth state. Exp. Parasitol. 110, 102-107.
Purdy, J.E., Donelson, J.E., Wilson, M.E., 2005b. Regulation of genes encoding the major surface protease of Leishmania chagasi via mRNA stability. Mol. Biochem. Parasitol. 142, 88-97.
Purkayastha, A., McCue, L.A., McDonough, K.A., 2002. Identification of a Mycobacterium tuberculosis putative classical nitroreductase gene whose expression is coregulated with that of the acr gene within macrophages, in standing versus shaking cultures, and under low oxygen conditions. Infect. Immun. 70, 1518-1529.
Quijada, L., Soto, M., Requena, J.M., 2005. Genomic DNA macroarrays as a tool for analysis of gene expression in Leishmania. Exp Parasitol. 111, 64-70.
Ravel C., Dubessay P., Britto C., Blaineau C., Bastien P., Pages M., 1999. High conservation of the fine-scale organisation of chromosome 5 between two pathogenic Leishmania species. Nucleic. Acids. Res. 27, 2473-2477.
Ravel, C., Macari, F., Bastien, P., Pagès, M., Blaineau, C., 1995a. Conservation among Old World Leishmania species of six physical linkage groups defined in Leishmania infantum small chromosomes. Mol. Biochem. Parasitol. 69, 1-8.
Ravel, C., Wincker, P., Bastien, P., Blaineau, C., Pages, M., 1995b. A polymorphic minisatellite sequence in the subtelomeric regions of chromosomes I and V in Leishmania infantum. Mol. Biochem. Parasitol. 74, 31 -41.Rochette, A, Raymond, F., Ubeda, J., Smith, M., Messier, N., Boisvert, S., Rigault, P., Corbeil, J., Ouellette M., and Papadopoulou, B., Genome-wide gene expression profiling analysis of Leishmania major and Leishmania infantum developmental stages reveals substantial differencesbetween the two speciesBMC Genomics 2008, 9:255 doi:10.1186/11471-2164-9-255
Rogers, M.E., Chance, M.L., Bates, P.A., 2002. The role of promastigote secretory gel in the origin and transmission of the infective stage of Leishmania mexicana by the sandfly Lutzomyia longipalpis. Parasitology. 124, 495-507.
Sambrook, J., Fritsch, E. F., Maniatis, T., (1989). Molecular cloning. A laboratory manual. Cold Spring Harbor , NY : Cold Spring Harbor Laboratory Press.
Sarlauskas, J., Nemeikaite-Ceniene, A., Anusevicius, Z., Miseviciene, L., Julvez, M.M., Medina, M., Gomez-Moreno, C., Cenas, N., 2004. Flavoenzyme-catalyzed redox cycling of hydroxylamino- and amino metabolites of 2,4,6-trinitrotoluene: implications for their cytotoxicity. Arch. Biochem. Biophys. 425, 184-192.
Saxena, A., Lahav, T., Holland, N., Aggarwal, G., Anupama, A., Huang, Y., Volpin, H., Myler, P.J., Zilberstein, D., 2007. Analysis of the Leishmania donovani transcriptome reveals an ordered progression of transient and permanent changes in gene expression during differentiation. Mol. Biochem. Parasitol. 152, 53-65.
Saxena, A., Worthey, E.A., Yan, S., Leland, A., Stuart, K.D., Myler, P.J., 2003. Evaluation of differential gene expression in Leishmania major Friedlin procyclics and metacyclics using DNA microarray analysis. Mol. Biochem. Parasitol. 129, 103-114.
Searle, P.F., Chen, M.J., Hu, L., Race, P.R., Lovering, A.L., Grove, J.I., Guise, C., Jaberipour, M., James, N.D., Mautner, V., Young, L.S., Kerr, D.J., Mountain, A., White, S.A., Hyde, E.I., 2004. Nitroreductase: a prodrug -activating enzyme for cancer gene therapy. Clin. Exp. Pharmacol. Physiol. 31, 811-816.
Srividya, G., Duncan, R., Sharma, P., Raju, B.V., Nakhasi, H.L., Salotra, P., 2007. Transcriptome analysis during the process of in vitro differentiation of Leishmania donovani using genomic microarrays. Parasitology. 134, 527-539.
Sulahian, A., Garin, Y.J., Pratlong, F., Dedet, J.P., Derouin, F., 1997. Experimental pathogenicity of viscerotropic and dermotropic isolates of Leishmania infantum from immunocompromised and immunocompetent patients in a murine model. FEMS. Immunol. Med. Microbiol. 17, 131-138.
Tamar, S., Dumas, C., Papadopoulou, B., 2000. Chromosome structure and sequence organization between pathogenic and non-pathogenic Leishmania spp. Mol. Biochem. Parasitol. 111, 401-414.
Tang, B., Li, Y.N., Kruger, W.D., 2000. Defects in methylthioadenosine phosphorylase are associated with but not responsible for methionine-dependent tumor cell growth. Cancer. Res. 60, 5543-5547.
C Tuerk and L Gold Systematic evolution of ligands by exponential enrichment: RNA ligands to bacteriophage T4 DNA polymerase Science 3 August 1990 249: 505-510.Uliana, S.R., Goyal, N., Freymuller, E., Smith, D.F., 1999. Leishmania: overexpression and comparative structural analysis of the stage-regulated meta 1 gene. Exp. Parasitol. 92, 183-191.
Wang C, Zhang M, Yang G, Zhang D, Ding H, et al. (2003) Single-stranded DNA aptamers that bind differentiated but not parental cells: Subtractive systematic evolution of ligands by exponential enrichment. J Biotechnol 102: 15-22.
Wincker, P., Ravel, C., Blaineau, C., Pages, M., Jauffret, Y., Dedet, J.P., Bastien, P., 1996. The Leishmania genome comprises 36 chromosomes conserved across widely divergent human pathogenic species. Nucleic. Acids. Res. 24, 688-694.
Wincker, P., Ravel, C., Britto, C., Dubessay, P., Bastien, P., Pagès, M., Blaineau, C., 1997. A direct method for the chromosomal assignment of DNA markers in Leishmania. Gene. 194, 77-80.

## Claims

1. A method to screen for at least one genetic element differentially expressed between a first *Leishmania* sample (L₁) and a second *Leishmania* sample (L₂), comprising the steps:
a) generating a first cDNA pool and a second cDNA pool from said L₁ and said L₂ respectively;
b) contacting at least a portion of a nucleic acid library comprising genomic DNA enriched for the DNA of at least one specific chromosome of a reference *Leishmania* isolate, with said first cDNA pool and said second cDNA pool;
c) identifying at least one clone from said nucleic acid library with which said first cDNA pool and/or said second cDNA pool hybridise, so as to generate a first clone pool and a second clone pool respectively;
d) isolating at least one clone from said first clone pool which is not present in said second clone pool;
e) identifying said at least one genetic element which is differentially expressed between said L₁ and said L₂ from step d).

2. The method according to claim 1, wherein said L₁ is a visceral isolate and said L₂ is a cutaneous isolate.

3. The method according to claim 1 or 2, wherein said L₁ is a promastigote life stage and said L₂ is a amastigote life stage.

4. The method according to claim 1, 2 or 3, wherein said L₁ is PNA+ and said L₂ is PNA-.

5. The method according to claim 1, 2, 3 or 4, wherein said L₁ and said L₂ are selected from the group comprising: *L. infantum; L. major; L. braziliensis.*

6. The method according to any one of claims 1, wherein said said at least one specific chromosome is selected from the group comprising: chromosome 4, chromosome 5 and chromosome 35.

## Patentansprüche

1. Verfahren zur Durchmusterung auf wenigstens ein genetisches Element, das zwischen einer ersten *Leishmania*-Probe (L₁) und einer zweiten *Leishmania*-Probe (L₂) differenziell exprimiert wird, umfassend die Schritte:
(a) Erzeugen eines ersten cDNA-Pools und eines zweiten cDNA-Pools aus L₁ bzw. L₂;
(b) In Kontakt bringen wenigstens eines Teils einer Nukleinsäure-Bibliothek, die genomische DNA umfasst, die bezüglich der DNA wenigstens eines spezifischen Chromosoms eines Referenz-Leishmania-Isolats angereichert ist, mit dem ersten cDNA-Pool und dem zweiten cDNA-Pool;
(c) Identifizieren wenigstens eines Klons aus der Nukleinsäure-Bibliothek, mit dem der erste cDNA-Pool und/oder der zweite cDNA-Pool hybridisiert/hybridisieren, um so einen ersten Klonpool bzw. einen zweiten Klonpool zu erzeugen;
(d) Isolieren wenigstens eines Klons aus dem ersten Klonpool, der in dem zweiten Klonpool nicht vorhanden ist;
(e) Identifizieren des wenigstens einen genetischen Elements, das zwischen L₁ und L₂ differenziell exprimiert wird, aus Schritt (d).

2. Verfahren gemäß Anspruch 1, wobei L₁ ein viszerales Isolat ist und L₂ ein kutanes Isolat ist.

3. Verfahren gemäß Anspruch 1 oder 2, wobei L₁ ein promastigotes Lebensstadium ist und L₂ ein amastigotes Lebensstadium ist.

4. Verfahren gemäß Anspruch 1, 2 oder 3, wobei L₁ PNA+ ist und L₂ PNA- ist.

5. Verfahren gemäß Anspruch 1, 2, 3 oder 4, wobei L₁ und L₂ aus der Gruppe, umfassend *L. infantum; L. major; L. braziliensis,* ausgewählt sind.

6. Verfahren gemäß Anspruch 1, wobei das wenigstens eine spezifische Chromosom aus der Gruppe, umfassend Chromosom 4, Chromosom 5 und Chromosom 35, ausgewählt wird.

## Revendications

1. Méthode pour cribler au moins un élément génétique exprimé de façon différentielle entre un premier échantillon de *Leishmania* (L₁) et un second échantillon de *Leishmania* (L₂), comprenant les étapes de :
a) générer un premier ensemble d'ADNc et un second ensemble d'ADNc à partir de respectivement, lesdits L₁ et L₂;
b) mettre en contact au moins une partie d'une banque d'acide nucléique comprenant de l'ADN génomique enrichi en ADN d'au moins un chromosome spécifique d'un isolat de référence de *Leishmania,* avec ledit premier ensemble d'ADNc et ledit second ensemble d'ADNc ;
c) identifier au moins un clone de ladite banque d'acide nucléique avec lequel s'hybride ledit premier ensemble d'ADNc et ledit second ensemble d'ADNc, de façon à générer respectivement, un premier ensemble de clones et un second ensemble de clones;
d) isoler au moins un clone dudit premier ensemble de clones qui n'est pas présent dans ledit second ensemble de clones ;
e) identifier ledit au moins un élément génétique qui est exprimé de façon différentielle entre ledit L₁ et ledit L₂, à partir de l'étape d).

2. Méthode selon la revendication 1, dans laquelle ledit L₁ est un isolat viscéral et ledit L₂ est un isolat cutané.

3. Méthode selon la revendication 1 ou 2, dans laquelle ledit L₁ est au stade de vie promastigote et ledit L₂ est au stade de vie amastigote.

4. Méthode selon l'une quelconque des revendications 1, 2 ou 3, dans laquelle ledit L₁ est PNA+ et ledit L₂ est PNA-.

5. Méthode selon l'une quelconque des revendications 1, 2, 3 ou 4, dans laquelle ledit L₁ et ledit L₂ sont sélectionnés dans le groupe comprenant : *L.infantum ; L. major ; L. braziliensis.*

6. Méthode selon la revendication 1, dans laquelle ledit au moins un chromosome spécifique est sélectionné dans le groupe comprenant : le chromosome 4, le chromosome 5 et le chromosome 35.


### SEQUENCE LISTING

<110> INSTITUT PASTEUR INSTITUT PASTEUR DE TUNIS
<120> Methods to identify Leishmania virulence factors and the use of such virulence factors as therapeutic, diagnostic and vaccine targets
<130> F226 - 163 EP
<160> 43
<170> PatentIn version 3.3
<210> 1
   <211> 20
   <212> DNA
   <213> Artificial
<220>
   <223> 3547F/R Forward
<400> 1
   gcgtcgaaga tggacgtgga 20
<210> 2
   <211> 21
   <212> DNA
   <213> Artificial
<220>
   <223> 3547F/R Reverse
<400> 2
   gtcctctttc agctgcagct t 21
<210> 3
   <211> 24
   <212> DNA
   <213> Artificial
<220>
   <223> LATF/R Forward
<400> 3
   caatgagaac ctgcatgtcg ttgg 24
<210> 4
   <211> 24
   <212> DNA
   <213> Artificial
<220>
   <223> LATF/R Reverse
<400> 4
   ggaggacgtg cttcaattca tgga 24
<210> 5
   <211> 22
   <212> DNA
   <213> Artificial
<220>
   <223> 517F/R Forward
<400> 5
   cttcttcgtg tgggtgacgg ta 22
<210> 6
   <211> 20
   <212> DNA
   <213> Artificial
<220>
   <223> 517F/R Reverse
<400> 6
   ccggcggctc tttctctgtc 20
<210> 7
   <211> 21
   <212> DNA
   <213> Artificial
<220>
   <223> 542F/R Forward
<400> 7
   ctcggcgagc tcctctttcg t 21
<210> 8
   <211> 24
   <212> DNA
   <213> Artificial
<220>
   <223> 542F/R Reverse
<400> 8
   gtagacggcc ctcactacat acct 24
<210> 9
   <211> 23
   <212> DNA
   <213> Artificial
<220>
   <223> 582F/R Forward
<400> 9
   acatccttta gcaggaggcc gaa 23
<210> 10
   <211> 23
   <212> DNA
   <213> Artificial
<220>
   <223> 582F/R Reverse
<400> 10
   gcctcctcat cctcgtttcc atc 23
<210> 11
   <211> 21
   <212> DNA
   <213> Artificial
<220>
   <223> NRF/R Forward ,
<400> 11
   tcacggcatc ttcgtcgatg c 21
<210> 12
   <211> 20
   <212> DNA
   <213> Artificial
<220>
   <223> NRF/R Reverse
<400> 12
   gccacagcaa caccagcagt 20
<210> 13
   <211> 20
   <212> DNA
   <213> Artificial
<220>
   <223> LT7F/R Forward
<400> 13
   ctagaaacgc agagtacagc 20
<210> 14
   <211> 20
   <212> DNA
   <213> Artificial
<220>
   <223> LT7F/R
<400> 14
   ccctgtacat gtcactctca 20
<210> 15
   <211> 20
   <212> DNA
   <213> Artificial
<220>
   <223> MAPF/R Forward
<400> 15
   gacaagacgt acatgcgcaa 20
<210> 16
   <211> 18
   <212> DNA
   <213> Artificial
<220>
   <223> MAPF/R Reverse
<400> 16
   atgtgctccg gctttgtc 18
<210> 17
   <211> 21
   <212> DNA
   <213> Artificial
<220>
   <223> TubF/R Forward
<400> 17
   atgcgtgagg ctatctgcat c 21
<210> 18
   <211> 21
   <212> DNA
   <213> Artificial
<220>
   <223> TubF/R Reverse
<400> 18
   gtcagcacga agtggatgcg c 21
<210> 19
   <211> 21
   <212> DNA
   <213> Artificial
<220>
   <223> MetF/R Forward
<400> 19
   ttgcttggca agcacaaggt t 21
<210> 20
   <211> 21
   <212> DNA
   <213> Artificial
<220>
   <223> MetF/R Reverse
<400> 20
   gcaggaacga gcttgatgat g 21
<210> 21
   <211> 41
   <212> PRT
   <213> Leishmania infantum
<400> 21
   Leu Pro Arg His Gly Pro His His Gln Tyr Asn Pro Ser Glu Ile Asn
   1               5                   10                  15
   Tyr Arg Ala Asn Ile Cys Ala Leu Lys Gln Met Gly Val Arg Tyr Ile
               20                  25                  30
   Leu Ala Ile Asn Ala Val Gly Ser Leu
                   35                  40
<210> 22
   <211> 104
   <212> PRT
   <213> Leishmania infantum
<400> 22
   Ile Pro Tyr Lys Leu Ala Asp Ile Gly Glu Gly Ile Thr Glu Val Gln
   1 5 10 15
   Val Leu Gly Val Arg Val Lys Ala Gly Asp Thr Ile Asn Glu Phe Asp
   20 25 30
   Pro Ile Cys Glu Val Gln Ser Asp Lys Ala Thr Val Asp Ile Thr Ser
   35 40 45
   Arg Tyr Thr Gly Val Val Lys Ala Val Tyr Leu Gln Pro Gly Ala Thr
   50 55 60
   Ala Lys Val Gly Ser Val Met Leu Asp Ile Gly Asp Thr Ile Asn Glu
   65 70 75 80
   Phe Asp Pro Ile Cys Glu Val Gln Ser Asp Lys Ala Thr Val Asp Ile
   85 90 95
   Thr Ser Arg Tyr Thr Gly Val Val
   100
<210> 23
   <211> 201
   <212> PRT
   <213> Leishmania infantum
<400> 23
   Arg Trp Thr Cys Arg Gln Phe Asp Thr Ala Lys Pro Ile Asp Leu Asp
   1 5 10 15
   Thr Leu Lys Arg Val Leu Ala Ala Thr Thr Arg Ala Pro Thr Gly Phe
   20 25 30
   Asn Leu Gln Gly Trp His Ala Val Val Val Thr Asn Thr Ala Val Arg
   35 40 45
   Glu Gln Leu Phe Lys Ala Ala Leu Gly Gln Pro Gln Val Leu Gln Ala
   50 55 60
   Pro Ala Thr Val Val Phe Val Gly Asp Thr Glu Pro Glu Arg Asn Ala
   65 70 75 80
   Pro Gln Ala Leu Glu Met Gly Leu Glu Thr Gly Tyr Tyr Ser Pro Leu
   85 90 95
   Tyr Gly Ala Ala Tyr Leu Arg Asn Ile Tyr Tyr Phe Met His Gly Gly
   100 105 110
   Pro Met Gln Ser Met Ala Ala Val Lys Ser Val Val Ser Ala Trp Tyr
   115 120 125
   Ser Gln Ala Ala Gly Thr Pro Leu Ile Ser Val Pro Val Ser Arg Ala
   130 135 140
   Gly Tyr Ala Trp Lys Gln Thr Met Ile Pro Ala Thr Thr Phe Val Asn
   145 150 155 160
   Leu Cys Ala Ala Ala Gly Trp Asp Thr Cys Met Met Glu Gly Ile Asp
   165 170 175
   Glu Asp Ala Val Lys Arg Ala Leu Gly Val Pro Ala Glu Arg Tyr Thr
   180 185 190
   Val Pro Val Ile Ile Ser Val Gly Tyr
   195 200
<210> 24
   <211> 179
   <212> PRT
   <213> Leishmania infantum
<400> 24
   Ser Lys Gln Glu Tyr Trp Asp Arg Arg Tyr Thr Glu Glu Glu His Tyr
   1 5 10 15
   Asp Trp Phe Pro Ser Val Tyr Pro Met Cys Val Ala Ala Ala Phe Glu
   20 25 30
   Thr Val Glu Ala Val Tyr Arg Val Gln Arg Ser Thr Gly Ala Phe Glu
   35 40 45
   Gly Thr Leu Lys Val Leu His Leu Gly Thr Gly Ser Ser Thr Leu Cys
   50 55 60
   Ala Asp Ile Cys Ala Ala Tyr Glu Ala Lys Tyr Pro Thr Glu Asp Ser
   65 70 75 80
   Arg Pro Tyr Arg Leu Val Gln Val Ala Thr Asp Tyr Ser Ala Val Val
   85 90 95
   Ile Glu His Met Lys Thr Lys Tyr Gly Pro Ala His Pro Leu Glu Asp
   100 105 110
   Val His Trp Val Val Ala Asp Ile Arg Asp Leu Arg Arg Val Arg Glu
   115 120 125
   Gln Phe Gly Pro Phe Phe Asp Val Val Leu Asp Lys Gly Thr Met Asp
   130 135 140
   Ala Leu Gln Ala Asp Lys Ala Asn Gln Asn Met Asp Asp Asp Ile Glu
   145 150 155 160
   Arg Met Leu Cys Glu Val Ser Arg Cys Val Glu Gly Gly Val Gly Thr
   165 170 175
   Leu Val Tyr
<210> 25
   <211> 736
   <212> DNA
   <213> Leishmania infantum
<400> 25
   atgatggagc cgactagaaa cgcagagtac agcaagcagg aatactggga ccggcgctac 60
   acggaggagg agcactatga ctggtttccc tccgtgtacc catgtgtgtc gctgcagcct 120
   ttgagaccgt tgaggctgtc tatcgtgtgc agcgcagcac aggggcattc gaaggcacgc 180
   taaaggtgtt gcatcttggt actggtagct caaccctctg cgccgacatc tgcgccgcct 240
   acgaggcaaa atatccgacg gaagactcga ggccctaccg actcgtgcag gtggccactg 300
   actactctgc tgtggtcatc gagcatatga agaccaagta cggtccagcc catccactgg 360
   aagacgtaca ctgggtggtt gccgatatcc gcgatctccg tcgcgtgcgt gagcagtttg 420
   gtcctttctt cgacgtggtg ctggacaagg gcaccatgga cgcgctgcag gcggacaagg 480
   caaaccagaa tatggacgac gacattgaac ggatgctctg cgaggtgaga ggtgcgtcga 540
   gggcggtgtt ggcacgctcg tgtatcgtgt gtttgtgcag atcacgtggg aaattcctta 600
   cttgcgcctt cactacacca cgaagaatgc cacgcacacc ttcgcctggg gcaccaatgt 660
   cacctaccgc tttctgggtg agagtgacat gtacaggatg tacacgtacg aggtgtcacc 720
   gccctcttcc gagtag 736
<210> 26
   <211> 245
   <212> PRT
   <213> Leishmania infantum
<400> 26
   Met Met Glu Pro Thr Arg Asn Ala Glu Tyr Ser Lys Gln Glu Tyr Trp
   1               5                   10                  15
   Asp Arg Arg Tyr Thr Glu Glu Glu His Tyr Asp Trp Phe Pro Ser Val
               20                  25                  30
   Tyr Pro Met Cys Val Ala Ala Ala Phe Glu Thr Val Glu Ala Val Tyr
           35                  40                  45
   Arg Val Gln Arg Ser Thr Gly Ala Phe Glu Gly Thr Leu Lys Val Leu
       50                  55                  60
   His Leu Gly Thr Gly Ser Ser Thr Leu Cys Ala Asp Ile Cys Ala Ala
   65                  70                  75                  80
   Tyr Glu Ala Lys Tyr Pro Thr Glu Asp Ser Arg Pro Tyr Arg Leu Val
                   85                  90                  95
   Gln Val Ala Thr Asp Tyr Ser Ala Val Val Ile Glu His Met Lys Thr
               100                 105                 110
   Lys Tyr Gly Pro Ala His Pro Leu Glu Asp Val His Trp Val Val Ala
           115                 120                 125
   Asp Ile Arg Asp Leu Arg Arg Val Arg Glu Gln Phe Gly Pro Phe Phe
       130                 135                 140
   Asp Val Val Leu Asp Lys Gly Thr Met Asp Ala Leu Gln Ala Asp Lys
   145                 150                 155                 160
   Ala Asn Gln Asn Met Asp Asp Asp Ile Glu Arg Met Leu Cys Glu Val
                   165                 170                 175
   Ser Arg Cys Val Glu Gly Gly Val Gly Thr Leu Val Tyr Arg Val Phe
               180                 185                 190
   Val Gln Ile Thr Trp Glu Ile Pro Tyr Leu Arg Leu His Tyr Thr Thr
           195                 200                 205
   Lys Asn Ala Thr His Thr Phe Ala Trp Gly Thr Asn Val Thr Tyr Arg
       210                 215                 220
   Phe Leu Gly Glu Ser Asp Met Tyr Arg Met Tyr Thr Tyr Glu Val Ser
   225                 230                 235                 240
   Pro Pro Ser Ser Glu
                   245
<210> 27
   <211> 2280
   <212> DNA
   <213> Leishmania infantum
<400> 27
   atggtgtccg tgttagtaca gaggcagcaa catgagaccg ccaggcgcaa ggagcgcctt    60
   cgcgccgccc accaggcagc ggcggcgctc tccatccagc aggctcgcgc ctatcagaag   120
   cagaagcggc aacaggaggc gtgcgttcgc gaggacgccc gtcagctgtg gttgcaatcg   180
   aacgcgcgcg acttggcggc catcgacaga ctcgttgcgc gcgccactac cgagcagggc   240
   gaagcagagg aggcggctgg gcactacgag gccaatctgc aggcggaggc gtgcgaggag   300
   gcggccgcgt ggcaggcgga gcggcagttg gaggaagcac ggcaccgcct cgccgttgcc 360
   tacacccgaa cacagacgca tgagcggacg aagccggcac gccaagccga gcagcgtcgt 420
   acagccgtgc gtgtgacgga gaaggtgagg gcggcgcggg tggcggctgc ggcaccggcg 480
   gccctttctc tgtcgagcgt tctaggcagc ttgcagggcg cgccagtggc cacgcacgta 540
   ccgcgcgcca gcgtccccgt gccgcgacca agtctcgagc ccctgcgtaa cgttgctggg 600
   tcaaccgctg gagcagccct tgccgatgcc ccgcctgctg cacagtcgcc cctgcgccgt 660
   tggcgcatat cccacgtcgc cccgcacgtg catgtcacct tgcacggtga cccgacgcat 720
   cgcgtcaccg ctcgtgaaga gaacgagata agcgctctgg cggccgcgga tgatgcagtc 780
   cctgcttctg cagacgccta tgcagcagaa tgtgcagctg tccagcagca gagggacgag 840
   gtactgaaag tcggccgaga gcgtgcagcg cagagggctg cgtctctctt gcgccaccag 900
   cgtgcgaagg agctgcagga agaggaggag cagcagacac agcgggagcg gcttgctgcg 960
   gtcaaggaac gctaccgtca cccacacgaa gaaggcgccc caagtggcgg caagaaagcc 1020
   gcggcgccgg aagtcggcac acaccctgcc acagccacaa ccggcagcag cagcggcagt 1080
   aacggcagtc cgaatcacca acaccgacgt cctcaacggc agcgtacgcc tcagcggctt 1140
   gcctgccatc agcaagcata cataaagggc gaagaagaat tccgcgcggc gttcgtggag 1200
   gcgccacccc tcgtggtgcg cctggatgaa gagccgccgc gtcgtgcacc gctaagcgag 1260
   ctcttgagac ccgtgaaggt ggcggaactg ctgtacagca ttgcaaactc tgtcccagta 1320
   gatcccactc cagtcacacc ggcgccgcat catggatccg gcgccgccgc agccggttca 1380
   ccgtcttttc cgatgacagg cgaggtgctg ccatccgcgc aaccctccgc cagagcgcgc 1440
   gtgctgccat cagtgcctct ccgcatgctg cccctcgatg agcgaagcag cgctgctcgc 1500
   gcgaaagctg agtcggcacc agaggacagg gctgctgcga caacacctct gagcagcggc 1560
   gaggagggct gctcgttgcc gccgccgcca cctgcgccga gaggctccag cggcgcgcca 1620
   cgcgcttctc ttcgcgcgtc tgcagaggcg tcgaccgcac cgtcgccgat gcggctcgat 1680
   gttccagtga gccgtacatc gcgcgcgcgg acgccgtcgc cagagaaggc tgcgactgta 1740
   gaggagcttg tcgctctctc tcctctcacc ccggctcccc ctgcggcggg cacatgcgtg 1800
   cccgagtcac cgtctccgcc gccaccgtgc gctggggggg ctttgagaga gcttgctcaa 1860
   acagctgcta acatgggtgc ttcagatgtg acgatggctt atcggcagca gcccttgaca 1920
   gcgccagggg ggagacgggc cacttccgcc cacgagaatg cgccagcatc gccatctgcg 1980
   ctatcctcga tgatggagcg cacaccggct caccaccgcc accgtcgcag cgccacgccg 2040
   ccgtcgtcgc tgtcctcctc cactaccacc accactgaga ctgccgagcc gccgagcggc 2100
   tcctcttccc cggcctccgt ggaaggcagc agcagcagcg gtaccgacgc ctcctcctct 2160
   tcatatgaca gcgccagcac cagtggatcg tcacgccacc cgatgccggt gatgacggcg 2220
   gaacagctaa agctggcact cctgcgtctg cgcagccgca tcaggtcagc acagatgtag 2280
<210> 28
   <211> 759
   <212> PRT
   <213> Leishmania infantum
<400> 28
   Met Val Ser Val Leu Val Gln Arg Gln Gln His Glu Thr Ala Arg Arg
   1 5 10 15
   Lys Glu Arg Leu Arg Ala Ala His Gln Ala Ala Ala Ala Leu Ser Ile
   20 25 30
   Gln Gln Ala Arg Ala Tyr Gln Lys Gln Lys Arg Gln Gln Glu Ala Cys
   35 40 45
   Val Arg Glu Asp Ala Arg Gln Leu Trp Leu Gln Ser Asn Ala Arg Asp
   50 55 60
   Leu Ala Ala Ile Asp Arg Leu Val Ala Arg Ala Thr Thr Glu Gln Gly
   65 70 75 80
   Glu Ala Glu Glu Ala Ala Gly His Tyr Glu Ala Asn Leu Gln Ala Glu
   85 90 95
   Ala Cys Glu Glu Ala Ala Ala Trp Gln Ala Glu Arg Gln Leu Glu Glu
   100 105 110
   Ala Arg His Arg Leu Ala Val Ala Tyr Thr Arg Thr Gln Thr His Glu
   115 120 125
   Arg Thr Lys Pro Ala Arg Gln Ala Glu Gln Arg Arg Thr Ala Val Arg
   130 135 140
   Val Thr Glu Lys Val Arg Ala Ala Arg Val Ala Ala Ala Ala Pro Ala
   145 150 155 160
   Ala Leu Ser Leu Ser Ser Val Leu Gly Ser Leu Gln Gly Ala Pro Val
   165 170 175
   Ala Thr His Val Pro Arg Ala Ser Val Pro Val Pro Arg Pro Ser Leu
   180 185 190
   Glu Pro Leu Arg Asn Val Ala Gly Ser Thr Ala Gly Ala Ala Leu Ala
   195 200 205
   Asp Ala Pro Pro Ala Ala Gln Ser Pro Leu Arg Arg Trp Arg Ile Ser
   210 215 220
   His Val Ala Pro His Val His Val Thr Leu His Gly Asp Pro Thr His
   225 230 235 240
   Arg Val Thr Ala Arg Glu Glu Asn Glu Ile Ser Ala Leu Ala Ala Ala
   245 250 255
   Asp Asp Ala Val Pro Ala Ser Ala Asp Ala Tyr Ala Ala Glu Cys Ala
   260 265 270
   Ala Val Gln Gln Gln Arg Asp Glu Val Leu Lys Val Gly Arg Glu Arg
   275 280 285
   Ala Ala Gln Arg Ala Ala Ser Leu Leu Arg His Gln Arg Ala Lys Glu
   290 295 300
   Leu Gln Glu Glu Glu Glu Gln Gln Thr Gln Arg Glu Arg Leu Ala Ala
   305 310 315 320
   Val Lys Glu Arg Tyr Arg His Pro His Glu Glu Gly Ala Pro Ser Gly
   325 330 335
   Gly Lys Lys Ala Ala Ala Pro Glu Val Gly Thr His Pro Ala Thr Ala
   340 345 350
   Thr Thr Gly Ser Ser Ser Gly Ser Asn Gly Ser Pro Asn His Gln His
   355 360 365
   Arg Arg Pro Gln Arg Gln Arg Thr Pro Gln Arg Leu Ala Cys His Gln
   370 375 380
   Gln Ala Tyr Ile Lys Gly Glu Glu Glu Phe Arg Ala Ala Phe Val Glu
   385 390 395 400
   Ala Pro Pro Leu Val Val Arg Leu Asp Glu Glu Pro Pro Arg Arg Ala
   405 410 415
   Pro Leu Ser Glu Leu Leu Arg Pro Val Lys Val Ala Glu Leu Leu Tyr
   420 425 430
   Ser Ile Ala Asn Ser Val Pro Val Asp Pro Thr Pro Val Thr Pro Ala
   435 440 445
   Pro His His Gly Ser Gly Ala Ala Ala Ala Gly Ser Pro Ser Phe Pro
   450 455 460
   Met Thr Gly Glu Val Leu Pro Ser Ala Gln Pro Ser Ala Arg Ala Arg
   465 470 475 480
   Val Leu Pro Ser Val Pro Leu Arg Met Leu Pro Leu Asp Glu Arg Ser
   485 490 495
   Ser Ala Ala Arg Ala Lys Ala Glu Ser Ala Pro Glu Asp Arg Ala Ala
   500 505 510
   Ala Thr Thr Pro Leu Ser Ser Gly Glu Glu Gly Cys Ser Leu Pro Pro
   515 520 525
   Pro Pro Pro Ala Pro Arg Gly Ser Ser Gly Ala Pro Arg Ala Ser Leu
       530                 535                 540
   Arg Ala Ser Ala Glu Ala Ser Thr Ala Pro Ser Pro Met Arg Leu Asp
   545                 550                 555                 560
   Val Pro Val Ser Arg Thr Ser Arg Ala Arg Thr Pro Ser Pro Glu Lys
                   565                 570                 575
   Ala Ala Thr Val Glu Glu Leu Val Ala Leu Ser Pro Leu Thr Pro Ala
               580                 585                 590
   Pro Pro Ala Ala Gly Thr Cys Val Pro Glu Ser Pro Ser Pro Pro Pro
           595                 600                 605
   Pro Cys Ala Gly Gly Ala Leu Arg Glu Leu Ala Gln Thr Ala Ala Asn
       610                 615                 620
   Met Gly Ala Ser Asp Val Thr Met Ala Tyr Arg Gln Gln Pro Leu Thr
   625                 630                 635                 640
   Ala Pro Gly Gly Arg Arg Ala Thr Ser Ala His Glu Asn Ala Pro Ala
                   645                 650                 655
   Ser Pro Ser Ala Leu Ser Ser Met Met Glu Arg Thr Pro Ala His His
               660                 665                 670
   Arg His Arg Arg Ser Ala Thr Pro Pro Ser Ser Leu Ser Ser Ser Thr
           675                 680                 685
   Thr Thr Thr Thr Glu Thr Ala Glu Pro Pro Ser Gly Ser Ser Ser Pro
       690                 695                 700
   Ala Ser Val Glu Gly Ser Ser Ser Ser Gly Thr Asp Ala Ser Ser Ser
   705                 710                 715                 720
   Ser Tyr Asp Ser Ala Ser Thr Ser Gly Ser Ser Arg His Pro Met Pro
                   725                 730                 735
   Val Met Thr Ala Glu Gln Leu Lys Leu Ala Leu Leu Arg Leu Arg Ser
               740                 745                 750
   Arg Ile Arg Ser Ala Gln Met
           755
<210> 29
   <211> 921
   <212> DNA
   <213> Leishmania infantum
<400> 29
   atgtacggta atccgcacaa ggagcccgtc gcgatcgccg tcatcggcgg ctctggcatc    60
   tacaagctgg actgcttgca ggatgcaaag tactacgacg taccgacccc gtatggcaac   120
   ccgagcgggc agctgtgcgt ggccaaggtc gacggtgtgc cgtgcgtctt cctgccgcgt   180
   cacggtccgc accaccagta caaccccagc gagatcaact accgcgccaa catctgcgcg   240
   ctgaagcaga tgggcgtgcg ctacatcctc gccatcaacg ctgttggatc gctggacgag   300
   tcctgcaagc ccggcgacct cgtgctgtgc gaccagatca tcgacaagac gtacgcgcgc   360
   aaggcaacct tcttcgaggg cggcgtcgtc gcgcacgcgg acttcgccca cccgacgtcg   420
   cgcatcttca acaacatcgc gcatgaggcg ctgctgcgct gctttccgga cgtggcggcc   480
   ggcaagggca cgttccagat tcacagcagc ggcaccctcg tgacgatgga aggtccgcag   540
   ttcagcacta aggccgagtc cctcctcaac aagcagatgg gcggccacct gatcggtatg   600
   acgtcggcaa cggaggcgcg tctggcgcgt gaggcggaga tcgcatacgc gacagtggcg   660
   atggtgaccg acatggacgc gtggagcgac gcgccgcacg tggacgcggc gcaggtgacg   720
   aaggtgatgg cggcgaatgt ggagaaggcg cagcgctacc cgtccgagat catcaagtcc   780
   ctcgcccaga atctcttcga tgatccggcg caccacaccc tggagtacgc catcgtgaca   840
   aagccggagc acatccccgc cgagacgaag cagcgcatcg cgccgctcgt ggcgagcaag   900
   tacccgcagt tcgccccgta g                                             921
<210> 30
   <211> 306
   <212> PRT
   <213> Leishmania infantum
<400> 30
   Met Tyr Gly Asn Pro His Lys Glu Pro Val Ala Ile Ala Val Ile Gly
   1               5                   10                  15
   Gly Ser Gly Ile Tyr Lys Leu Asp Cys Leu Gln Asp Ala Lys Tyr Tyr
               20                  25                  30
   Asp Val Pro Thr Pro Tyr Gly Asn Pro Ser Gly Gln Leu Cys Val Ala
           35                  40                  45
   Lys Val Asp Gly Val Pro Cys Val Phe Leu Pro Arg His Gly Pro His
       50                  55                  60
   His Gln Tyr Asn Pro Ser Glu Ile Asn Tyr Arg Ala Asn Ile Cys Ala
   65                  70                  75                  80
   Leu Lys Gln Met Gly Val Arg Tyr Ile Leu Ala Ile Asn Ala Val Gly
                   85                  90                  95
   Ser Leu Asp Glu Ser Cys Lys Pro Gly Asp Leu Val Leu Cys Asp Gln
               100                 105                 110
   Ile Ile Asp Lys Thr Tyr Ala Arg Lys Ala Thr Phe Phe Glu Gly Gly
   115 120 125
   Val Val Ala His Ala Asp Phe Ala His Pro Thr Ser Arg Ile Phe Asn
   130 135 140
   Asn Ile Ala His Glu Ala Leu Leu Arg Cys Phe Pro Asp Val Ala Ala
   145 150 155 160
   Gly Lys Gly Thr Phe Gln Ile His Ser Ser Gly Thr Leu Val Thr Met
   165 170 175
   Glu Gly Pro Gln Phe Ser Thr Lys Ala Glu Ser Leu Leu Asn Lys Gln
   180 185 190
   Met Gly Gly His Leu Ile Gly Met Thr Ser Ala Thr Glu Ala Arg Leu
   195 200 205
   Ala Arg Glu Ala Glu Ile Ala Tyr Ala Thr Val Ala Met Val Thr Asp
   210 215 220
   Met Asp Ala Trp Ser Asp Ala Pro His Val Asp Ala Ala Gln Val Thr
   225 230 235 240
   Lys Val Met Ala Ala Asn Val Glu Lys Ala Gln Arg Tyr Pro Ser Glu
   245 250 255
   Ile Ile Lys Ser Leu Ala Gln Asn Leu Phe Asp Asp Pro Ala His His
   260 265 270
   Thr Leu Glu Tyr Ala Ile Val Thr Lys Pro Glu His Ile Pro Ala Glu
   275 280 285
   Thr Lys Gln Arg Ile Ala Pro Leu Val Ala Ser Lys Tyr Pro Gln Phe
   290 295 300
   Ala Pro
   305
<210> 31
   <211> 1143
   <212> DNA
   <213> Leishmania infantum
<400> 31
   atgcgtgctg cgagttgcat ggcgcaccgc tgcatcggcg cggctgccgg tgccgtcact     60
   gtcgcccgcg cgcctggctg gccctacacg gcgtcgcagc ggcacttctt tgcgacggcg    120
   tgcgcactgc tcggccgcag catcccgtac aagctggccg acatcggcga agggataaca    180
   gaggtgcagg tgctgggcgt ccgcgtgaag gccggcgaca ccatcaacga gttcgacccc    240
   atctgcgagg tgcaaagcga caaggccacc gtcgacatca cgtcccgcta cacgggcgtt    300
   gtgaaggcgg tgtacctgca gcctggcgcg accgccaagg tgggctcggt catgctcgac    360
   atcgtgccag agggcgccga cgacgcgcca gaggccgcct cgccgtcccg cggcgcgcca    420
   tcgccttcgt cggcgtcgca ttctgctttg caggcagcct actccgcgcc acagccatca    480
   tcggtcccct cggcgggtaa ggtgcttgcg acaccggcaa cgcggtacct ggcgcgggaa    540
   cacaagctgg acctcgcaca tgtgccggcc accggcaagg gcggacgagt aacaaaggag    600
   gacgtgcttc aattcatgga tgcgagcatg tctgcagcag cggcgccatc gaccccatct    660
   acggcgtcgg ttgccgcaac tgcgccacct ggcaccgtcg tgtctggact tcagacggag    720
   gcgggtgaca ccgtcatgcc catcaccggc gtgcgccgcg gcatggtcaa gacgatgagc    780
   caggctgcgt cgatccccac cttcaccttc agcgaggagt gcgagctcac ccggctcatg    840
   gcggtgcgcg ggtcgctgaa ggatatggtg aaggaccgca gcaaaggcaa ggcaaagctg    900
   tccttcatgc ctttctttct caaggcggcg tctatcgcgc tgcagcatca cccggacatc    960
   aacgctcact gcccggcgga ctgctcggcg ttggtgcgca aggcggcaca caacatcggc   1020
   ttcgctatgg acacgccgaa cgggctcatc gtgccggttg ttaaacatgt ggagcgcaag   1080
   tcaatcctcg acatcgccaa cgacatgcag gttctcattg agcgcggcag agcaacaagc   1140
   tga                                                                 1143
<210> 32
   <211> 380
   <212> PRT
   <213> Leishmania infantum
<400> 32
   Met Arg Ala Ala Ser Cys Met Ala His Arg Cys Ile Gly Ala Ala Ala
   1               5                   10                  15
   Gly Ala Val Thr Val Ala Arg Ala Pro Gly Trp Pro Tyr Thr Ala Ser
               20                  25                  30
   Gln Arg His Phe Phe Ala Thr Ala Cys Ala Leu Leu Gly Arg Ser Ile
           35                  40                  45
   Pro Tyr Lys Leu Ala Asp Ile Gly Glu Gly Ile Thr Glu Val Gln Val
       50                  55                  60
   Leu Gly Val Arg Val Lys Ala Gly Asp Thr Ile Asn Glu Phe Asp Pro
   65                  70                  75                  80
   Ile Cys Glu Val Gln Ser Asp Lys Ala Thr Val Asp Ile Thr Ser Arg
                   85                  90                  95
   Tyr Thr Gly Val Val Lys Ala Val Tyr Leu Gln Pro Gly Ala Thr Ala
               100                 105                 110
   Lys Val Gly Ser Val Met Leu Asp Ile Val Pro Glu Gly Ala Asp Asp
           115                 120                 125
   Ala Pro Glu Ala Ala Ser Pro Ser Arg Gly Ala Pro Ser Pro Ser Ser
   130 135 140
   Ala Ser His Ser Ala Leu Gln Ala Ala Tyr Ser Ala Pro Gln Pro Ser
   145 150 155 160
   Ser Val Pro Ser Ala Gly Lys Val Leu Ala Thr Pro Ala Thr Arg Tyr
   165 170 175
   Leu Ala Arg Glu His Lys Leu Asp Leu Ala His Val Pro Ala Thr Gly
   180 185 190
   Lys Gly Gly Arg Val Thr Lys Glu Asp Val Leu Gln Phe Met Asp Ala
   195 200 205
   Ser Met Ser Ala Ala Ala Ala Pro Ser Thr Pro Ser Thr Ala Ser Val
   210 215 220
   Ala Ala Thr Ala Pro Pro Gly Thr Val Val Ser Gly Leu Gln Thr Glu
   225 230 235 240
   Ala Gly Asp Thr Val Met Pro Ile Thr Gly Val Arg Arg Gly Met Val
   245 250 255
   Lys Thr Met Ser Gln Ala Ala Ser Ile Pro Thr Phe Thr Phe Ser Glu
   260 265 270
   Glu Cys Glu Leu Thr Arg Leu Met Ala Val Arg Gly Ser Leu Lys Asp
   275 280 285
   Met Val Lys Asp Arg Ser Lys Gly Lys Ala Lys Leu Ser Phe Met Pro
   290 295 300
   Phe Phe Leu Lys Ala Ala Ser Ile Ala Leu Gln His His Pro Asp Ile
   305 310 315 320
   Asn Ala His Cys Pro Ala Asp Cys Ser Ala Leu Val Arg Lys Ala Ala
   325 330 335
   His Asn Ile Gly Phe Ala Met Asp Thr Pro Asn Gly Leu Ile Val Pro
   340 345 350
   Val Val Lys His Val Glu Arg Lys Ser Ile Leu Asp Ile Ala Asn Asp
   355 360 365
   Met Gln Val Leu Ile Glu Arg Gly Arg Ala Thr Ser
   370 375 380
<210> 33
   <211> 972
   <212> DNA
   <213> Leishmania infantum
<400> 33
   atgcttcgcc gcagtcgccg cttgctgttg gctgcagcag gggcccgccc tgccgccttc 60
   acccctcgag ccagagaggc cgccggcacc tgcggcaacg aggagcgcga cagcaacacc 120
   agcagtggcc atggcggctt tctagccacg ctgcgccatc tcgtggcgtg gaaccgcacg 180
   aacgcggcgg ccgccgcatc gtcgtcatcc gcgtcgtgtg cagaaacccc agcatgcgtc 240
   agcaacaacg cagcactcga cgccgtcgag gccgtcgtgc gcgcccggtg gacgtgccgg 300
   cagttcgaca cggcgaagcc gatagacctc gacaccctca agcgcgtctt ggcggcgacg 360
   acccgcgccc cgaccggttt caaccttcag ggctggcacg cggtcgtcgt caccaacacg 420
   gcggtgcggg agcagctctt caaggcagcg ctcggccagc cgcaagtgct gcaggcgccg 480
   gcgactgtcg tcttcgttgg agacacggag ccggagcgga acgcgccgca ggcgcttgag 540
   atggggctcg agacgggcta ctatagcccc ctctacggtg ccgcctacct gcgcaacatc 600
   tactacttca tgcatggcgg tccgatgcag tcgatggccg ccgtgaagtc ggtcgtgagt 660
   gcgtggtaca gccaggcggc tggcacgccg ctcatctccg tcccggtgag ccgcgccggc 720
   tacgcgtgga agcagacgat gattccggcg acgacgttcg tgaatctctg cgccgccgcc 780
   ggctgggaca cgtgcatgat ggagggcatc gacgaggatg ccgtcaagcg agccttggga 840
   gtgccggcgg aacggtacac ggtgccggtc atcatcagcg tcggctacgc gaccgccgcg 900
   gaggccgaga aacgccaggt gggcagctcc cgcttcgcga cgccgcacac cgtgcggtgg 960
   aacaagttct ag 972
<210> 34
   <211> 323
   <212> PRT
   <213> Leishmania infantum
<400> 34
   Met Leu Arg Arg Ser Arg Arg Leu Leu Leu Ala Ala Ala Gly Ala Arg
   1 5 10 15
   Pro Ala Ala Phe Thr Pro Arg Ala Arg Glu Ala Ala Gly Thr Cys Gly
   20 25 30
   Asn Glu Glu Arg Asp Ser Asn Thr Ser Ser Gly His Gly Gly Phe Leu
   35 40 45
   Ala Thr Leu Arg His Leu Val Ala Trp Asn Arg Thr Asn Ala Ala Ala
   50 55 60
   Ala Ala Ser Ser Ser Ser Ala Ser Cys Ala Glu Thr Pro Ala Cys Val
   65 70 75 80
   Ser Asn Asn Ala Ala Leu Asp Ala Val Glu Ala Val Val Arg Ala Arg
   85 90 95
   Trp Thr Cys Arg Gln Phe Asp Thr Ala Lys Pro Ile Asp Leu Asp Thr
   100 105 110
   Leu Lys Arg Val Leu Ala Ala Thr Thr Arg Ala Pro Thr Gly Phe Asn
   115 120 125
   Leu Gln Gly Trp His Ala Val Val Val Thr Asn Thr Ala Val Arg Glu
   130 135 140
   Gln Leu Phe Lys Ala Ala Leu Gly Gln Pro Gln Val Leu Gln Ala Pro
   145 150 155 160
   Ala Thr Val Val Phe Val Gly Asp Thr Glu Pro Glu Arg Asn Ala Pro
   165 170 175
   Gln Ala Leu Glu Met Gly Leu Glu Thr Gly Tyr Tyr Ser Pro Leu Tyr
   180 185 190
   Gly Ala Ala Tyr Leu Arg Asn Ile Tyr Tyr Phe Met His Gly Gly Pro
   195 200 205
   Met Gln Ser Met Ala Ala Val Lys Ser Val Val Ser Ala Trp Tyr Ser
   210 215 220
   Gln Ala Ala Gly Thr Pro Leu Ile Ser Val Pro Val Ser Arg Ala Gly
   225 230 235 240
   Tyr Ala Trp Lys Gln Thr Met Ile Pro Ala Thr Thr Phe Val Asn Leu
   245 250 255
   Cys Ala Ala Ala Gly Trp Asp Thr Cys Met Met Glu Gly Ile Asp Glu
   260 265 270
   Asp Ala Val Lys Arg Ala Leu Gly Val Pro Ala Glu Arg Tyr Thr Val
   275 280 285
   Pro Val Ile Ile Ser Val Gly Tyr Ala Thr Ala Ala Glu Ala Glu Lys
   290 295 300
   Arg Gln Val Gly Ser Ser Arg Phe Ala Thr Pro His Thr Val Arg Trp
   305 310 315 320
   Asn Lys Phe
<210> 35
   <211> 3255
   <212> DNA
   <213> Leishmania infantum
<400> 35
   atgccaatcg ctcctcgcca aggccgcaaa ggggggaaga tatcctcctc gccggctatc 60
   gcctcgtcgg actcgccgtc gatggtgtcg cctggttcgc tgccgtctgg cagcgctggc 120
   atctccctcc ccacagatcc taacacgact ctgcaattca acatcgaggc tctgatgagt 180
   aagaagcgtc gcagcccttc caaggcaaag gctgcggcgc gcgcgaacga gtccagcgca 240
   tccgcttcct cggcgacgga ggcggtcaag gcagccagcg gcacaagggg cgcctccacc 300
   accgcggctg cggggtccgc cggggcgtct gccgcggccg gtcccgcgga gacaatcgcg 360
   aacccctcgc ttgtgacgcc acccggcgcg gactcgaacg tcttcaagtt cgacctcgac 420
   gccatccagc aagttggcca gggcgtcgtg ttcggcggca ataagacgat gcggacggat 480
   tcgcacaagc ccgattcgga cccagcggtg gtgtctgcaa agctgaaaca gctgatcgcc 540
   tcccaggacg catacttcca cggtggcaac gccgcggccg ccgacgtcga gagcgacgaa 600
   gtgctggggt taaaggaggt agcctcacat caagtagtgc tgctgcagcg accctacgcg 660
   gctctgcgtc tcgctgtcgc gcttagccga aaaagcaaag agcgcgtgct ggtggtggtg 720
   ccgcattact cagacgtctc cgatatcgcg gcggcgctgc gggcgctgag cccatctcgc 780
   ggccccgtcg gcgagctgag cgggcagtcg tttgtcggcg aggcggacac agcactgtgg 840
   gtgacggatg tggatacggc gcttgtgtac ctaacatcgc agcgcggcat ggggccgttc 900
   acgcatctcg tgctgcccaa cctgacacgc atgaatcccc tggtctccta cttcttgtgg 960
   ggtctgcggg aacgcgtgta ccggcaaagc gccatcctgg acacgaccgc cccgccgctg 1020
   cacgtcattg taagcgtcag cggtgccatg acggagcgga tgcagcagtt cttcgcgaag 1080
   cagacggtgg cgacgccaaa cacggcgccg gtgcagcccc tcgttgagtt ctcgtacgat 1140
   gaggcgaacg cgctggcaga gatggacgtg ttggatgtca cgatcgatga gtctggcaag 1200
   taccctggcc cacaccgcaa gatcatcgac cactccgtgc aaatggcggc gacgctggtg 1260
   ggccgtattt tctcctgctc cgccgcgtgc ccgcaggcta tttacatcct cactggtgac 1320
   tcacgcgaga tgatcgacgc gctacacaag gagcgccttc tcgacacaac ggtttatgcc 1380
   gggcgcttcc cctccaagga ggacgccggc gccacaccgt cgaagcatcg tgtgtgcgtg 1440
   ctgcactccg tcgtctcctt cacgaactac aacaacgaag atgcgacgtt tgtgctggac 1500
   atggcaacga cccgccgcct cgccgtacag aacaagtcgg agggcttcat ggcttcgagc 1560
   gcgtcggagt gggcgtcgaa gatggacgtg gaggagcgcc ggcagctgcc cggcgccaag 1620
   taccccggct gctacatcgc cctgtatccc ccggcggccg atgccgtgct gcccactacc 1680
   gaggctcccc agccgacggt atacgaggtg gaggacgcgc tgctgcagtg ctcacgagcg 1740
   cagctgccga tccagcgtgc gaaccaggag atggtgtgcc cggtggaggc ggagagcatt 1800
   gaacaggtgc agcacagtct ctcggagaag tgcctcattg cgaacctgtc cgatttcagc 1860
   ctcaccttta cgggcgagat agcgtcgcgg ctgcccctcg aggtggactt ggctcatttc 1920
   gtcatgaact gctgcacact tggccacggc gaggtcggcg tcattgtggc cggcgtctgc 1980
   gctctgccat accgatttcc gaccggcgag tcagcagagg ggtttgcctc gtggaagagg 2040
   tgtgtgattg agacacgcca agagtacgct ggtgacattg ccaatcagag tgatctcttg   2100
   gccgatgtgc tggtgttcct ggagtggtgg cggctcaagg cgagcggagc ctcgacagcc   2160
   gactttgtgg caaagatgca gctgaaagag gaccgcctcg agcacgtgcg cggcctcatc   2220
   acctacctgc gcgtccagat ctctgactac gctttcgtcg acgacctcga ggacgaggca   2280
   accctgtcgg cggtgatgca gagcatcaag tcaaacgcgt ccatctttac cttctttgag   2340
   gccgtcgccc tcgctcgccg gctcttcttt gtacgcgacg ccggctccat caacgtcaag   2400
   gatcgggctg gcgccttggt ctttgtgcgg acgtcgaaga aggttgtgcc caacaccgcc   2460
   agcccgagca gcgtggtgtg ggagtccggc gccgccttgg tcgccgtgga cctgcgtaat   2520
   ctcgttagca acatcacctg cgctcgcgtt tcggcggtga acaacactta cctgtttgcg   2580
   gcgctgctgc tgttgtaccc gcaggtggag tactctgccc cagtcgaggt gccctccaag   2640
   ggtggccgcg tcgtgtactt tggcgtcaca tgcaaccgcc agatgaagcg ctttcgcgtc   2700
   agcatcggtg aggctgcgca gattcttgat ttccgagaga agtggaacca tgcgatacgg   2760
   tacctgcagg tcctccgcac agcaaagaag ccgctatcgc ggcacaagtt caactttatg   2820
   ctgaaggaag aggaccgcca cttcgatttg gaggagctgc gcgcggaggt gcagcgggag   2880
   ctgcagaacc tcgtgaccga gatagaggtg gcggagcacc aggcaagctt caccactcac   2940
   ggagtgcact gccttgcgcc aaaacaagtg gcagggattg cggatagcgt cgacgcgtct   3000
   gacgtggatg tgagcaggaa gttccacgac ggcgacctct ggacaagttc ctttgcctcc   3060
   aagacagaca cgcctgcaac catggcggca gccacgagca ttaccgccaa ccacgcgctc   3120
   actacaacac aatcatcagc tcttacgttc ccgatgaatg cggtcagcat cgatgatgac   3180
   gaggacgacg aagttgaggt gatggaggag ctgtatttcc gcatgaacgg tcccatcatt   3240
   gacgatgacg agtag                                                    3255
<210> 36
   <211> 336
   <212> DNA
   <213> Leishmania infantum
<400> 36
   atggcctccg acgaccgcat cacgttggca gatgacgcct ccgtcatctg cgaagacgtc    60
   gtcaacgcgc tcttcagccg cgaaacgcgc tatcaacaca gcaagatcgc cggcctcgtc   120
   tccgccatct ccgatcaggt ggtgcagcgg ctgacgcagg aagcgaagct gccgcgcaag   180
   tacgtcgtcc tcgtcacgat cctccagaaa aacggcgctg gcgtgcagat gatctcgtcg   240
   tgctcatgga acccaacgag tgatgcgtgc tacgtgtaca aggcggagaa caaagtcatg   300
   tactgtatca ttacggtcta cggtgtcacc gtgtag                             336
<210> 37
   <211> 2826
   <212> DNA
   <213> Leishmania infantum
<400> 37
   atgggcaagt cgaacccgcg acgccgcaac aaggcgtccg ccaagaaccg cccaaagggc 60
   caccaagatt gcacagaggt ggaaaaggac gtcgcgggcc acgttgtcct ggcaccggcg 120
   cagccgcccc atacattcac cccgctctgc gtgcagcaac tgcgggagtc tcgagagtac 180
   gctgtgctgg aggatgcgtg cagcgacatt gcgtactttg cgctgcaact gcggcacaac 240
   aacgacttct tgcgtgaagc cgtgccgcag cgcctcgcac agctgctcat cttctcccct 300
   ccctatgagc caccgcagct caacacgaac gcagatgcaa cgccggtggt ggcatcgcca 360
   acgttgtcgg ctgcagcgac ggcgggggag gaggagccgc aaccgcacaa gggcatcctg 420
   cacatgcaga ttgcggcggc agaggcgctg cgcactcttg ttgtgaacag tgagcaggac 480
   cgcgtcatcg atgcactcac gagcgctggc gcggacgcgg catcagggga tcgtttcgcg 540
   gaagggctgg cgcgcctcgt gcaggcgggc tggcagcgtg tgcagcaggc ccgccgacgc 600
   atggcgcccg aggattggat gggcgtcaac ggtgaagaag gtgaagaaga cgctggtagt 660
   ggtggtggtg atgaggccgc cgcggagggt gaggccgaac ctgctgccga cgcgagcgcg 720
   cgtcaccacc gccgccgtca gtccgggttc acgcgaaact tgtatttcat cctcctgcgc 780
   cacctggagg aggtgctagc gctcgccagc gtgtgtgtgg aggcgagcga ggtgtgcgcg 840
   cagacgtttt ccagcacttc ggcactcctg ctactgctcg acgtgctgcg cgtagcgatc 900
   acgacgacgt gggagacgct tctgcacccc tccgccgact actacgccga tgaagcgcag 960
   gtacctgtgg ctagcgaaac ggcgctcgcc attcgcgcct tcaagcggcg ggaggctggc 1020
   ctgctcgcct cgctggcggt tagcacagca gacttgttgc tcctgctcag ccccgagaac 1080
   cagtcactgg cgcaggtgtt caccggatca agcgagggca acggcgcagc ggtggccgcc 1140
   tcctcatctt cgtttccatc tgcagccggc agcagccccg ccatgacggc agagcagacg 1200
   gcgttcttga atactgtagt cgacgccgtc gacctgcgtg cctggctcgc ggagaagccg 1260
   gcagtcgaag tgcagctgca ggagcgcttc tccgacgcct ccctgcccag cttcctcggt 1320
   gcacagcaca cagctgtggc aatggagcga gagcgcgtct tgcacgacct tcttcgcgcg 1380
   accctctcga tccagggcgt gctcctgcac attgcaccgg ttgcagcgaa cctgcagcgc 1440
   gtcctgccgc tgctagatga caccctgcac aaggcgtcac tgccaatgca tgtgtggtgc 1500
   ggtgtgctgc cgcttctcat gggaacatgc gagcgcgccg acgacgacgt gcgagcggcg 1560
   gcagcgacgc tgaccacgca ccggctgcgc agcactcaag cggcggtccg cctactgcat 1620
   gtgatggtga acgctgtggg cgagaagaac gacacggacc ggtacgagga cgacgaagcc 1680
   gcgttcgccg ccaacccact ggccaagatt ctgcagtcag gagagcttct ctacgccttc 1740
   ggcctcctgc taaaggatgt gctctggatg tcgccgaaca gcgactccag cggtaacgca 1800
   gccaccgtgg agggccccgc gcacccgacg gcgacacgtg acgtcatggc ttggcaggcc 1860
   cagcagcgcg ctctgcgcgc tgaggccagc tcgaacgctg cggcgacgga gatgcagatg 1920
   gtgctgctcg ccacggaagt gaacgtgtgg gaggtggcaa gcacgctgct gctgatgctc 1980
   cctgtggcga gcctcggcga tcctgctccg acgtggcggg cactcttgca ggccgtgctg 2040
   agccgctatc agcttcacct cgacgccgcg gccatggagg cgtcggaggg agaggggcgc 2100
   agcgcacacg cgaacattct gcagcactcc gcccgcacgc atcagcttct ctggatgcag 2160
   cttgagtcgc tcgggcagat gctctggacg atgcagcgca agcagagcaa ggcgagcggt 2220
   ggcagcgccg cggtgacagc ctgcaaccac gtccaggcga catccgagga tgtcgacctg 2280
   ctcatccgtg tctcctggga ggcgtcggca agcacgctgc tgaggcagtc gtgcgtcggc 2340
   gccgtgggac tgctgtgcgc gtcgatgcag cgggaggagg ccgtggccac ggcggcgcgc 2400
   tttgccctgg ccgtggtcgc caacggcgga ccagcggcga cgtcctacgg gaagaagctc 2460
   ctcgacgtgt acgcgcaact tgtgccaggc aaaacgccca cctaccccgc ggagcggcgg 2520
   gtgtggctgc agcagcttgc gctggcggac gccgccgcca cggtccgctg cgaagcggcg 2580
   aacaccttga tagatctctt cctcgacgag cgccacgacg ccgccgtgta ctggccgctg 2640
   ggcgtacaag agaagctgag cgacttcaag aggcagctcg ccatctacgt gaaacggcgt 2700
   caacagctgg ataaggagac gaagcggctc taccacgttt cgacggagac gccagatgca 2760
   gagcagtggt cggaggtgct ggagaacctc tccgggttta tcgactacaa ggcacaccac 2820
   aagtag 2826
<210> 38
   <211> 4266
   <212> DNA
   <213> Leishmania infantum
<400> 38
   atgggcaacg atttgtcgac catgtcgggt gggggccgca accttgaggt ctggggtgaa 60
   gcgcgcgcca gacgcgtctc gaacgagctg ccgaccccgc gcaccatccc tctcggtcat 120
   ggggacaatc tcgtgccacc ggcgctgctc ctggacgctg agatgcgctc agccgcagac 180
   gaagcaagta gcacctcctc gttgcggtct gccggcacgg tgtcgaccta ctccactcag 240
   cactcggcac tttcaaaggc aaaccgaagc ccggatccac agccccggag gcgtagcatg 300
   tcgatgagtg atatgaccgc tgcgacagcc ttgctggcag ccgcggcgcc gtcacagcag 360
   cagcagcagc agcgacagct gcagaggcgc gcgcagtact ccatgtacgc tcggcagatg 420
   cggcgacccg gctgtgacgg gggccggtac gggttgcagc atcacaacag cggcgttgac 480
   ttcatggtcg ccaacgagtt tttcaactgc agcgacagca cccttggcag cgaggcggat 540
   ctcgatagca gcagtggcat cggaagcagc ggtagcgatg gtaatgatga cgcaggaggg 600
   gtgcaccacc gttgtggcat ggcaccaagg gagcccacct tgccgtacgg ttccggccgc 660
   tgcctgaccg gcaatgagct ctccgcgcat tccccgcggc gtggacggaa cgaggtggat 720
   ggagcgccgg ccctcggctc gactgctacg gccgctgccg ccgcctctgc tacgtcgcca 780
   gcgcacgcga agtcgagcag cacaggcagg gcagagacga taacgacgaa ggccgtctcc 840
   gtggtagtct ccggcgtcga ctgcagcagt gatccccctc cagctctcac cagcagccgc 900
   aacagcagca acggcagtca cgacagcaag tcctcggctg cgagtgggaa gaaaggcggt 960
   gccacacctt tgccgtcggc gaggcagggc aattggcaca agcggctctt gcggcccgtc 1020
   tcacgaggca gcattcgtga tgacggcatc agcggcagcg ccacgcagtg gggctcgcct 1080
   gacgccgccg atggtgctct cagctcgcac gaggaaaaga agtgcaagac gggcaagaga 1140
   aagacgagca agccgagaat ccacccggag ggactcttgg agctgaagtt tccgcatcgc 1200
   acgcgcagct cagcagcagc gccgcgaacg tcgtccgcgg cagccggaac cgcgaccacc 1260
   acgatcggtg tagaggcgct ggtgtcgagg atgaagctgt ccaccacgaa ggagtcgccg 1320
   agcgtcgacg caaagcagcg gcacaccagt ggcgacctca gcggcagcac cgcgccggct 1380
   ctgtcgaatg agaagcgtgg cggccagtgc gactctttca cgtgcgtgtc gtcctcgcca 1440
   gcgcagcagc atcacgccgc cggcaaggat ggcagaggta aacccggcgc gaaccgcagc 1500
   agcgacccgc ctcgctcgtc ggagtccccg gcgcatcatc acagccgtca agtgcaacat 1560
   catcactgcg gtggagacga tggaagcaat gccgttccag agacggtgtt gcccagcctt 1620
   gtagattcaa agtcgcacca tcgccaaccc agacgcaacc gcagcctcgg tggcaagctc 1680
   gacctgcacg acgagagccc caacgacttc ttcgcttcga cgcagcggag cgggcagtac 1740
   cggatgcttt accgaatgga cagcgaaagc gggagctcaa ccgcctccat gcgcgaggca 1800
   gtgtgcatgg ctaccggcgg cggcgcggcg gcggcgcact cctttggcag cctcttcgcc 1860
   gactcgcggg ccgcttcgct atactcggtg tcgtcgtcgc ccgggcaagc gtggacaggt 1920
   gactacgctg gccgcggagt gatgcaacta acagccgcgg tggatcaccg cgcgctggac 1980
   atgcagaaag tcgacgacgt cggcggcacg agtgaggcca gccggcatca ccctagtcgt 2040
   ggactgctct gcagcggaag ctgcccggcc tgtgtgaata gcacaggcac ctttaacgcg 2100
   acctcgcaac cgcagcaggc ggatctgcag cgcaaaatag agatgcacct ccagcggatg 2160
   acctacacgt cgaagtcgcc cagcctcgtc accagcaacc aagtcgtctc tgtgaggcca 2220
   gagaatagcc aaggtacccg acactcgctg atatctacgt tgccgatgcc tctgaacgtc 2280
   ggcgccgctg aagcgcctgc caaccgcggg caggtcggcg ccgccgcagt gatgcgtgaa 2340
   gcgctgtcgg ccggtgcgtc atgccgtgac ggccacggcc acgtccacac cgccgctagc 2400
   gtgagcagat gcagcaacgg tagcggcagc tctagcttcg gctcgagcag cagcggggat 2460
   cgcagtatca gccctgacgt aagtgtcaga gagctcgtga agtcgtctcg ggtgaagccg 2520
   ccagccaagg cagagtcgcc gccgctcctg ccacaaccac ccccgccaca gacggcggtg 2580
   atgcggacca agacacatag cttccgagga cacggcggca gacgtggacc cacgaacctg 2640
   tccgccaccg aaggcttggc ggcgtccgcg gtgtcgccga agaggcacag cagctgcgct 2700
   ggccggcggc atcatcacgg caacgttagc agcaacagtg gcgacgctct gcacgcaacg 2760
   gcgagtgtcc tcacctcggt acaaggcagc tgcagcgcgt tgcggccgac gcgggcagac 2820
   agcaaggacc tcattctccg gcccgaccgc actgcctctg cacgccgtca ccgccgcggt 2880
   ggtggcgatg agaacgcgcc ggaaggcagt ggcagccgtc agcggcaccc gcagaaccga 2940
   aaactttctt ccaggatgca ttttacctct ttcagcttta gcagtgacag cttcctatac 3000
   ggcaaggtgc gtggccatct ggcgaaggcg gcggcagtgg ctgcaaactc gagcagcatc 3060
   gagcgtcggc gtcacgcgtc gggctgtgaa agcagcggag gcacgcaggc acacgcgcgt 3120
   caccacgctc acggtgaagt cgactcggtt gcgctggggg cctccccatc cgcatcatcg 3180
   cgcaagggcg aggccgagtg tcgccatcgc ggcagcgggt gcggcaatga gagtgggatg 3240
   ggcagcgtgg tggctgactt ggacgacctc ctcctcggcg ccgatggcag ggccccctct 3300
   ggcaacctga cggcgctcta cccgcgctcc gcctcatcat cgttgtcgct ggcgccatcg 3360
   tcccgaaaca gcagcaccaa cggcagctgc gtcagcagcg gctccgtgcg tgcgctgcgc 3420
   aagagggctg cgagagcgcg ggacctgtcg gccagctcgt cagtcgactc cgaggcaagc 3480
   ggccgccttc atccacattg gacagcaccg ccgctgcccc agcagcagca gtatggagca 3540
   gctggtgcga cggcaacggc gtcgtcaatg taccgactgc ccctcgtgaa gacgtcgaac 3600
   agctgcggtg gagctgtggc cgctaacgga agcgcgagca gcgccgtgac cggtggaacg 3660
   cgcggcatga agctgccatc ctttaagccg aacatgggcg tgggcattag caacagccct 3720
   gacacgagca gcggccgccc tgtgaacatc aactacgttc ttcagccgct gcgaagcccg 3780
   gcggcgaacg gctcggcaac gatggcagca gcgtcaccag agatccacag cgtagccaag 3840
   agccacagca catcctctgg cagtggcgtt gccgcagaca cgtcgggtgc gatgccgacg 3900
   gtccatgggc acgaaaaggc gtcccggcag ccctccatgg tgcccaaagt gccttgcgcc 3960
   ctcttcaatg gcagtggcag cggtgcgcac ctgccacgaa tgccgccagc aagttcgcct 4020
   cgacacgcca gcagcaagag agacggtggc accgactcct tgacggtgtc gttcccggac 4080
   gccaacggcc gtggcgacgg tggtagtggc gctgctgcgt cgtcctccgc ggcgtcgctg 4140
   ttggcagggc catcccgtga ctcgagcgcg aagcgacgtc gccccacgac gcagagtttc 4200
   acgccgtggt ctgtggacat gagtcgcttc aaggcgctgc aagaggcgca ggagtcgccg 4260
   agttag 4266
<210> 39
   <211> 2604
   <212> DNA
   <213> Leishmania infantum
<400> 39
   atggagccgc gatggagggg tggccgtctc tccacggagc aggtggcgga cctgcgcact 60
   gaactagggc gtctggtcgc ggcgctcgcg gtcgcgtttc aggcaggcag agcagaagag 120
   gaaggacagc atacgcagga cgttgccgct gacgcgcatg acgcgaaagg cagtgcgcac 180
   acctcgagta gcgcaccgct cagcgacccg ctggcggaag aggcggccgc gctgcttctg 240
   aagccgttcg aagagatgca cctctctggc taccggcttg gcgatgccgc gctcacgtcc 300
   ccgagcatgc ccgacctgcc gtcctttctc tctgttgtgt ctcggcacac gcggctccgc 360
   gtgctcgatt tgagcttcaa caatctgaca ccgcaggtgc tggcgcccct cctccaagcc 420
   attcgtggtc tgccgttgct ggcggtgctg aagctcagcg gcaacatgct cggatcggcg 480
   cgcagtgacg actacaacgg cagccgcagc gagaaatggg acttgtccac cgtgagtgtc 540
   gatgaagagc tcggagatgc cgcgacggcg gctgcacggc ttggccgtta tctcgccaca 600
   aacccggcgc tgctggagtt ggcactgttt cactgcgatc tgaacgatca cgacgtgcgc 660
   gagctgctct ccggccttgt tcacccccgc aacaccaccc tgcacacatt gcagctctcc 720
   tggaacccgg cgtgcacctc gcgcagtgcg gagatggccc tgcagctggt cacggacctc 780
   ggaaacacca ccctgtgcga gctggagcta gagggcgagg cgccggcgac acaggtgcgt 840
   cttgagtacg cgtgcacccc cgacggtgtc ttccgctccg gacacaccct catcccggag 900
   agcgcggcgg caggggcagc gaccggcgac ggcaacgacg ataacagcgg caatgcgggc 960
   acggtgatca caagtgcggc agctgccgac gtggtgagcc ttgagcagct tcggcagcag 1020
   cagcagcaac tcgccgagat ccagaagcac ctcacacctg cccagggggc ccgctacgtg 1080
   aaccgctgcc gctacctcgc cgaggacttg gacgggtcac gtgtgttggc accgttggtg 1140
   atgcgagagc tgaaggcggt gctgtccatc cggctgcctc cgaggccgcg cgtcgacgaa 1200
   cgccacggcg cgagcgagtt gaacgtcgcc gccttcgcag aacgggagac ggcttctgcc 1260
   tctgctgctg gcgcgggagc acacagccaa gacggcagcg gagagctctt cacgtccgct 1320
   gcggcggtgg actgcggcgg cagcgaccct cacagtggcc gtgccgtgcc gtctccggcg 1380
   gccgctgcca actcgtccgc cggcctccag cggcggtcct tcgagggtgt ttctgcaatg 1440
   actgacggcc atgtggcaag tgttgcgcca cccccgcagg caacggatgc gccgcgcccg 1500
   cgcacgtctc ccccgatgca cctgcgccgt cggtggccca cccccgcggt gagcacgggc 1560
   agcgtagcca gtcccgcgtc ggggcgacac gcggcgcagc agcagcagca ccctcgcgca 1620
   ccgcaagagc tgcgtgatgc tcgcttcgaa acaccacagc gcctcgccgc tgtgatggcc 1680
   gacgccgagg catttcgccg tcgcctgcaa cctgtgcagc ttgacactca cgcgccgccg 1740
   tgcgccgagg tcagtcgata ccgcagcggc gcgcacagcg catcggtgac gaaaacgaga 1800
   tccctgtggt acgccgacga cggctttgtg ttgcgcccga atgggttgtc gaaggtgctc 1860
   gttgcaccgg tgctccctgg tggcgcgacg gccttcgcaa acgtggcact ggaggacgtg 1920
   gaagaccgcc ccctgcagcc ctgttggtgc acaccgcgcc agacgtctcc gatgactggc 1980
   atctacgctg gcatactgca ctaccactgc gtgcacgagg caacggcaca gcaacagaag 2040
   cgacagtacg gcacctcctc gtcgccaccg ccatcgacgg ctgcgacagc ggtgaagccg 2100
   gcggacccca gcgcgtccaa gagccggctg ccgctgctgc ttccccagcc ctcgcagcag 2160
   ccgcagccgc agctgcagcc gcaacagctg cccagcacgg ctgagatgta ctgcggatgc 2220
   cagggcaccg gtcatctctg cctctccgcg tgtgtggcct cagagggggc gggggctggt 2280
   cgacgccgcc atcgcatgcg gtcgacgttg cacgtaaagg ctgcaaaggc agtgggcaag 2340
   tctgcaggga gcggttatgg tgcgtcagcg tcccgcaaat ccgaccgcac gcgcgagcgc 2400
   ttcctcgact ggacggccac caatggtgac gctgtcaaca ccctcttgag caccgttgca 2460
   gcagcggcac caccggcgct gcatctgagc accgtgcgca cggcatgcgg tggtctctcg 2520
   atcgcgctca actacagccc agtgacccat ttctccgcgc cacacgtgag ctgcgcatca 2580
   ggcatgacgc tagcggagtt ctaa 2604
<210> 40
   <211> 2028
   <212> DNA
   <213> Leishmania infantum
<400> 40
   atgctccggc gaggtctgtg gcgatatcgc cgaatcagct acgtgacgga tgtcgagggg      60
   gacttcgcgt actttcagcg cttcgtctgt atctcacgag tgcttcggtg ggaaccggcg     120
   gaggctgcgg tctcctccaa ccctccccgc cctttcgccg ggggcagcaa cggctcgaga      180
   gtcgcgaatg gtcggtgggc ccccttgtcc tctgccgtgc ttccggcccc ctcgggggcg    240
   ttcccagcaa gcgatgctgc tgccagcacg caagcactgg gcaccaccac cacgcctgcc     300
   gtcacggatg cctacgagcc ggagtattgc cgtcttgtag acctctctcg ctatcgtcta     360
   ggcttccaag acgcgacctc acacttcgtc tttggtggcg acgccttcga ccacggcagc     420
   gacatcacgt tcggcaaggc gctgctcgac ttcaagcgcc gcttcccatc acgcgtgcac     480
   ctgctgcttg ggaaccggga cgtgaacaag atggccatgt acccacgcat ggcgtgcgag     540
   gtcgagggca tggcccccga cgcagccgag gacagcgtct tcacgctaac gccgccatcg     600
   atgaagaccg tcagggcggc gtcgctgacg gaagcgctgc ggtaccgaga cttcctactg     660
   gagcagcgac agcgacaaga agcgcaacgc caggacggcg cagggcacgc cgcatcaagc     720
   ccgtatggcg gcgcctcttg cggcagcagc ggcgataccc tgcgcacaga cgccgtttct     780
   ttcctgcagt gggcattgca gtacaaactc ggcggcccga acgcctttgc ccaccggcgg     840
   caggagttgc gcgagatggc ggccatgatg gccgaagggt gtggggcggt ggcggcggca     900
   ccccccgccg ctggtgcgga ggctatgagg gacggcattg cgtatgacga agacgccgtg     960
   gtcgccgcct cgttcttcac tgccgcgcag cccggaggcg tctactacga gtacatccgc    1020
   gctggcgttc tcagcgtcgt cctggacggc gtccttttcg tccacggagg cgtgaacgtc    1080
   agcaacgccg gctttgtgcc aagcctggag gcgacctcct acgcggagca ggtggccgtt    1140
   gggcagtggt ggctgccaga ggtcgctccg caggaggtga cgaccacgcc gactgcgtcc    1200
   tcggcgacct cggcgctggg ctggctcgct gccttggagc agtttaaagc ggctgccttc    1260
   tccgattggg tgaacggcgc tgccctgcgc ggcgaggcgc tgcgcgcgta cgtgtacccc    1320
   cgcgttgtgg ccccgcactc gatcgcggtg gggacggtga tgaacgcaga cggccctcac    1380
   tacatacctc tcactgttgc cgcctacctt ctccagtccg gcatccacac cgtgtgcgcc    1440
   gggcaccagc cggtcggcga cacccccgcc atcatccgcc agcccggtgg cttcacgatc    1500
   atagacgcgg acaactccta ctgcggccgt ggcaacaagt tctgcacgcg gttcaaccgc    1560
   cgcggcgcgg cggtgatgga ggtcttgttt gagcacccgg acgaccacgg cggcgacgat    1620
   aacgtggcgc cgcacgacga ggttgctgcg ccatcgctga ccgtccacgg ctaccgtgcc    1680
   gatggtacgc ctttcgagtt cgacgtctac agcgactggc acgtcgggcg ttacgtcggc    1740
   gacggttggt gggtgcggct gcccccagag gccaccgctg ccacttcatc gtctttcaga    1800
   ggcaacgagg cagcgcacgc gggactctac gagctgcgcc gcacccagga cgggttccgg    1860
   cacgaggaga cgcggtgggc cacagcagcg gaggtagacg cgtggctgag gcaggtcgcg    1920
   gcaagcggga aggcgacggt gccgggagag ttggcgccgc ggcacacgaa agaggagctc    1980
   gccgaggtgc gcgcccaccg cctcaagacg aaggtgaagc gcacctga                 2028
<210> 41
   <211> 789
   <212> DNA
   <213> Leishmania infantum
<400> 41
   atgcagcctg ttcaaagtcc tccagtagcc ccgccgtcgg taccggtcgc ggcgccaaag     60
   aagaccccga ttgatatatc tgcgctgaag ctcaagatgt cggccagcgt tcgagcaacc    120
   ctggctgctg ctggtgtgct tggccagtgc cctcggccga ttgaggcagc tgacgaggat    180
   gcgctgctta agctgaagcc gatgaagaca atagtgccct ccgtcaagaa agttgtggcg    240
   ccggttgcaa cgacggctcc gccgccgaag gtgcgccgcg tcgagagcag ttccagctca    300
   tcgtccgata gctcgtcctc gtcctccgat gacgactcca gcagtgatga cagctccggc    360
   tcggactctt cgtcctactc ctccgacgat cgctcctcct cgtcagctga gtcagtggtg    420
   tcaggggagg caacgctctt ccacattgcc cagtctcaag gcctggtgaa caaggaggtc    480
   ctggcacagg aagaggagga agtccccacg ctggtgcctc cgcgtcctcc ggtggtgcga    540
   tctttcccca atgacatcgg caaggctctg gaacgctacc gggaacgact caaccgcgag    600
   gagaacatca ttcgcatcaa ggatgacaac aaggctgtgt cgctcggcac cagtaaaatc    660
   aactacatcg acccccgtat catctgctcg tgggcaaagg cgcaagacgt gccgatcaac    720
   aagatcttct ctgcaaccat ccagaagaag ttcccgtggg ccatgaatgc cgagaacttc    780
   gatttttga                                                            789
<210> 42
   <211> 477
   <212> DNA
   <213> Leishmania infantum
<400> 42
   atgcggcgtt gtttgacccc agccagtgcg tgggtgtcca cgtggaggag ggtgacgacc     60
   gtgtcgcgtg ccgttggcta caaccgtagc cactgtccac ctttgacgga aaccgacgcg    120
   agcaagacga cgtcggcggc ttcgaccacc gcaccggccg cgccgacctc tgaggactac    180
   cgcgccctgc tactggacct cttgctgtac cgtcgcgaga cgttgactcc agtcgtggaa    240
   gcccttcgcc tcgagagaga cgaggtggaa aaaaagatgc agcagcttga cacgcacgtg    300
   cgcgagctca gctccattcg cgatgagctt cggcaactgg ccgaggcggt gctggacgca    360
   gagcgcacgc gtttgcacga ctttgtgatg aagcccagca accctcctgc cgcggagagc    420
   accatggcgg cacaaggcgc agcagagagc gaagaggttg aggaaattgt gctgtag       477
<210> 43
   <211> 1644
   <212> DNA
   <213> Leishmania infantum
<400> 43
   atgggtagcg ggtatctgaa gcggaagcgg cagcgccagg agctgctgaa cgacttctac 60
   tctgctctgt cgtcctcgtc atttgacact actggtgacc aatttagcaa gctggtgacg 120
   cgcaccgttt accactaccc ctcgtccctc ccgttccccg tgctcctcga tgacgacttc 180
   cccgctgagc cgtaccgatc ccgcaacaag gacattaaga gcgccatcca ctggggacag 240
   cgtaaacttc ttctctctga gattcaactc ctctccatgt actgccggcc agagatctcg 300
   tatcacatcg tgtatgctgg cgccgctcct gggacccatt tggcgtttct ggacgagatg 360
   ttctcgtgcc ggcacacgtg ggagctgatc gaccccggcc agtttgaccg cgcggtgctg 420
   gagccgcgcg caaacttcag gttgcgcaac gagttcttca ccaacgcgac agcctacggc 480
   atcaacgcgc gccgcctcac cgaagtcctc ccagcacttg gctttgtata ccagaacgct 540
   gcggttgaca gtgtgcaggc tgacaagaag gcgattcacg agcagcttca gggcattgtt 600
   ggcacgctgg acgtggcccg tggtaccgag gatatccctg ccatgtacga agcgccgctg 660
   tcgcttccta aaggcttgga gctgctgtcg caggttgcgc tggagcgcaa taagcctctg 720
   ctcttcatct cggatatccg ctccggcagt gtggcgctgc cgaacttcga ggaccacgtg 780
   gcagagaaca tgcgcgcgca ggagtgctgg acaaacatct tgcacggcga gcacagcatg 840
   cttaagttcc gccttcccta caccagcaaa tcgactaagt tcggcggcca gggcggcgtg 900
   cgtgaaacgc gcctgattcg cgaggacggc actgtcccct atctacgtgg tgacctgctg 960
   cttcccatct ggacgcggcc aacaagcacg gagggtcggc ttgtggtgcc acaaggcgcc 1020
   ttccagaagg actacaacgt agcccacgtg gaggaccaat tcttcttctt caactctagg 1080
   gttcgggagc aagtacactt caatcacctg gtcgttcatg acggcgagct cgatcatcac 1140
   tacgacgccg cagcggaggt gcactgcctc cttacctact tgcgctttat acggccagag 1200
   ctgaagaccg cgaaaacggg ggagctgcga aaggaggttc tgcgcgtggc gcactccatc 1260
   acggcgcacc ttggcatcac ttttgaggat gctattcgtc gtcgcgacgc acttatgatc 1320
   aaccacgcca agaagagtca gaacaaggcg gtggagggcc acaaggacga cgcttacggc 1380
   gacgaggaag tggaggagca ggaggcgaag agtgctgcat ccaaggtgcg cgctggctcg 1440
   aacgagagcg ccgcggcggc ggacggcgct gctgctggca caacagcgcc ggtgccgctg 1500
   tcggagtggg agcagacgac gcgactcatg gtgcgtctgt caaactacga ccgcaaccgg 1560
   ccggtgtgga gccggaacgt gagagaggag gagcacagca agactagcgg tttttgggtg 1620
   actacgaaga tgccacaggc gtga 1644